# EUROPEAN PATENT APPLICATION

(11) **EP 4 494 541 A1**
(43) Date of publication of application: **22.01.2025**
(21) Application number: 24781047.6
(22) Date of filing: 05.03.2024
(51) Int. Cl.: A47L 23/20, F26B 21/00, F26B 21/06, F26B 21/08, A61L 2/07, A61L 2/24

(54) **SHOE CARE APPARATUS**

(30) Priority: 30.03.2023 KR 20230042036
(71) Applicant: LG Electronics Inc., Yeongdeungpo-gu Seoul 07336 (KR)
(72) Inventor: KIM, So Ra, Seoul 08592 (KR); CHUN, Chan Ho, Seoul 08592 (KR); CHOI, Kyoung Min, Seoul 08592 (KR)
(74) Representative: Schott, Jakob Valentin
(86) International application number: PCT/KR2024/002791
(87) International publication number: WO 2024/205054

(57) **Abstract**

Provided is a shoe care device in which a shoe is treated by circulating air flow. The shoe care device according to one aspect of the present invention includes: an inner cabinet having an accommodation space configured to accommodate a shoe; a connection path configured to provide a flow path through which air from the accommodation space is introduced and then discharged back into the accommodation space; a nozzle duct installed inside the inner cabinet to provide an air passage and having an upper discharge port that opens upward; a nozzle coupled to an end of the nozzle duct to be inserted into the shoe inside the inner cabinet and having a lower discharge port that opens downward to spray air into the shoe; a blowing part disposed in the connection path to blow air; a dehumidifying part disposed in the connection path to dehumidify the air; a heating part disposed in the connection path to heat the air; a regeneration path branching from the connection path to allow the air heated by the heating path to move therethrough; and a steam generator configured to supply steam to the inner cabinet. The steam supplied to the inner cabinet is sprayed to the shoe through the upper discharge port and the lower discharge port.

## Description

### TECHNICAL FIELD

The present invention relates to a shoes care device, and more particular, to a shoes care device which treats shoes by air circulation.

### BACKGROUND

Shoes may get wet by a wearer's sweat, external contaminants, or rain or snow. Wearing such shoes may make the wearer uncomfortable, and in the condition, germs may also breed or stink in the shoes.

Accordingly, there is an increasing interest in a shoes care device 1, which removes germs and odors by performing a predetermined treatment on the shoes so that a user can comfortably wear the shoes all the times.

For the shoe care device described above, Korea Patent Laid-Open Publication No. 10-2021-0158763 (hereinafter, referred to as "Prior Document 1") discloses a "Shoe Management Device" in which a shoe sterilization device includes an accommodation part, first to third supply parts, and the like.

Here, the accommodation part includes therein an accommodation space configured to accommodate shoes, and the first to third supply parts are configured to guide fluid and supply the fluid into the shoes accommodated in the accommodation space

According to Prior Document 1, it is possible to smoothly supply fluid into the shoes so that the time and cost required to dry the shoes can be reduced.

However, in Prior Document 1, since the fluid can be supplied only into the shoes through the first to third supply parts, fluid to be used to treat the shoes cannot be supplied in a variety of ways, to the exteriors of the shoes aside from being supplied to the interiors of the shoes. Thus, there is a limitation in that only the treatment concentrated on the interiors of the shoes can be achieved.

In particular, since no special consideration is given to the configuration to prevent condensed water, which is generated in the first to third supply parts in the process of supplying fluid into the shoes through the first to third supply parts, from flowing into the shoes, there is a problem in that the effective treatment of the shoes cannot be achieved when the condensed water flows into the shoes.

In addition, Korean Patent No. 1037245 (hereinafter, referred to as "Prior Document 2") discloses "Apparatus for sterilization disposal of shoes", which includes a main body, an ultraviolet emission module, and a deodorization module.

According to Prior Document 2 above, the shoes are disposed in a sterilization chamber of the main body, and the ultraviolet emission module is driven to remove germs and odors of the shoes. Furthermore, the air in the sterilization chamber is inhaled into a ventilation pipe and discharged to the outside of the main body through an exhaust port through the deodorization module.

Here, the deodorization module includes a deodorization column made up of materials such as zeolite, activated carbon, and charcoal, and removes contaminants from the air discharged from the inside of the main body to the outside by the deodorization column.

According to Prior Document 2 above, the air from which moisture has been removed by a deodorization module including zeolite and activated carbon can be discharged to the outside of the apparatus for sterilization disposal of shoes.

However, in Prior Document 2 above, since the air is discharged to the outside of the apparatus for sterilization disposal of shoes, the air that has not sufficiently removed moisture or odors may be discharged to the outside of the apparatus for sterilization disposal of shoes, and such air may be discharged to the inside where the wearer resides.

In addition, Korean Patent Unexamined Publication No. 10-2000-0009653 (hereinafter referred to as "Prior Document 3") discloses "The shoes cabinet for sanitation", which includes a main body, a far infrared radiation part, a circulation fan, an air circulation passage, and a sanitary filter portion.

According to Prior Document 3 above, while storing shoes in shoe closet, hygiene treatments such as dehumidification, sterilization, and deodorization can be performed on the shoes by far-infrared rays and a filter during the storing of the shoes.

Here, since the sanitary filter portion is filled with excellent adsorptive materials such as charcoal, it can serve to adsorb moisture in the process of ventilating air and filter bacteria, as well as to capture malodorous substances.

According to Prior Document 3 above, the air is circulated by a circulating fan in a shoe closet, and the sanitary filter portion is disposed on a circulation path of the air to remove germs and odors in the air

However, since Prior Document 3 above does not consider a technology to prevent the reduction of the performance of the sanitary filter portion configured to remove moisture or odors, a user may not be satisfied because the shoes are not properly treated at the time of using a shoes care device.

As described above, for the shoes care device that removes germs or odors by treating the shoes in a predetermined manner, a task that has to be solved to ensure proper performance for shoes treatment while preventing the air used for dehumidification and deodorization from being exposed to the user is present in in the process of treating the shoes.

However, there is a limitation in that the conventional shoes care device cannot properly solve such a task.

In addition, when designing and manufacturing devices including a dehumidifier such as zeolite, it is necessary to consider how the dehumidification efficiency of a dehumidifying material can be further improved, whether the dehumidifying material can be effectively regenerated, whether water vapor generated during the use of the shoes care device and the regeneration of the dehumidifying material can be effectively removed or managed, whether moisture is left in an unintended area during use of the shoes care device and the regeneration of the dehumidifying, whether components are properly disposed in a limited space, and where the devices provides excellent use convenience. There is a need to develop a shoes care device that takes these matters into consideration.

In addition, the reduction of manufacturing costs, the convenience of manufacturing and assembling each component, and the convenience of maintenance needs to be considered in the development of the shoes care device.

### DISCLOSURE

### TECHNICAL PROBLEM

An object to be achieved by the present invention is to solve the aforementioned problems caused by a shoes care device which treats shoes by air circulation.

Specifically, an object to be achieved by the present invention is to provide a shoes care device which can ensure proper performance for shoes treatment at all times by performing dehumidification and deodorization on the shoes using a dehumidifying material to refresh the shoes and regenerating the used dehumidifying material.

An object to be achieved by the present invention is to provide a shoes care device which prevents air used for dehumidification and deodorization of shoes from being exposed to a user by an air circulation structure capable of dehumidifying the air inside an inner cabinet where the shoes are placed by using a dehumidifying material and supplying the dehumidified air again to the inner cabinet.

An object to be achieved by the present invention is to provide a shoe care device that allows steam treatment of shoes to be performed more appropriately so that treatment efficiency for the shoes can be optimally achieved without functional damage.

The technical objects of the present disclosure are not restricted to those set forth Here, and other unmentioned technical objects will be clearly understood by one of ordinary skill in the art to which the present disclosure pertains by referencing the detailed description of the present disclosure given below.

### TECHNICAL SOLUTION

In order to achieve the above and other objects, a shoes care device according to one aspect of the present invention is configured not only to perform dehumidification and deodorization of shoes using a dehumidifying part but also to regenerate the used dehumidifying part. Specifically, the shoes care device is configured not only to collect moisture and bacteria in air ventilated by the dehumidifying part in a module chamber but also to heat and regenerate the dehumidifying part in the module chamber.

In addition, according to one aspect of the present invention, the shoes care device is configured such that the air used for dehumidifying and deodorizing the shoes has an air circulation structure inside the shoes care device. Specifically, a connection path through which air is circulated is formed between a outlet and a nozzle disposed inside an inner cabinet, respectively.

In addition, the shoe care device according to one aspect of the present invention is configured to spray steam not only to the exteriors but also to the interiors of the shoes during steam treatment of the shoes. Specifically, the steam supplied to the inner cabinet is sprayed not only to the exteriors of the shoes through the upper discharge port of the nozzle duct, but also to the interiors of the shoes through a lower discharge port of the nozzle.

In addition, in the shoe care device according to one aspect of the present invention, steam from a steam generator may be supplied to a portion of the dry air duct and moved to the nozzle duct and the nozzle.

In addition, in the shoe care device according to one aspect of the present invention, the steam from the steam generator may be supplied to a steam separator and then moved to a dry air duct through a steam connection pipe.

In addition, in the shoe care device according to one aspect of the present invention, the dry air duct is disposed above the steam separator so that the steam can move upward.

In addition, the shoe care device according to one aspect of the present invention performs a steam stroke between the first regeneration stroke and the first drying stroke, and while the first regeneration stroke is being performed, the air that has passed through the dehumidifying part may flow into an accommodation space and increase the temperature of the accommodation space

In addition, the shoe care device according to one aspect of the present invention may perform the steam stroke in the state in which a damper blocks a dry air outlet of a module housing.

In addition, the shoe care device according to one aspect of the present invention may discharge condensed water inside the dry air duct to a condenser while the damper opens the dry air outlet of the module housing.

In addition, in the shoe care device according to one aspect of the present invention, the nozzle may include a nozzle body and a nozzle protrusion.

In addition, the shoe care device according to one aspect of the present invention may have a nozzle duct installed to face the front side from the rear wall of the inner cabinet.

In addition, the shoe care device according to one aspect of the present invention may have a discharge slope provided to guide the steam sprayed from the lower discharge port toward the rear side of the inner cabinet.

In addition, the shoe care device according to one aspect of the present invention may have a discharge through-hole in a portion of the discharge slope.

In addition, the shoe care device according to one aspect of the present invention may also spray steam through the auxiliary discharge port provided in the nozzle body within the inner cabinet.

In addition, in the shoe care device according to one aspect of the present invention, the steam supplied to the inner cabinet may be distributed to and sprayed from the upper discharge port and the auxiliary discharge port before sprayed to the interiors of the shoes.

In addition, in the shoe care device according to one aspect of the present invention, the nozzle duct may be coupled to the inner cabinet in a penetration structure.

In addition, in the shoe care device according to one aspect of the present invention, the nozzle duct may be sealed at a coupling surface other than the portion where the nozzle duct penetrates the inner cabinet, by a nozzle sealing part.

In addition, the shoe care device according to one aspect of the present invention may be configured such that the nozzle sealing part has heat-resistant performance.

The technical objects of the present disclosure are not restricted to those set forth Here, and other unmentioned technical objects will be clearly understood by one of ordinary skill in the art to which the present disclosure pertains by referencing the detailed description of the present disclosure given below.

### BRIEF DESCRIPTION OF THE DRAWINGS

FIG. 1a is a perspective view illustrating a shoes care device according to one embodiment of the present invention.
FIG. 1b is a perspective view of the shoes care device of FIG. 1a, when viewed from another direction, illustrating a state in which a door is opened.
FIG. 2a is a perspective view illustrating a state in which a part of the door and an outer cabinet are removed from the shoes care device of FIG. 1b.
FIG. 2b is a perspective view illustrating a state in which the shoes care device illustrated in FIG. 2a is viewed from another direction.
FIG. 3 is a front view illustrating a state in which a door is removed from the shoes care device illustrated in FIG. 1b. FIG. 3 illustrates shoes accommodated in an inner cabinet together
FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.
FIG. 5 is a perspective view illustrating a state in which the door, the outer cabinet, and the inner cabinet are removed from the shoes care device according to one embodiment of the present invention.
FIG. 6 is a perspective view illustrating a part of a machine room of the shoes care device illustrated in FIG. 5.
FIG. 7a is a view illustrating a steam valve according to one embodiment of the present invention, and illustrating a connection relationship considering the movement of steam.
FIG. 7b is an exploded perspective view illustrating the steam valve illustrated in FIG. 7a.
FIG. 8a is a cross-sectional view taken along line D-D' of the shoes care device illustrated in FIG. 3. FIG. 8a illustrates a state in which the door is included in the shoes care device, and does not illustrate the shoes.
FIG. 8b is a view illustrating a state in which a main shelf is removed from the shoes care device illustrated in FIG. 8a.
FIG. 9 is a cross-sectional view taken along line E-E' of the shoes care device illustrated in FIG. 3. FIG. 9 illustrates a state in which the door is included in the shoes care device.
FIG. 10a is an exploded perspective view illustrating a drying module in the shoes care device according to one embodiment of the present invention. FIG. 10b is an exploded perspective view illustrating a partial configuration of the drying module at a part to which a damper is coupled.
FIG. 11 is a diagram illustrating a connection relationship between components and a flow of fluid in the shoes care device according to one embodiment of the present invention.
FIG. 12 is a view illustrating in more detail a nozzle duct and a nozzle installed in the inner cabinet in the shoe care device according to one embodiment of the present invention.
FIG. 13 is a cross-sectional view illustrating coupling structures at one end and the other end of the nozzle duct of FIG. 12.
FIG. 14 is a cross-sectional view illustrating a portion where the nozzle duct of FIG. 12 is coupled to the inner cabinet.
FIG. 15 is a cross-sectional view illustrating a portion where the nozzle duct of FIG. 12 is coupled to the nozzle.
FIGS. 16 and 17 are views illustrating the state in which the angle of the nozzle duct of FIG. 12 is adjusted.
FIG. 18 is a cross-sectional view illustrating the interior of the nozzle duct of FIG. 12.
FIG. 19 is a view illustrating the nozzle duct and the nozzle of FIG. 12 viewed in another direction.
FIG. 20 is a view illustrating the nozzle duct and the nozzle of FIG. 12 viewed in another direction.
FIG. 21 is an exploded view illustrating the nozzle duct and the nozzle of FIG. 20.
FIG. 22 is a cross-sectional view illustrating the interior of the nozzle duct of FIG. 20.
FIGS. 23 and 24 are views illustrating the nozzle sealing part of FIG. 20 in more detail.
FIGS. 25 to 28 are views illustrating the nozzle supporter of FIG. 20 in more detail.
FIGS. 29 to 31 are views illustrating the hinge axis resistance part of FIG. 20 in more detail.
FIG. 32 is a cross-sectional view illustrating the interior of the nozzle illustrated in FIG. 20.
FIG. 33 is a perspective view illustrating a steam separator according to one embodiment of the present invention.
FIG. 34 is a cross-sectional view illustrating a separating inlet of the steam separator in the shoe care device according to one embodiment of the present invention.
FIG. 35 is a view schematically illustrating the flow of steam supplied to the inner cabinet in the shoe care device according to one embodiment of the present invention.
FIG. 36 is a view illustrating the progress of a stroke according to the type of an operation signal in a method of controlling the shoe care device according to one embodiment of the present invention.
FIG. 37 is a view showing the progress of strokes after switching to an operation mode in the method of controlling the shoe care device according to one embodiment of the present invention.
FIG. 38a is a view illustrating, by way of an example, a first execution mode in the method of controlling the shoe care device according to one embodiment of the present invention.
FIG. 38b is a view illustrating a modification of a sterilization course in the first execution mode of the shoe care device according to one embodiment of the present invention.
FIG. 38c is a view illustrating temperature changes inside and outside a shoe according to the execution of the sterilization course illustrated in FIG. 38b.
FIG. 39 is a view illustrating, by way of an example, a second execution mode in the method of controlling the shoe care device according to one embodiment of the present invention.
FIG. 40 is a view illustrating, by way of an example, a third execution mode in the method of controlling the shoe care device according to one embodiment of the present invention.
FIG. 41 is a view illustrating a change in the temperature of air passing through the dehumidifying part in the shoe care device according to one embodiment of the present invention.
FIG. 42 is a view illustrating a change in humidity according to temperature differences of air passing through the dehumidifying part in the shoe care device according to one embodiment of the present invention.

### DETAILED DESCRIPTION

Hereinafter, exemplary embodiments disclosed herein will be described in detail with reference to the accompanying drawings, and like reference numerals designate like elements, and redundant description thereof will be omitted. Suffixes "module" and "unit or portion" for elements used in the following description are merely provided for facilitation of preparing this specification, and thus they are not granted a specific meaning or function. Wherever possible, the same reference numbers will be used throughout the drawings to refer to the same or like parts. In the following description, known functions or structures, which may confuse the substance of the present disclosure, are not explained. The accompanying drawings are used to help easily explain various technical features and it should be understood that the exemplary embodiments presented herein are not limited by the accompanying drawings. As such, the present disclosure should be construed to extend to any alterations, equivalents and substitutes in addition to those which are particularly set out in the accompanying drawings.

Although the terms first, second, and the like, may be used herein to describe various elements, these elements should not be limited by these terms. These terms are generally only used to distinguish one element from another

When an element or layer is referred to as being "on," "engaged to," "connected to," or "coupled to" another element or layer, it may be directly on, engaged, connected, or coupled to the other element or layer, or intervening elements or layers may be present. In contrast, when an element is referred to as being "directly on," "directly engaged to," "directly connected to," or "directly coupled to" another element or layer, there may be no intervening elements or layers present.

The singular expressions include plural expressions unless the context clearly dictates otherwise.

It should be understood that the terms "comprises," "comprising," "includes," "including," "containing," "has," "having" or any other variation thereof specify the presence of stated features, integers, steps, operations, elements, and/or components, but do not preclude the presence or addition of one or more other features, integers, steps, operations, elements, and/or components.

A first direction (X direction), a second direction (Y direction), and a third direction (Z direction) described in one embodiment of the present invention may be directions orthogonal to each other.

Each of the first direction (X direction) and the second direction (Y direction) may be a direction parallel to the horizontal direction, and the third direction (Z direction) may be a direction parallel to the vertical direction. When the first direction (X direction) is parallel to a left-right direction, the second direction (Y direction) may be parallel to a front-rear direction. When the first direction (X direction) is parallel to the front-rear direction, the second direction (Y direction) may be parallel to the left-right direction.

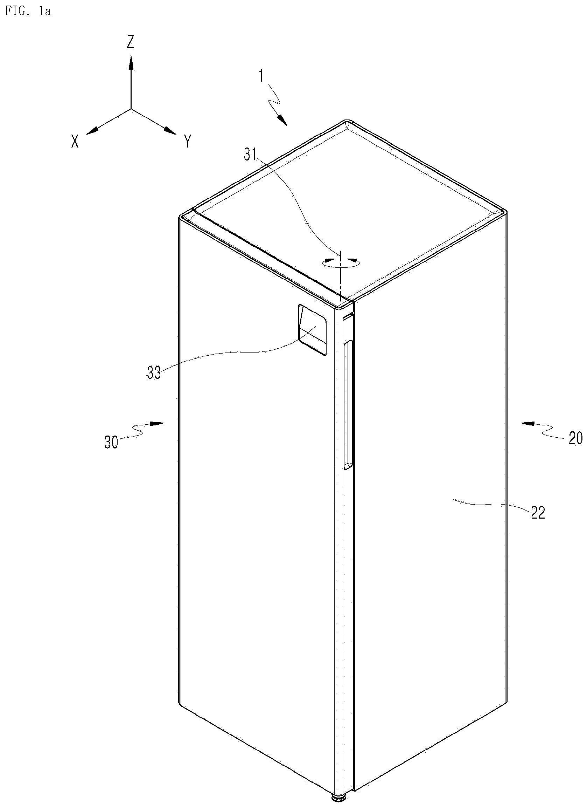

FIG. 1a is a perspective view illustrating a shoes care device according to one embodiment of the present invention.

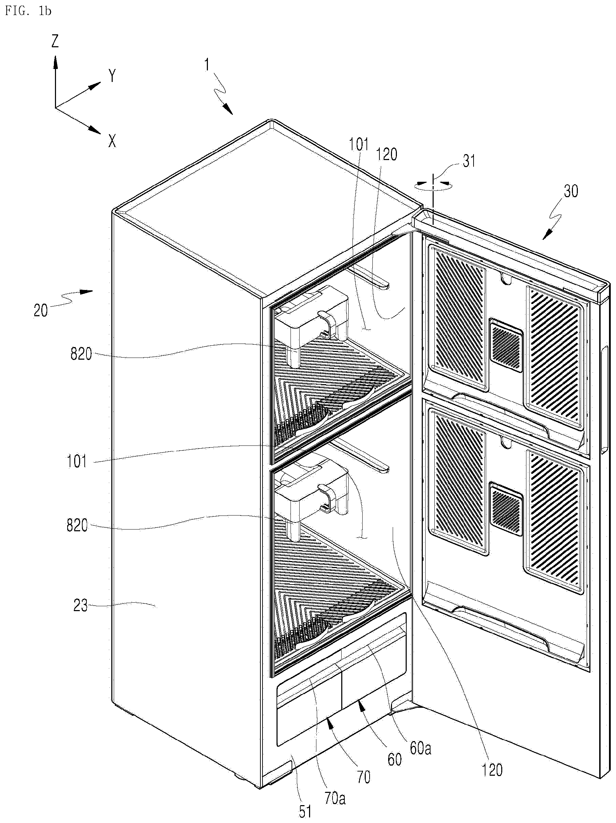

FIG. 1b is a perspective view of the shoes care device of FIG. 1a, when viewed from another direction, illustrating a state in which a door is opened.

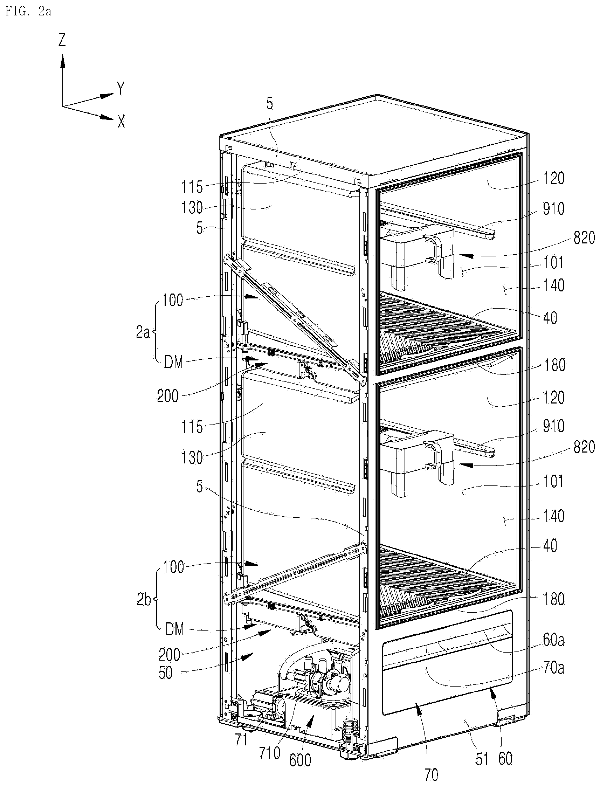

FIG. 2a is a perspective view illustrating a state in which a part of the door and an outer cabinet are removed from the shoes care device of FIG. 1b.

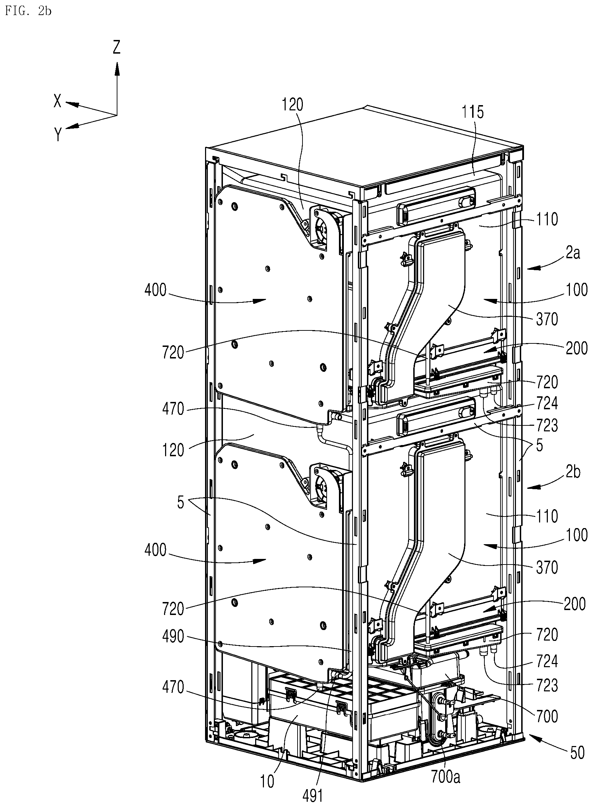

FIG. 2b is a perspective view illustrating a state in which the shoes care device illustrated in FIG. 2a is viewed from another direction.

A shoes care device 1 according to one embodiment of the present invention may include an outer cabinet 20, a door 30, an inner cabinet 100, and a machine room 50. The shoes care device 1 may include a main frame 5.

The outer cabinet 20 and the door 30 may form an overall appearance of the shoes care device 1. The outside of the shoes care device 1 may be formed in a hexahedral shape. That is, while the outer cabinet 20 and the door 30 are coupled to each other and the door 30 is closed, the appearance of the shoes care device 1 may be formed in a hexahedral shape. However, the shoes care device 1 according to one embodiment of the present invention is not limited to such a shape and may have various three-dimensional shapes.

When the door 30 forms the front of the shoes care device 1, the outer cabinet 20 may form an upper side surface, a left-side surface, a right-side surface, a rear surface, and bottom surfaces of the shoes care device 1.

The main frame 5 may form an overall framework of the shoes care device 1. The main frame 5 may have a hexahedral structure.

The outer cabinet 20 may be detachably fixed to the main frame 5.

The outer cabinet 20 may include an outer rear plate 21, a first outer side plate 22, and a second outer side plate 23.

The outer back plate 21, the first outer side plate 22, and the second outer side plate 23 may be formed integrally with each other, or the outer back plate 21, the first outer side plate 22, and the second outer side plate 23 may be individually formed.

The outer rear plate 21 forms a vertically erected wall surface. The outer rear plate 21 may form a surface orthogonal to the first direction (X direction). The outer rear plate 21 may form a rear wall surface in the first direction (X direction) on the outer cabinet 20. The outer rear plate 21 may form a rear surface in the first direction (X direction) in the shoes care device 1. The outer rear plate 21 may form an entire outer rear surface of the shoes care device 1.

The first outer side plate 22 and the second outer side plate 23 form vertically erected wall surfaces, respectively, and form opposing wall surfaces facing each other.

The first outer side plate 22 is disposed at any one side with respect to a reference plane RP that is parallel to the first direction (X direction) as a horizontal direction and is a vertical plane. The second outer side plate 23 is disposed on the opposite side of the first outer side plate 22 with respect to the reference plane RP. The first outer side plate 22 may form a left wall surface of the outer cabinet 20, and the second outer side plate 23 may form a right wall surface of the outer cabinet 20.

The outer cabinet 20 may be disposed outside the inner cabinet 100 and the machine room 50 to form an outer wall surface of the machine room 50. When a separate cabinet for the machine room 50 is not provided in the shoes care device 1, the outer cabinet 20 may form a wall separating the machine room 50 from the outside.

The door 30 is configured to open and close the inside of the shoes care device 1. The door 30 may form any one surface of the shoes care device 1. The door 30 may form a left side or a right side of the shoes care device 1, or may form a front side of the shoes care device 1.

In the shoes care device 1, the door 30 may be hinge-coupled.

In one embodiment, the door 30 may be hinge-coupled to the main frame 5. In another embodiment, the door 30 may be hinge-coupled to the outer cabinet 20, and in another embodiment, the door 30 may be hinge-coupled to the inner cabinet 100 and/or the machine room 50.

A hinge rotation axis 31 of the door 30 may be formed in the vertical direction. That is, in the shoes care device 1, the door 30 may be configured to be rotatable bidirectionally around a rotation axis 31 in the vertical direction.

In one embodiment, the shoes care device 1 may include one door 30. In another embodiment, the shoes care device 1 may include two or more doors.

When two doors are provided in the shoes care device 1, each door may be individually rotated around each rotation axis.

The first direction (X direction) described in one embodiment of the present invention may be parallel to or substantially parallel to the horizontal direction.

In one embodiment, the first direction (X direction) may be a direction from the rear of the shoes care device 1 to the front.

Hereinafter, except for a particularly limited case, it will be described that the door 30 is formed in a front side of the shoes care device 1. That is, a surface on which the door 30 is formed in the shoes care device 1 is described as a front surface of the shoes care device 1.

Furthermore, hereinafter, except for a particularly limited case, it will be described that the first direction (X direction) is parallel to the front-rear direction, the second direction (Y direction) is parallel to the left-right direction, and the third direction (Z direction) is parallel to the vertical direction.

The inner cabinet 100 and the machine room 50 may be provided inside the outer cabinet 20.

The inner cabinet 100 may be formed in a box shape, and a predetermined space may be formed therein. The space inside the inner cabinet 100 forms an accommodation space 101, and shoes S may be accommodated in the accommodation space 101.

A plurality of shoes S may be disposed together in the accommodation space 101 of one inner cabinet 100.

The inner cabinet 100 may be fixed to the main frame 5.

The inner cabinet 100 has a predetermined size along the first direction (X direction), the second direction (Y direction), and the third direction (Z direction).

The inner cabinet 100 is formed in the shape of a box opened to any one side. The inner cabinet 100 may be formed in a form opened to the front side of the shoes care device 1. The inner cabinet 100 may be configured to include a main opening 140. The main opening 140 may be provided to open the front side of the inner cabinet 100 in the first direction (X direction). The shoes may be disposed inside the inner cabinet 100 or withdrawn from the inner cabinet 100 through the main opening 140.

The main opening 140 of the inner cabinet 100 may be closed or opened by the door 30.

The inner cabinet 100 may include an inner rear plate 110, a first inner side plate 120, a second inner side plate 130, and an inner upper plate 115.

The inner rear plate 110, the first inner side plate 120, the second inner side plate 130, and the inner upper plate 115 may be formed integrally with each other. The inner cabinet 100 may be made of a single material and be formed by injection molding.

The inner rear plate 110 forms a vertically erected wall surface. The inner rear plate 110 may form a surface orthogonal to the first direction (X direction). The inner rear plate 110 may form a rear wall surface of the inner cabinet 100 in the first direction (X direction). The inner rear plate 110 may be formed parallel to the outer rear plate 21.

The first inner side plate 120 and the second inner side plate 130 form vertically erected wall surfaces, respectively, and form opposing wall surfaces facing each other.

The first inner side plate 120 is dispose on either side with respect to the reference plane RP that is parallel to the first direction (X direction) as the horizontal direction and is a vertical surface. The second inner side plate 130 is disposed on the opposite side of the first inner side plate 120 with respect to the reference plane RP. The first inner side plate 120 forms a left wall surface of the inner cabinet 100, and the second inner side plate 130 forms a right wall surface of the inner cabinet 100.

The first inner side plate 120 may be formed parallel to the first outer side plate 22, and the second inner side plate 130 may be formed parallel to the second outer side plate 23.

In one embodiment of the present invention, the inner cabinet 100 may have the form where a lower part thereof is opened. Accordingly, the inner cabinet 100 includes a lower opening 150 formed by opening the lower part thereof. When the inner cabinet 100 is formed in a hexahedral shape on the whole, the lower opening 150 may be formed large to form all or most of a lower surface of the inner cabinet 100 (see FIGS. 53 and 54).

However, the shoes care device 1 according to one embodiment of the present invention is not used in a state in which an entire lower part of the inner cabinet 100 is opened, but is used in a state in which the inner cabinet 100 and a module housing 200 are coupled to each other so that the lower opening 150 of the inner cabinet 100 is shielded by the module housing 200. That is, the shoes care device 1 is used so that an upper surface of the module housing 200 forms a bottom surface of the accommodation space 101 of the inner cabinet 100. A further explanation thereof will be described below.

A main shelf 40 may be provided in the inside 101 of the inner cabinet 100. The main shelf 40 may be formed such that the shoes S are settled on an upper surface thereof.

The main shelf 40 may be formed in the form of a plate with a predetermined area, or may be formed in the form of a grill where multiple bars are spaced apart from each other.

One main shelf 40 may be provided, or a plurality of main shelves 40 may be provided.

The main shelf 40 may have a substantially flat plate shape and be disposed on a bottom surface of the inner cabinet 100. The main shelf 40 is settled on an upper side of a module cover 202 of the inner cabinet 100. The main shelf 40 may be disposed on the upper side of the module cover 202 of the inner cabinet 100 in a stacked form.

The main shelf 40 is detachable from the inner cabinet 100, and when the main shelf 40 is withdrawn from the inner cabinet 100, the upper surface of the module housing 200 is exposed.

The main shelf 40 may have a rectangular shape when viewed in a plan view. The size of the main shelf 40 may be a size corresponding to the bottom of the accommodation space 101 of the inner cabinet 100. That is, when the main shelf 40 is disposed inside the inner cabinet 100, the main shelf 40 may form all or most of the bottom of the accommodation space 101 of the inner cabinet 100.

In one embodiment, the machine room 50 may be provided in a lower side of the inner cabinet 100. In the machine room 50, some of the components that constitutes the shoes care device 1 may be accommodated, and, in this case, the components that are accommodated in the machine room 50 may be fixed to a main frame 5 or to the inner cabinet 100 or the outer cabinet 20.

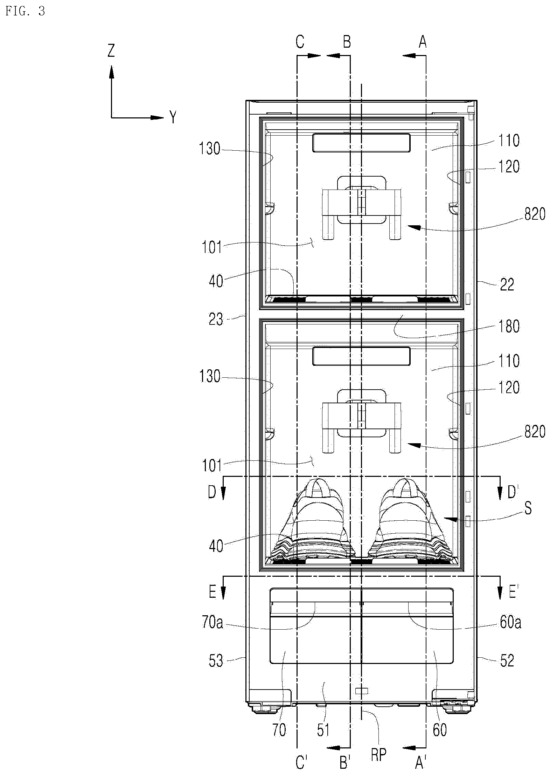

FIG. 3 is a front view illustrating a state in which a door is removed from the shoes care device illustrated in FIG. 1b. FIG. 3 illustrates shoes accommodated in an inner cabinet together

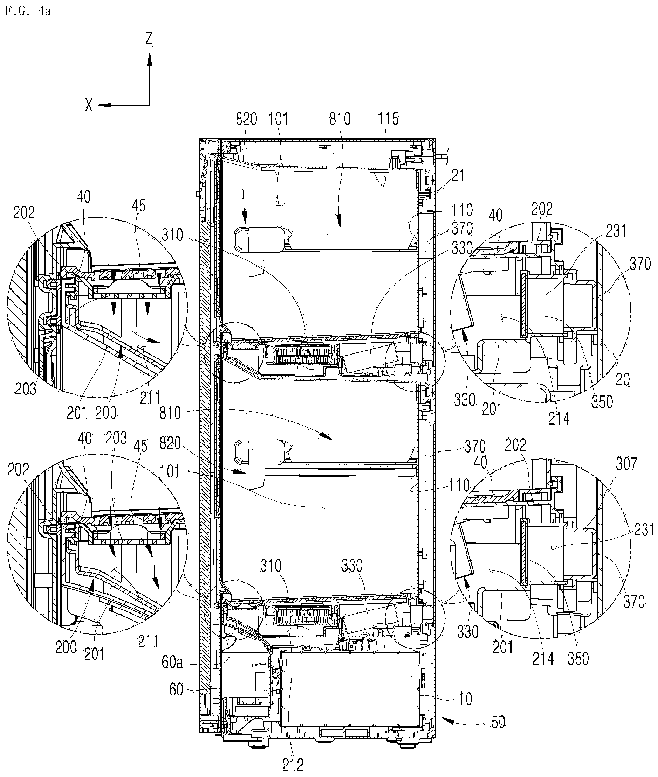

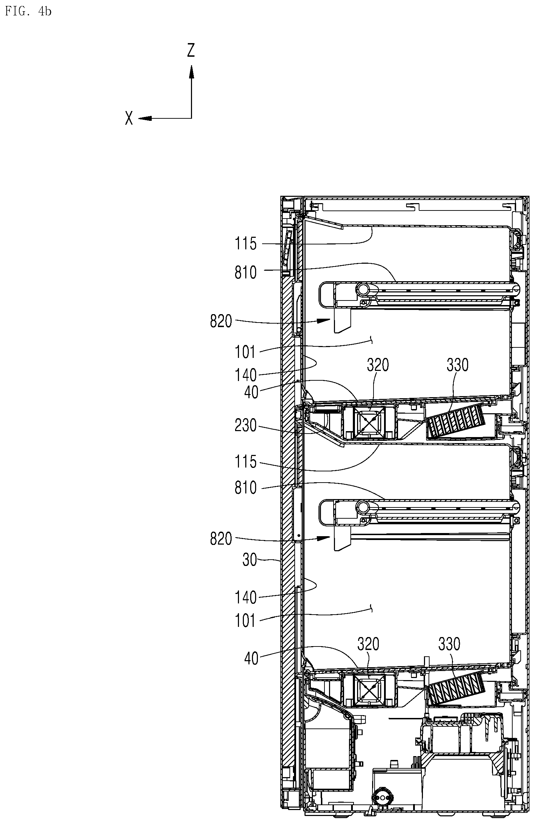

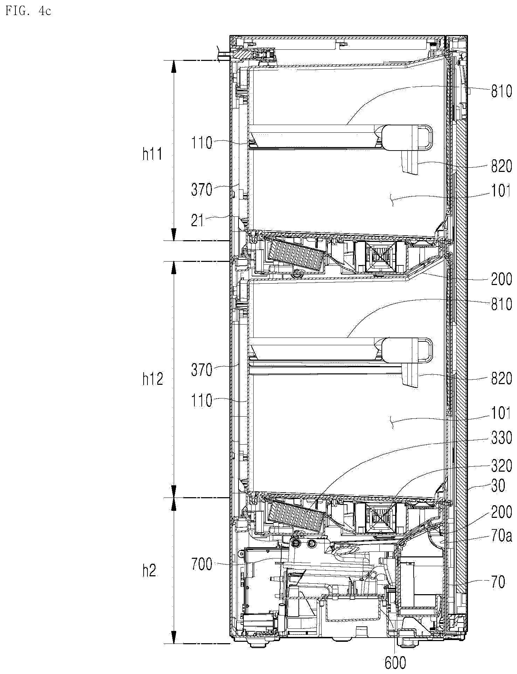

FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.

The shoes care device 1 according to one embodiment of the present invention includes management devices 2a and 2b. In the shoes care device 1, a plurality of management devices 2a and 2b may be provided. In one embodiment, the shoes care device 1 may include a first management device 2a and a second management device 2b. That is, the shoes care device 1 may include two separate management devices 2a and 2b.

The 'management device' described in the present invention may refer to a 'first management device 2a' and a 'second management device 2b', respectively, except for a particularly limited case.

The management devices 2a and 2b include the above-described inner cabinet 100.

In one embodiment of the present invention, since the inner cabinet 100 of the first management device 2a and the inner cabinet 100 of the second management device 2b may be distinguished from each other, the inner cabinet 100a of the first management device 2a may refer to a first inner cabinet 100a, and inner cabinet 100a of the second management device 100b may refer to a second inner cabinet 100b.

It may be understood that the 'inner cabinet 100' described in one embodiment of the present invention refers to each of the 'first inner cabinet 100a' and the 'second inner cabinet 100b', except for a particularly limited case.

When the door 30 is closed in the shoes care device 1, the door 30 closes the main opening 140 of the first management device 2a and also closes the main opening 140 of the second management device 2b. That is, the door 30 may simultaneously seal the accommodation space 101 of the inner cabinet 100 of the first management device 2a and the accommodation space 101 of the inner cabinet 100 of the second management device 2b. In this case, the accommodation space 101 of the inner cabinet 100 of the first management device 2a and the accommodation space 101 of the inner cabinet 100 of the second management device 2b may be configured such that they do not communicate with each other.

As described above, in one embodiment of the present invention, the accommodation space 101 of the inner cabinet 100 of the first management device 2a and the accommodation space 101 of the inner cabinet 100 of the second management device 2b may form independent spaces, and may form blocked spaces (not communicating with each other). Accordingly, the temperature and humidity of the accommodation space 101 of the first management device 2a and the temperature and humidity of the accommodation space 101 of the second management device 2b may be controlled differently from each other.

The management devices 2a and 2b may be configured to include a connection path F10. The management devices 2a and 2b may be configured to include a blowing part 310 and a dehumidifying part 330. The management devices 2a and 2b may include a outlet 203 and a nozzle 820.

The management devices 2a and 2b may include the module housing 200 forming a connection path F10.

The management devices 2a and 2b may include a regeneration path F20. The management devices 2a and 2b may include a heating part 320.

The management devices 2a and 2b may include a conversion flow path FlOa.

The management devices 2a and 2b may include a damper 350.

The shoes care device 1 may include a steam generator 700 and a steam valve 710.

The shoes care device 1 may include a sump 600, a water supply tank 60, and a drain tank 70.

Since all or part of the outer cabinet 20 may be spaced apart from the inner cabinet 100, a predetermined gap may be formed between the inner cabinet 100 and the outer cabinet 20.

In a space between the inner cabinet 100 and the outer cabinet 20, components constituting the shoes care device 1 may be provided, and various flow paths constituting the shoes care device 1 may be provided. In one embodiment of the present invention, part of the connection path F10 may be provided between the inner cabinet 100 and the outer cabinet 20, and part of the regeneration path F20 may be provided between the inner cabinet 100 and the outer cabinet 20.

A dry air duct 370 forming the connection path F10 may be provided between the outer rear plate 21 and the inner rear plate 110. Furthermore, a condenser 400 forming the regeneration path F20 may be provided between the outer rear plate 21 and the inner rear plate 110.

The connection path F10 forms a flow path of a fluid.

The connection path F10 forms a passage through which air and/or condensed water inside the shoes care device 1 moves

The dehumidifying part 330 is disposed inside the connection path F10 and includes a dehumidifying material. The dehumidifying part 330 may be entirely formed of the dehumidifying material, or a part thereof may be formed of the dehumidifying material. A further explanation of the dehumidifying part 330 will be described below.

According to one embodiment of the present invention, the shoes care device 1 has an air circulation structure in which the air inside the inner cabinet 100 in which the shoes are disposed is sucked into the connection path F10 to dehumidify the air using a dehumidifying material 331 and the dehumidified air may be supplied back into the inner cabinet 100.

The connection path F10 may be used as a means to achieve such an air circulation structure in the shoes care device 1. All or part of the connection path F10 may be formed of a pipe, a hose, a tube, a duct, a housing, or a combination thereof.

The module housing 200 according to one embodiment of the present invention forms part of the connection path F10.

The outlet 203 is formed in the module housing 200. The outlet 203 communicates with the accommodation space 101 of the inner cabinet 100, and forms an inlet of the module housing 200 through which the air of the accommodation space 101 of the inner cabinet 100 is suctioned into the module housing 200. The outlet 203 may be disposed below the main shelf 40. In this case, the main shelf 40 may be formed so as not to block the air in the accommodation space 101 from being sucked into the outlet 203. To this end, when the main shelf 40 is formed in a plate shape, a plurality of holes 45 penetrating in the vertical direction may be formed in the main shelf 40 so as to move the air.

In the shoes care device 1, the inner cabinet 100 and the machine room 50 may form a space separated from each other. Furthermore, the module housing 200 that is part of the management devices 2a and 2b may be provided between the inner cabinet 100 and the machine room 50.

The inner cabinet 100, the module housing 200, and the machine room 50 are provided inside the shoes care device 1 according to one embodiment of the present invention.

The inner cabinet 100, the module housing 200, and the machine room 50 may be continuously arranged from the upper side to the lower side. When the shoes care device 1 according to one embodiment of the present disclosure includes the first management device 2a and the second management device 2b, the first management device 2a may be disposed above the second management device 2b. That is, the first management device 2a, the second management device 2b, and the machine room 50 may be continuously arranged from the upper side to the lower side.

Since the first management device 2a and the second management device 2b include the inner cabinet 100 and the module housing 200, respectively, they may be continuously arranged in an order of the inner cabinet 100 of the first management device 2a, the module housing 200 of the first management device 2a, the inner cabinet 100 of the second management device 2b, the module housing 200 of the second management device 2b, the machine room 50 from the upper side to the lower side.

The inner cabinet 100 may form a space that mainly accommodates an article (shoes S) to be managed, and the module housing 200 and the machine room 50 may form a space that mainly accommodates components for an operation of the shoes care device 1.

In the shoes care device 1 according to one embodiment of the present invention, the blowing part 310, the dehumidifying part 330 (and the dehumidifying material 331), and the heating part 320 may be accommodated inside the module housing 200.

In addition, the machine room 50 may be configured to accommodate a controller 10, the sump 600, the steam generator 700, and the steam valve 710 therein. Furthermore, the machine room 50 may be configured to accommodate the water supply tank 60 and the drain tank 70.

Among the components constituting the management devices 2a and 2b, components not included in the module housing 200 may be fixedly coupled to the inner cabinet 100 and the outside of the module housing 200 or may be fixedly coupled to the main frame 5.

Components coupled to or accommodated in the machine room 50 may be fixedly coupled to the machine room 50.

The machine room 50 may include a first wall 51.

A first wall 51 forms one wall surface of the machine room 50. The first wall 51 may be erected in the vertical direction, or may be erected in the substantially vertical direction. In one embodiment, the first wall 51 may form a wall surface orthogonal to or inclined with the first direction (X direction).

The first wall 51 may form a front wall surface of the machine room 50, a left wall of the machine room 50, or a right wall of the machine room 50.

The machine room 50 may include a second wall 52 and a third wall 53. The second wall 52 and the third wall 53 form opposite wall surfaces facing each other in the machine room 50. The second wall 52 and the third wall 53 may be erected in the vertical direction, or may be erected in the substantially vertical direction.

When the first wall 51 forms a front wall of the machine room 50, the second wall 52 may form a left wall of the machine room 50, and the third wall 53 may form a right wall of the machine room 50.

The first wall 51 may be formed integrally with the inner cabinet 100, and the second wall 52 and the third wall 53 may be formed integrally with the outer cabinet 20, respectively.

Each of the water supply tank 60 and the drain tank 70 may be formed in the form of a container for accommodating water.

The water supply tank 60 may be configured to store water supplied into the shoes care device 1 inside. The water supply tank 60 may be configured to store water supplied into the steam generator 700 inside.

In order to supply water from the water supply tank 60 into the shoes care device 1, a water pump 61 may be connected to the water supply tank 60. The water supply tank 60 for moving water and the first water pump 61 may be connected by a pipe, a hose, etc.

The drain tank 70 may be configured to store water discharged from the shoes care device 1 inside. The drain tank 70 may store water condensed inside the shoes care device 1. The drain tank 70 may be configured to store water drained from the sump 600.

In order to discharge water to the drain tank 70, a water pump 71 (a second water pump) may be connected to the drain tank 70. The drain pipe 70 for moving water and the second water pump 71 may be connected by a pipe, a hose, etc.

The water supply tank 60 and the drain tank 70 may be coupled to the machine room 50 to get exposed from the outside of one wall surface of the machine room 50.

The water supply tank 60 and the drain tank 70 may be disposed in front of the machine room 50.

The water supply tank 60 and the drain tank 70 may form one wall surface of the machine room 50 along with the first wall 51. When the first wall 51 forms a front surface of the machine room 50, the water supply tank 60 and the drain tank 70 may be exposed from a front side of the machine room 50, and may be coupled to the machine room 50 to get exposed from the outside of the first wall 51.

As the water supply tank 60 and the drain tank 70 are exposed to the outside of the first wall 51, a user may inject water into the water supply tank 60 or discharge water from the drain tank 70.

The water supply tank 60 and the drain tank 70 may be configured to be detachable from the machine room 50. The water supply tank 60 and the drain tank 70 may be detached from the first wall 51. In order to facilitate the attachment and detachment of the water supply tank 60 and the drain tank 70, a handle 60a of the water supply tank 60 may be formed on an outer surface of the water supply tank 60, and a handle 70a of the drain tank 70 may be formed on an outer surface of the drain tank 70.

Each of the water supply tank 60 and the drain tank 70 may be configured to be separated from the machine room 50 in an outer direction of the first wall 51.

The controller 10 may be configured to control operations of each component in connection with each component constituting the shoes care device 1.

In order to control the controller 10, the shoes care device 1 may be provided with a storage medium in which an application program is stored, and the controller 10 may be configured to control the shoes care device 1 by driving an application program according to information input to the shoes care device 1 and information output from the shoes care device 1.

The controller 10 may control the first management device 2a and the second management device 2b constituting the shoes care device 1 to operate individually. The controller 10 may control the first management device 2a and the second management device 2b to operate in different states, and may control shoes (e.g., sneakers) in the first management device 2a and shoes (e.g., heels) in the second management device 2b to be managed under different conditions. Furthermore, the controller 10 may control the first management device 2a and the second management device 2b to interwork with each other.

The door 30 may be disposed on either the inner cabinet 100 or the machine room 50 on the same side as the first wall 51. When the door 30 forms the front surface of the shoes care device 1, the first wall 51 forms the front surface of the machine room 50, and the door 30 is disposed directly outside the first wall 51.

In one embodiment of the present invention, the door 30 may be configured to open and close the inner cabinet 100 and further expose or shield the front surface of the machine room 50.

The door 30 may be configured to expose or shield the inner cabinet 100, the water supply tank 60, and the drain tank 70.

As described above, in the shoes care device 1, the door 30, the water supply tank 60, and the drain tank 70 are formed on the same side, and when the door 30 is opened, the water supply tank 60 and the drain tank 70 may be exposed and separated from the shoes care device 1.

In the arrangement explained above, even if left and right sides and a rear side of the shoes care device 1 are blocked by other goods or structures, the door 30 may be opened on a front side of the shoes care device 1, and the water supply tank 60 and the drain tank 70 can be separated from or coupled again to the shoes care device 1.

A control panel 33 for controlling the shoes care device 1 is provided on an outer side of the door 30. The control panel 33 may be formed of a touch screen. A control unit (controller 10) is provided in the inner space of the door 30 to control each component of the shoes care device 1 in connection with the control panel 33. The controller 10 may be provided inside the machine room 50.

As illustrated in FIGS. 1a and 1b, in one embodiment, the door 30 may be configured to simultaneously expose or shield the inner cabinet 100 and the machine room 50.

In another embodiment, the door 30 may be configured to open and close only the inner cabinet 100. In this case, the machine room 50 may not be shielded by the door 30. Furthermore, in this case, the shoes care device 1 according to one embodiment of the present invention may be further provided with a dedicated door of the machine room 50 to open and close the machine room 50 separately from the door 30.

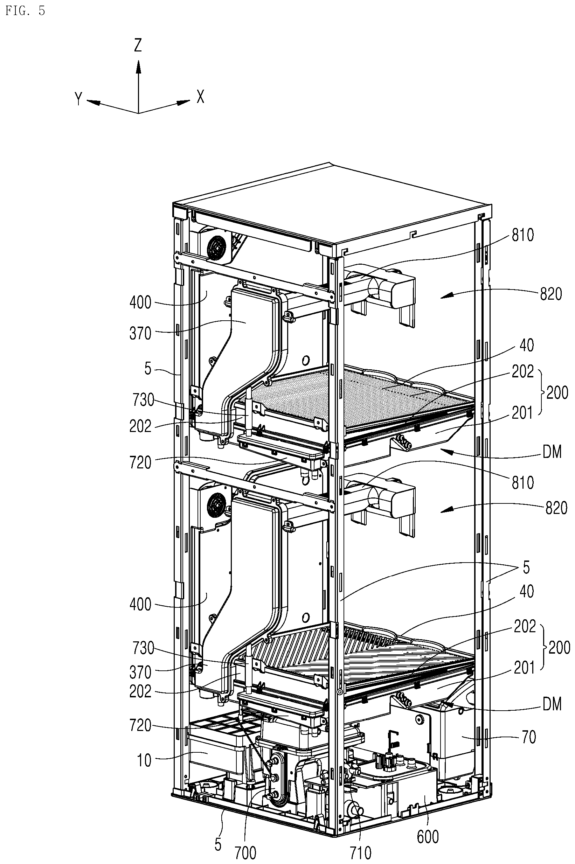

FIG. 5 is a perspective view illustrating a state in which the door 30, the outer cabinet 20, and the inner cabinet 100 are removed from the shoes care device 1 according to one embodiment of the present invention.

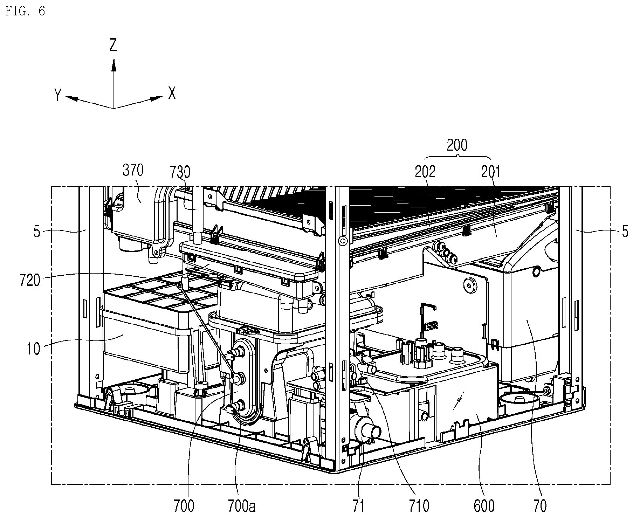

FIG. 6 is a perspective view illustrating a machine room part of the shoes care device 1 illustrated in FIG. 5.

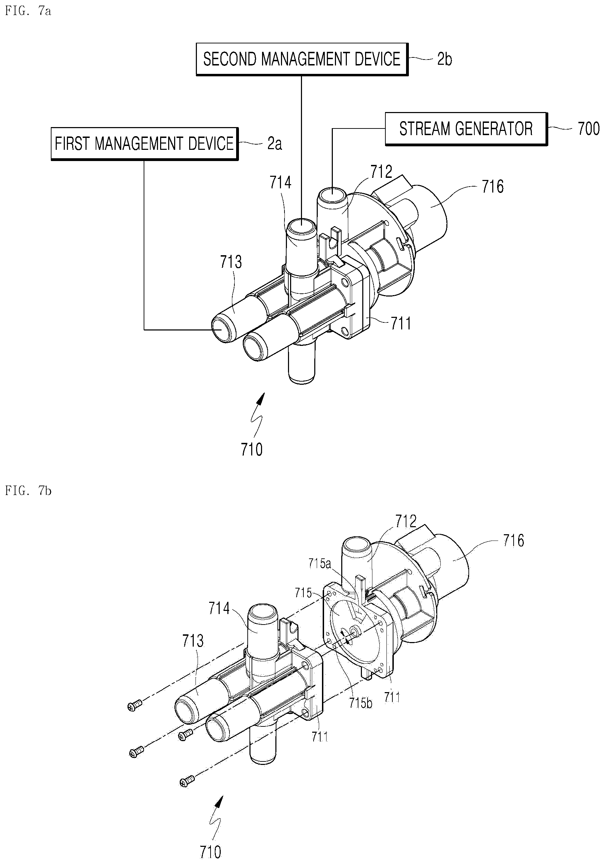

FIG. 7a is a view illustrating the steam valve 710 according to one embodiment of the present invention, and illustrating a connection relationship considering the movement of steam. FIG. 7b is an exploded perspective view illustrating the steam valve illustrated in FIG. 7a.

The shoes care device 1 is provided with the steam generator 700 as a device configured so as to generate moisture inside the inner cabinet 100. The steam generator 700 may be provided inside the machine room 50. The steam generator 700 is configured to generate steam and selectively supply moisture and steam to the inside of the inner cabinet 100.

The shoes care device 1 according to one embodiment may be provided with one steam generator 700, and the shoes care device 1 according to another embodiment may be provided with two or more steam generators 700.

When the shoes care device 1 is provided with one steam generator 700, the steam generator 700 may be configured to supply steam into the inner cabinet 100 of the first management device 2a and/or into the inner cabinet 100 of the second management device 2b.

When the shoes care device 1, one steam generator 700 may be provided with two or more steam generators 700, one of the steam generators 700 may supply steam to the inner cabinet 100 of the first management device 2a, and the other steam generator 700 may supply steam to the inner cabinet 100 of the second management device 2b.

Moist air formed by the steam generator 700 ('air' described in one embodiment of the present invention may be 'air including moisture') is supplied toward the accommodation space 101 of the inner cabinet 100, and moisture may be circulated in the accommodation space 101 of the inner cabinet 100, and accordingly, the moisture may be supplied to the shoes S.

The shoes care device 1 according to one embodiment of the present invention may be a refresher device that refreshes the shoes.

Here, refreshing may refer to a process of removing contaminants, deodorizing, sanitizing, preventing static electricity, or warming by supplying air, heated air, water, mist, steam, etc. to the shoes.

The steam generator 700 may supply steam to the accommodation space 101 of the inner cabinet 100 in which the shoes S are accommodated, and may perform steam treatment on the shoes, which is further meant to exert a refreshing effect due to swelling of shoes materials as well as sterilization by high-temperature steam.

The steam generator 700 is provided with a separate heater 700a that heats an inner space and water in the inner space and is configured to heat water to generate steam and supply the heated water to the accommodation space 101 of the inner cabinet 100.

An external faucet, etc., as a water supply source for supplying water to the steam generator 700, may be used, or a container-type water supply tank provided on one side of the machine room 50 may be used. The steam generator 700 may generate steam by receiving water from the water supply tank 60.

The water supply tank 60 for the movement of water and the steam generator 700 may be connected by a pipe, a hose, etc.

The steam generator 700 for the movement of steam and the inner cabinet 100 may be connected by a pipe, a hose, etc. In the shoes care device 1 according to one embodiment of the present invention, as described below, the steam generated by the steam generator 700 may be supplied into the inner cabinet 100 after passing through the steam valve 710 and a steam separator 720. In this case, in order to move the steam, the steam generator 700 and the steam valve 710 may be connected by a pipe, a hose, etc., and the steam valve 710 and the steam separator 720 may also be connected by a pipe, a hose, etc.

The steam valve 710 may be disposed adjacent to the steam generator 700, and the steam valve 710 may be provided in the machine room 50. The steam generator 700 and the steam valve 710 may be provided below the second management device 2b.

The steam valve 710 is configured to selectively communicate with each of the accommodation space 101 of the first management device 2a and the accommodation space 101 of the second management device 2b, respectively.

When the steam of the steam generator 700 is supplied to the accommodation space 101 of the first management device 2a and/or the accommodation space 101 of the second management device 2b, the steam valve 710 operates to control whether the stream is supplied or not, and an operation of the stream 710 is driven by the controller 10.

The steam valve 710 includes a valve housing 711, a valve inlet 712, a first valve outlet 713, a second valve outlet 714, a valve disk 715, and a valve motor 716. In the steam valve 710 illustrated in FIGS. 7a and 7b, the other outlets except the valve inlet 712, the first valve outlet 713, and the second valve outlet 714 may be blocked by a stopper and deactivated.

The valve housing 711 forms a body of the steam valve 710, and has a predetermined inner space formed inside.

The valve inlet 712 may have a tubular shape and be coupled to the valve housing 711 to communicate with the inner space of the valve housing 711. The valve inlet 712 is a part connected to the steam generator 700, and steam may flow into the steam valve 710 (inside the valve housing 711) through the valve inlet 712.

The first valve outlet 713 and the second valve outlet 714 may be formed in a tubular shape and be coupled to the valve housing 711 to communicate with the inner space of the valve housing 711.

The first valve outlet 713 and the second valve outlet 714 are outlets through which steam is discharged from the steam valve 710. The first valve outlet 713 is connected to the accommodation space 101 of the first management device 2a, and the second valve outlet 714 is connected to the accommodation space 101 of the second management device 2b.

The valve disk 715 is provided inside the steam valve 710 (inside the valve housing 711) and is configured to open and close a flow path inside the steam valve 710. The valve disk 715 may be configured to selectively open and close a flow path of the first valve outlet 713 and a flow path of the second valve outlet 714.

The valve disk 715 is disposed between the valve inlet 712 and the first valve outlet 713, or between the valve inlet 712 and the second valve outlet 714, inside the valve housing 711. The valve disk 715 allows the valve inlet 712 and the first valve outlet 713 to communicate with each other or block the communication therewith, and also allows the valve inlet 712 and the second valve outlet 714 to communicate with each other or block the communication therewith.

In one embodiment of the present invention, the valve disk 715 may be formed in a circular plate shape and may include a valve hole 715a. The valve hole 715a is a hole penetrating the valve disk 715. The valve disk 715 may be rotatably coupled to the valve housing 711 around a valve rotation axis 715b, and the valve hole 715a may be formed eccentrically from the valve rotation axis 715b.

The valve motor 716 is coupled to the valve housing 711, and the valve motor 716 is coupled to the rotation axis 715b of the valve disk 715 to rotate the valve disk 715.

The controller 10 may control the steam valve 710 by controlling an operation of the valve motor 716.

The valve disk 715 rotates by the operation of the valve motor 716, and the valve disk 715 opens or closes a flow path inside the steam valve 710 (inside the valve housing 711) depending on the degree of rotation of the valve disk 715.

In one embodiment, depending on the degree of rotation of the valve disk 715, when the valve inlet 712 and the first valve outlet 713 communicate with each other through the valve hole 715a, the valve inlet 712 and the second valve outlet 714 are blocked from communicating with each other by the valve disk 715, or when the valve inlet 712 and the second valve outlet 714 communicate with each other through the valve hole 715a, the valve outlet 712 and the first valve outlet 713 may be blocked from communicating with each other by the valve disk 715.

In another embodiment, depending on the degree of rotation of the valve disk 715, the valve inlet 712 may communicate with both the first valve outlet 713 and the second valve outlet 714, or the valve inlet 712 may be blocked from communicating with both the first valve outlet 713 and the second valve outlet 714.

In the shoes care device 1 according to one embodiment of the present invention, the valve disk 715 may close a flow path of the second valve outlet 714 while opening a flow path of the first valve outlet 713, and may also close the flow path of the first valve outlet 713 and open the flow path of the second valve outlet 714.

The steam valve 710 made as described above may operate such that only the valve inlet 712 and the first valve outlet 713 communicate with each other, or only the valve inlet 712 and the second valve outlet 714 communicate with each other.

In the arrangement explained above, all the steam generated by the steam generator 700 may be supplied to the accommodation space 101 of the first management device 2a, or may be supplied to the accommodation space 101 of the second management device 2b. In this case, the steam generated by the steam generator 700 may be supplied to the accommodation space 101 of the first management device 2a or the accommodation space 101 of the second management device 2b without a pressure drop, and even when only one steam generator 700 is provided in the shoes care device 1, the steam may be sufficiently and stably supplied to the accommodation spaces 101 of each of the two inner cabinets 100.

In one embodiment of the present invention, the controller 10 may control the stream valve 710 such that when the heating part 320 of the first management device 2a is turned off (when a heater 321 of the heating part 320 is turned off), the valve disk 715 closes or opens the first valve outlet 713, and when the heating part 320 of the first management device 2a is turned on (when the heater 321 of the heating part 320 is turned on), the valve disk 715 closes the first valve outlet 713.

In addition, the controller 10 may control the stream valve 170 such that when the heating part 320 of the second management device 2b is turned off (when the heater 321 of the heating part 320 is turned off), the valve disk 715 closes or opens the second valve outlet 714, and when the heating part 320 of the second management device 2b is turned on (when the heater 321 of the heating part 320 is turned on), the valve disk 715 closes the second valve outlet 714.

In the shoes care device 1 according to one embodiment of the present invention, by controlling the steam valve 710 by the controller 10, the steam generated in the steam generator 700 may be selectively or simultaneously supplied to the accommodation space 101 of the first management device 2a and the accommodation space 101 of the second management device 2b, and whether the steam is supplied or not may be controlled according to a use state of the shoes care device 1.

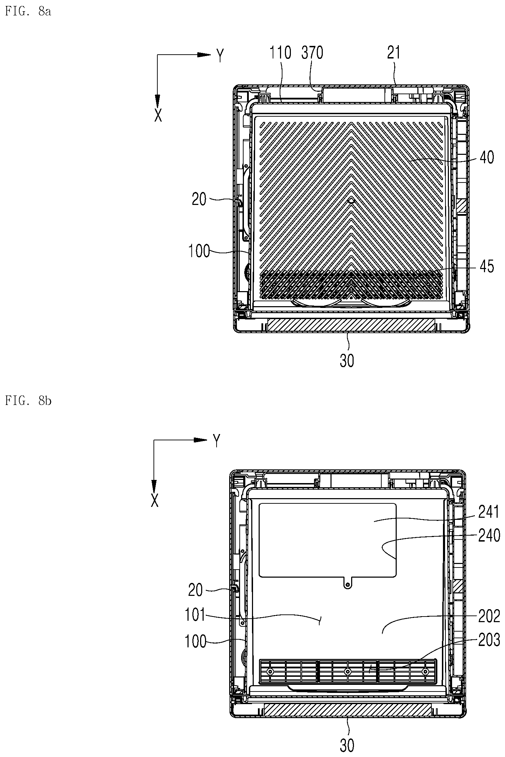

FIG. 8a is a cross-sectional view taken along line D-D' of the shoes care device 1 illustrated in FIG. 3.

FIG. 8b is a view illustrating a state in which the main shelf 40 is removed from the shoes care device 1 illustrated in FIG. 8a.

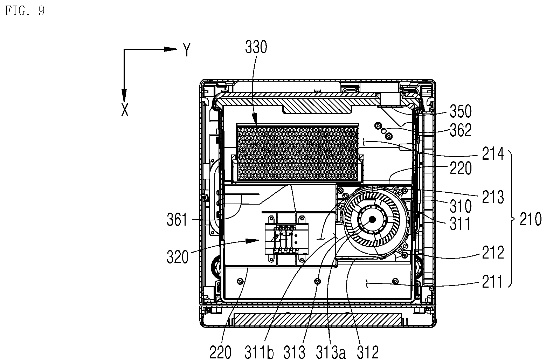

FIG. 9 is a cross-sectional view taken along line E-E' of the shoes care device 1illustrated in FIG. 3.

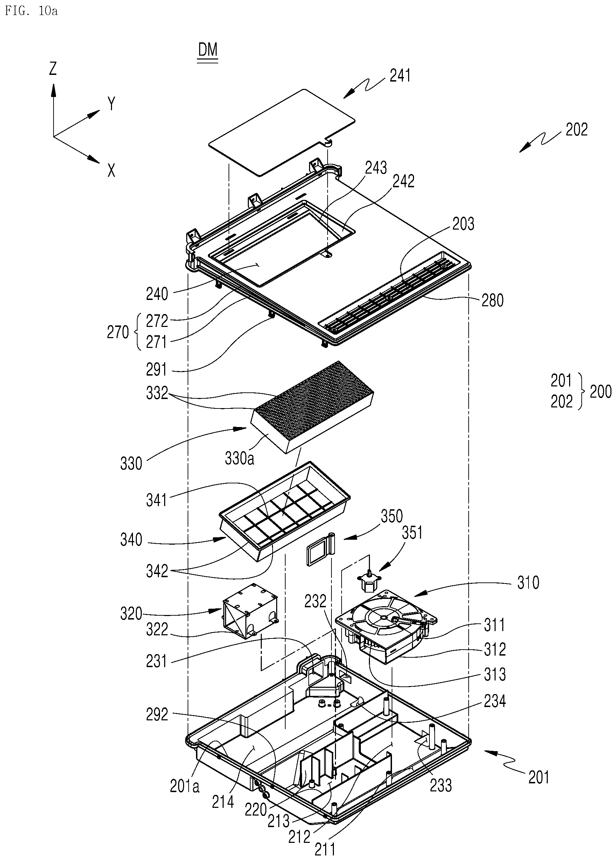

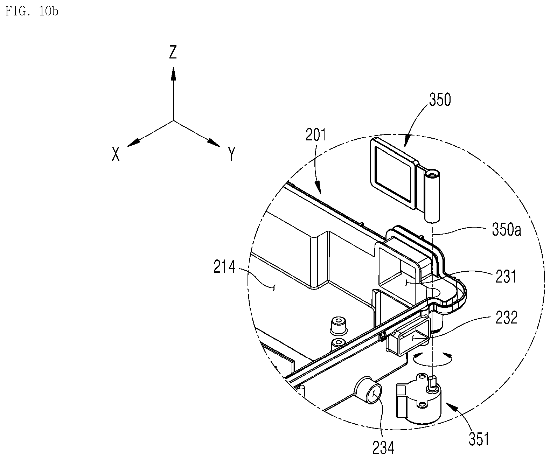

FIG. 10a is an exploded perspective view illustrating a drying module in the shoes care device 1 according to one embodiment of the present invention. FIG. 10b is an exploded perspective view illustrating a partial configuration of the drying module at a part to which the damper 350 is coupled.

In the shoes care device 1 according to one embodiment of the present invention, the dehumidifying part 330 may be used as a means that dehumidifies air

As described above, the dehumidifying part 330 may be provided inside the module housing 200.

The dehumidifying part 330 is configured to have a predetermined volume. The dehumidifying part 330 may be configured to be porous by itself. A plurality of pores may be formed over an entire volume of the dehumidifying part 330, and air may move by penetrating the dehumidifying part 330 through such pores.

When the dehumidifying part 330 is composed of a combination of a plurality of dehumidifying materials, the plurality of dehumidifying materials may be fixed to each other by a separate fixing means, or may be fixed to each other by adhesion.

The dehumidifying part 330 may be formed of dehumidifying materials, and may be configured to include the dehumidifying materials.

The dehumidifying material 331 according to one embodiment of the present invention is configured to include a material capable of reducing humidity by absorbing moisture in the air. The dehumidifying material 331 may be formed of various materials or a combination of the materials within the range of absorbing or adhering the moisture in the air, and may be formed in various shapes and structures.

The dehumidifying material 331 according to one embodiment of the present invention may be referred to as a desiccant or an adsorbent.

The dehumidifying material 331 according to one embodiment of the present invention may be formed of a microporous material. The dehumidifying material 331 according to one embodiment of the present invention may include silica gel, activated carbon, activated alumina (AL2O3), diatomaceous earth, etc.

Specifically, the dehumidifying material 331 according to one embodiment of the present invention may be formed of zeolite or may be configured to include zeolite.

The zeolite is a natural and synthetic silicate mineral in which tunnels or open channels having a size of approximately 3 to 10 angstroms (Å) are regularly arranged, and may function as dehumidification by adsorbing the moisture in the air

When the zeolite is heated, moisture adsorbed on the zeolite may be separated into a large amount of steam. According to the characteristics of the zeolite, the zeolite may be regenerated in a state capable of not only performing the dehumidification function to remove the moisture from the air but also performing the dehumidification function by heating the zeolite and separating the moisture adsorbed to the zeolite.

The zeolite may be formed in the form of small grains (or stones) having the size (diameter) of several micrometers to several tens of micrometers, and the dehumidifying material 331 described in one embodiment of the present invention may refer to a combination of the grains (or stones). Each of the grains (or stones) may be agglomerated or combined with each other to form a single structure.

In another embodiment, the dehumidifying part 330 may include a dehumidifying body 330a and the dehumidifying material 331.

The dehumidifying body 330a may be formed to have a predetermined volume. In one embodiment, the dehumidifying body 330a may be formed in a substantially hexahedral shape.

In order to allow air to move through the dehumidifying body 330a, the dehumidifying body 330a may be provided with a plurality of dehumidification through holes 332 penetrated in one direction. A cross section of the dehumidifying through hole 332 may be formed in a circular shape, a polygonal shape, etc. The dehumidifying through hole 332 may have a hexagonal cross section.

In the dehumidifying body 330a, the dehumidifying through hole 332 may all have the same shape and size, or may have different shapes and sizes.

The dehumidifying body 330a may be formed of or include materials such as synthetic resin, metal, ceramic, etc. The dehumidifying body 330a may be formed of a combination of fibers, and may be formed of a nonwoven fabric, etc.

The dehumidifying material 331 may be coated on the dehumidifying body 330a. The dehumidifying material 331 may be coated on the outside and inside of the dehumidifying body 330a. Specifically, the dehumidifying material 331 may be coated on a surface in which the dehumidifying through hole 332 is formed.

When the dehumidifying body 330a is formed of a combination of fibers, the zeolite may be first coated on each fiber as the dehumidifying material 331, and the zeolite-coated fiber may be processed to form the dehumidifying body 330a and simultaneously form the dehumidifying part 330.

Regarding the coating of the zeolite, a manufacturing method of a zeolite coated ceramic paper (Korean Patent No. 10-1004826) is known, and Korean Patent No. 10-1173213 and Korean Patent No. 10-0941521 also describes a method of coating zeolite on a surface of a material. The dehumidifying part 330 according to one embodiment of the present invention may be formed by coating the gelled zeolite precursor on the dehumidifying body 330a or materials constituting the dehumidifying body 330a and then performing heat treatment when the dehumidifying material 331 is formed of the zeolite.

In one embodiment of the present invention, the coating of the dehumidifying material 331 (zeolite) may be performed by using various known or possible methods, and is not limited to a certain manufacturing method related to the coating of the dehumidifying material 331.

The blowing part 310 is provided inside the connection path F10. A blowing fan 313 may be provided inside the blowing part 310. Since the blowing part 310 is provided in the connection path F10, air flows in the connection path F10 when the blowing part 310 is driven, and since the connection path F10 also communicates with the accommodation space 101 of the inner cabinet 100, the air flows and moves in the accommodation space 101 by driving the blowing part 310.

In this way, the air may be sucked from the inner cabinet 100 into the connection path F10 by the driving of the blowing part 310 (a rotation of the blowing fan 313), and the air inside the connection path F10 may be ventilated.

In one embodiment, the blowing part 310 is provided inside the module housing 200 forming the connection path F10, and the blowing part 310 is driven to suction the air inside the inner cabinet 100 into an inlet 203. The air inside the connection path F10 passes through the module housing 200 constituting the connection path F10, the dry air duct 370, and a nozzle duct 810 and is then discharged back into the inner cabinet 820.

As such, the flow of air may be generated in the shoes care device 1 by the driving of the blowing part 310.

Dry air may be supplied to the inside of the inner cabinet 100 by the blowing part 310.

The heating part 320 is provided at one side of the dehumidifying part 330 and the blowing part 310 in the connection path F10. The heating part 320 may be provided inside the module housing 200. Based on a movement direction of the air inside the module housing 200, the module housing 200 may be arranged in an order of the blowing part 310, the heating part 320, and the dehumidifying part 330. That is, the air introduced into the outlet 203 of the module housing 200 moves along the connection path F10 by passing sequentially through the blowing part 310, the heating part 320, and the dehumidifying part 330.

The heating part 320 is disposed inside the module housing 200 and is configured to heat the air of the module chamber 210 inside the module housing 200.

The heating part 320 may be configured to heat the dehumidifying part 330. The heating part 320 may be configured to heat the dehumidifying material 331 constituting the dehumidifying part 330.

The air heated by the heating part 320 by the driving of the blowing part 310 moves directly to the dehumidifying part 330, thus heating the dehumidifying part 330. To this end, the heating part 320 is disposed inside the module housing 200 adjacent to the dehumidifying part 330. Specifically, the heating part 320 is disposed inside the module housing 200 adjacent to the dehumidifying part 330 based on a movement path of the air inside the module housing 200.

In the module housing 200, the dehumidification by the dehumidifying material 331 or the regeneration of the dehumidifying material 331 may be achieved by selectively heating the heating part 320.

The heating part 320 may be fixedly coupled to the module housing 200 in the module housing 200.

The heating part 320 may be made up of various devices and structures within a range capable of heating the air inside the module housing 200 or supplying heat to the dehumidifying part 330.

The heating part 320 may be formed of an electric heater 321. In one embodiment of the present invention, the heating part 320 may include the heater 321. The heater 321 includes a heating element, and may be configured to supply heat to the periphery while the heating element generates heat by supplied electric energy. The heater 321 may include a nichrome wire as the heating element.

The heater 321 of the heating part 320 may be formed in a ring shape, and the air may move by penetrating the center and surroundings of the ring-shaped heater 321 and be simultaneously heated. The heater 321 of the heating part 320 may be repeatedly formed in a second module chamber 213 along the movement direction of air.

The heater 321 of the heating part 320 may be formed in a circular ring shape or a rectangular ring shape.

The heating part 320 may include a heater flange 322 to which the heater 321 is fixed

The heater flange 322 may be formed in the form of a metallic plate.

The heater flange 322 may be formed of a combination of flat plates in the second module chamber 213 along the movement direction of air. The heater flange 322 has a cross section that may be formed of a plate shape or a combination of plates in the second module chamber 213 along the movement direction of air (the second direction (Y direction)).

The heater flange 322 may include an outer flange 322a and an inner flange 322b.

The outer flange 322a may be formed in a tubular shape along the second direction (Y direction). An interior of the outer flange 322a is provided with a space to move air along the movement direction of air (a direction parallel to the second direction (Y direction)) in the second module chamber 213.

The inner flange 322b is fixed to the interior of the outer flange 322a. The inner flange 322b may include two or more plates crossing each other, and the heater 321 of the heating part 320 may be fixed to the inner flange 322b.

The heater flange 322 may be formed in various forms that fix the heater 321 of the heating part 320 and do not interfere with a flow of air moving through the second module chamber 213.

In one embodiment of the present invention, the blowing part 310, the heating part 320, the dehumidifying part 330, and the module housing 200 may form one set.

The set may be provided in a plural form. The shoes care device 1 according to one embodiment may be provided with two sets.

Such a set may be provided in each of the first management device 2a and the second management device 2b.

Such a set may form a drying module DM in the shoes care device 1 according to one embodiment of the present invention.

That is, in one embodiment of the present invention, the drying module DM may include the module housing 200, the blowing part 310, the heating part 320, and the dehumidifying part 330. Furthermore, the drying module DM is provided in each of the first management device 2a and the second management device 2b.

In the shoes care device 1 according to one embodiment of the present invention, a plurality of drying modules DM may be provided.

In the shoes care device 1 according to one embodiment of the present invention, a pair of drying modules DM may be provided. When the shoes care device 1 is provided with the pair of drying modules DM, one of the drying modules DM may form a 'drying module A (DM1)' as a drying module of the first management device 2a, and the other may form a 'drying module B (DM2) ' as a drying module of the second management device 2b.

The 'drying module' described in one embodiment of the present invention may be understood to refer to each of the 'drying module A' and the 'drying module B' except as otherwise particularly limited.

In the shoes care device 1 according to one embodiment of the present invention, the drying module A (DM1) and the drying module B (DM2) may operate in different modes. When the drying module A DM1 operates in a moisture absorption mode, the drying module B DM2 may operate in a regeneration mode. Conversely, when the drying module A DM1 operates in the regeneration mode, the drying module B DM2 may operates in the moisture absorption mode.

The 'moisture absorption mode' described in the present invention means a case in which the dehumidifying part 330 adsorbs moisture in the air, and the 'regeneration mode' means a case in which the moisture adsorbed to the dehumidifying part 330 is separated by heating the dehumidifying part 330.

Naturally, both the drying module A DM1 and the drying module B DM2 may operate in the moisture absorption mode or may operate in the regeneration mode.

The module housing 200 may be fixedly coupled to a lower side of the inner cabinet 100. The module housing 200 may be detachably coupled to a lower side of the inner cabinet 100.

The module housing 200 includes the module chamber 210 that is a space having other components accommodated inside. That is, the module chamber 210 is a space inside the module housing 200 distinguished from an external space of the module housing 200. As described above, the module housing 200 forms part of the connection path F10, and accordingly, the module chamber 210 is configured to communicate with a space outside the module housing 200. The module chamber 210 communicates with the accommodation space 101 of the inner cabinet 100.

The module housing 200 may include a module case 201 and a module cover 202.

The module case 201 and the module cover 202 may be formed by injection molding, respectively, and may be assembled with each other after manufacturing to form the module housing 200.

The module case 201 is formed in the form of a container that is concave substantially downwards, and forms the module chamber 210 of the module housing 200.

The module case 201 may be configured in the form of a container opened upwards, and includes a module opening 201a.

In a plan view, an area of the module opening 201a may be larger than or equal to an area of the module chamber 210.

The module chamber 210 may include a first module chamber 212, a second module chamber 213, and a third module chamber 214. The module chamber 210 may include a suction module chamber 211.

In order to distinguish between the suction module chamber 211, the first module chamber 212, the second module chamber 213, and the third module chamber 214, a module partition wall 220 may be formed inside the module housing 200. Furthermore, the module partition wall 220 guides the movement of air so that air moves in a predetermined direction inside the module housing 200.

The suction module chamber 211 is a first space where air is introduced into the module housing 200.

The first module chamber 212 is a space in which the blowing part 310 is accommodated, the second module chamber 213 is a space in which the heating part 320 is accommodated, and the third module chamber 214 is a space in which the dehumidifying part 330 is accommodated.

In one embodiment of the present invention, the suction module chamber 211, the first module chamber 212, the second module chamber 213, and the third module chamber 214 may be formed at different positions in a plan view.

In addition, the air of the module chamber 210 may be configured to move the suction module chamber 211, the first module chamber 212, the second module chamber 213, and the third module chamber 214 sequentially. That is, when the blowing part 310 is driven, air moves sequentially through the suction module chamber 211, the first module chamber 212, the second module chamber 213, and the third module chamber 214 inside the module housing 200.

The module case 201 may include a dry air outlet 231 and a wet air outlet 232.

The dry air outlet 231 may be formed in a hole shape opened to allow air in the third module chamber 214 to flow out. The dry air outlet 231 is formed adjacent to the third module chamber 214. The dry air outlet 231 may be formed on one edge of the module case 201. Furthermore, the dry air outlet 231 may be connected to the accommodation space 101 through the dry air duct 370 and the nozzle duct 810.

The wet air outlet 232 may be formed in a hole shape opened to allow the air in the third module chamber 214 to flow out. The wet air outlet 232 is formed adjacent to the third module chamber 214. The wet air outlet 232 may be formed on a frame on one side of the module case 201. Furthermore, the wet air outlet 232 may be connected to the condenser 400.

The dry air outlet 231 and the wet air outlet 232 may be formed adjacent to each other. The dry air outlet 231 and the wet air outlet 232 may be formed adjacent to any one vertex part of the module housing 200.

The suction module chamber 211 is formed adjacent to the first module chamber 212, and a bottom surface of the suction module chamber 211 may be inclined downwardly toward the first module chamber 212. Accordingly, air introduced into the suction module chamber 211 may naturally move toward the first module chamber 212 by hitting the bottom surface of the suction module chamber 211 forming an inclined surface, and a condensed water introduced into the suction module chamber 211 may move along the bottom surface of the suction module chamber 211 forming the inclined surface, and may move to the first module chamber 212.

The blowing part 310 may be assembled to the module housing 200 while being spaced apart from a bottom surface of the first module chamber 212. Furthermore, in this case, the blowing part 310 may be configured such that air may be introduced from a lower side of the first module chamber 212 to an interior of the blowing part 310 inside the first module chamber 212.

The module case 201 may include a first condensed water discharge hole 233.

The first condensed water discharge hole 233 is formed in a hole shape penetrating the module case 201. The first condensed water discharge hole 233 is formed on an edge of the module case 201 adjacent to a condenser 400 and formed to be equal to or lower than the bottom surface of the first module chamber 212, and communicates with the condenser 400. Among the bottom surfaces of the first module chamber 212, the first condensed water discharge hole 233 may form the lowest part, or the bottom surface of the first module chamber 212 may be formed such that a height thereof is lowered toward or at least equal to the first condensed water discharge hole 233.

As such, the first condensed water discharge hole 233 may be lower than the bottom surface of the first module chamber 212, and accordingly, the condensed water introduced into the first condensed water discharge hole 232 may move toward the first condensed water discharge hole 233 and flow into the condenser 400 through the first condensed water discharge hole 233.

Meanwhile, since the first condensed water discharge hole 233 is a hole in which the module housing 200 and the condenser 400 communicate with each other, the air inside the condenser 400 may flow into the module housing 200 through the first condensed water discharge hole 233. The air introduced into the module housing 200 through the first condensed water discharge hole 233 from an interior of the condenser 400 may move along the first module chamber 212, the second module chamber 213, and the third module chamber 214 by an operation of the blowing part 310 and may be introduced again into the condenser 400 and condensed.

The shoes care device 1 according to one embodiment of the present invention includes the condenser 400 coupled to an outer surface of the inner cabinet 100 and forming a regeneration path F20. In a plan view, the first module chamber 212 may be provided between the second module chamber 213 and the condenser 400. Since the first module chamber 212 is disposed between the second module chamber 213 and the condenser 400, a direct heat exchange between the condenser 400 and the heating part 320 is blocked, and the heat may be prevented from being transferred to the condenser 400 when the heating part 320 is heated inside the second module chamber 213.

Accordingly, when the dehumidifying part 330 is regenerated, condensation depending on heating of air by the heater 321 of the heating part 320 and cooling of air inside the condenser 400 may be effective performed.

The dehumidifying part 330 may be coupled to the module housing 200 while being spaced apart from a bottom surface of the third module chamber 214. Furthermore, in this case, the air inside the third module chamber 214 may move downwards through the dehumidifying part 330 from an upper side of the third module chamber 214.

The module case 201 may include a second condensed water discharge hole 234.

The second condensed water discharge hole 234 is formed in a hole shape penetrating the module case 201. The second condensed water discharge hole 234 is formed on the edge of the module case 201 adjacent to the condenser 400 and formed to be equal to or lower than the bottom surface of the third module chamber 214, and communicates with the condenser 400. Among the bottom surfaces of the third module chamber 214, the second condensed water discharge hole 234 may form the lowest part, or the bottom surface of the third module chamber 214 may be formed such that a height thereof is lowered toward or at least equal to the second condensed water discharge hole 234.

The second condensed water discharge hole 234 may be formed adjacent to the wet air outlet 232.

In this way, the second condensed water discharge hole 234 may be lower than the bottom surface of the third module chamber 214, and accordingly, condensed water introduced into the third module chamber 214 may move toward the second condensed water discharge hole 234, and may flow into the condenser 400 through the second condensed water discharge hole 234.

Meanwhile, since the second condensed water discharge hole 234 is a hole in which the module housing 200 and the condenser 400 communicate with each other, the air inside the condenser 400 may flow into the module housing 200 through the second condensed water discharge hole 234. In this way, the air introduced from the interior of the condenser 400 into the module housing 200 through the second condensed water discharge hole 234 moves directly to the wet air outlet 232 by the driving of the blowing part 310, and may be introduced again into the condenser 400 and condensed.

The module housing 200 may include the module cover 202.

The module cover 202 is coupled to the module case 201 while shielding the module opening 201a from an upper side of the module case 201. The module cover 202 may be detachably coupled to the module case 201. A plurality of locking projections 292 may protrude from one of the module cover 202 and the module case 201, and a plurality of locking grooves 291 into which the locking projections 292 are inserted and locked may be formed on the other. The locking projections 292 and locking grooves 291 are provided in a plural form, respectively, and may be spaced apart along an edge of the module housing 200 and repeatedly formed.

With the blowing part 310, the heating part 320, and the dehumidifying part 330 accommodated in the module case 201, the module cover 202 may shield the blowing part 310, the heating part 320, and the dehumidifying part 330 and may be coupled to the module case 201.

The shoes care device according to one embodiment of the present invention may be formed in a structure in which the dehumidifying part 330 may be detachable from the module housing 200. The structure of the shoes care device provides an advantageous advantage in maintaining and managing the dehumidifying part 330 and the shoes care device 1 on the whole.

On the other hand, the dehumidifying part 330 may be repeatedly used by regeneration, but with the repeated use, the dehumidifying part 330 needs to be replaced.

Considering these descriptions, the shoes care device 1 according to one specific embodiment of the present invention may be configured to separate and replace the dehumidifying part 330.

In one embodiment of the present invention, the module cover 202 of the module housing 200 may form a bottom surface of the inner cabinet 100.

The module cover 202 may form a boundary surface between the inner cabinet 100 and the module housing 200. The module cover 202 may be formed in a substantially rectangular shape.

The module cover 202 may be configured in substantially parallel with the horizontal direction.

Alternatively, the module cover 202 may be inclined to any one side. In one embodiment, an upper surface of the module cover 202 may be inclined downwardly toward the first direction (X direction) (a front side of the shoes care device 1).

In one embodiment of the present invention, the main shelf 40 is mounted in close contact with an upper side surface of the module cover 202, and the main shelf 40 mounted on the upper side surface of the module cover 202 is also configured to be inclined when the upper side surface of the module cover 202 is inclined. In this case, since an upper surface of the main shelf 40 is inclined, water (e.g., condensed water) placed on the upper surface of the main shelf 40 may flow along an inclined direction.

The shoes care device 1 may include a dehumidifying material cover 241.

The dehumidifying material cover 241 forms part of the module cover 202 that is the bottom of the inner cabinet 100. Furthermore, the dehumidifying material cover 241 may be detached from the module cover 202 of the inner cabinet 100 or may be hinge-coupled to the module cover 202.

In the module cover 202, a dehumidifying material exit 240 which is an opening of a shape and a size corresponding to the dehumidifying material cover 241 may be formed. The dehumidifying material cover 241 may be configured to open and close the dehumidifying material exit 240. The dehumidifying material cover 241 may be tightly coupled to the dehumidifying material exit 240. At least a part of the dehumidifying material cover 241 may be separated from the module cover 202. In one embodiment, the dehumidifying material exit 240 of the module cover 202 may be opened while completely separating the dehumidifying material cover 241 from the module cover 202, and in another embodiment, the dehumidifying material cover 241 of the module cover 202 may be opened while rotating the dehumidifying material cover 241 around a hinge axis. The dehumidifying part 330 may be introduced into or withdrawn from the module housing 200 through the dehumidifying material exit 240.

The dehumidifying material exit 240 and the dehumidifying material cover 241 may be formed in a position corresponding to the third module chamber 214 in a plan view. That is, the dehumidifying material exit 240 and the dehumidifying material cover 241 may be formed directly above the third module chamber 214. The shoes care device 1 according to one embodiment of the present invention may be configured such that the first module chamber 212 and the second module chamber 213 are not exposed in a plan view in a state where the dehumidifier cover 241 is opened.

When the dehumidifying material cover 241 is opened in the module cover 202, the third module chamber 214 disposed on a lower part of the module cover 202 is exposed through the dehumidifying material exit 240 of the module cover 202, and the dehumidifying part 330 may be settled inside the module case 201, or may be immediately withdrawn and separated from the module case 201.

The sizes and shapes of the dehumidifying material cover 241 and the dehumidifying material exit 240 are variously provided within the range capable of withdrawing or inserting the dehumidifying part 330.

The dehumidifying material cover 241 may be formed in a rectangular plate shape.

The length of the dehumidifying material cover 241 in the first direction (X direction) may be equal to or longer than the length of the dehumidifying part 330, and the length of the dehumidifying material cover 241 in the second direction (Y direction) may be equal to or longer than the length of the dehumidifying part 330.

As described above, in the shoes care device 1 according to one embodiment of the present invention, the heating part 320 and the dehumidifying part 330 are formed at different positions in a top plane view, and when the dehumidifying material cover 241 is opened on the module cover 202 that forms the bottom surface of the inner cabinet 100, the dehumidifying part 330 disposed directly below the dehumidifying material cover 241 may be withdrawn from the module housing 200, and the dehumidifying part 330 may be easily replaced by the user

In addition, since only the third module chamber 214 is exposed in a state in which the dehumidifier cover 241 is opened, and the first module chamber 212 and the second module chamber 213 are not exposed, the blowing part 310 accommodated in the first module chamber 212 and the heating part 320 accommodated in the second module chamber 213 are not exposed. That is, since the blowing part 310 and the heating part 320 are not directly exposed to the user, safety accidents due to an unintended operation of the blowing part 310 and/or the heating part 320 can be prevented.

The dehumidifying material cover 241 may be configured to separately shield the dehumidifying part 330. A space between the dehumidifying material cover 241 and the dehumidifying part 330 may form a part of the connection path F10.

As described above, the outlet 203 forms an inlet through which the air inside the inner cabinet 100 is sucked into the module housing 200. The outlet 203 may form a start part of the connection path F10. The outlet 203 may be formed in the shape of a hole vertically penetrated from the bottom surface (the upper surface of the module cover 202) of the inner cabinet 100.

A network such as a grid shape, a mesh shape, etc., may be formed on the outlet 203.

The outlet 203 may be formed parallel to the second direction (Y direction). That is, the outlet 203 may be formed in a long hole shape in the module cover 202 along the second direction (Y direction).

The outlet 203 may be formed on an edge of the module cover 202. The outlet 203 may be formed on the edge of the module cover 202 along the second direction (Y direction).

The outlet 203 may be formed on a front part or a rear part of the module cover 202 based on the first direction (X direction).

The outlet 203 may be disposed relatively close to the door 30 in the module cover 202. That is, the outlet 203 may be disposed relatively in the front of the module cover 202.

The upper surface of the module cover 202 may be inclined downwardly toward the outlet 203. That is, a part of the module cover 202 where the outlet 203 is formed may be configured to be the lowest. Accordingly, when water is present on the module cover 202 or the main shelf 40, such water may flow along the surface of the module cover 202 by gravity and flow into the inlet 203.

In the shoes care device 1 according to one embodiment of the present invention, the module chamber 210 is provided inside the module housing 200, and the module chamber 210 includes a first module chamber 212, a second module chamber 213, and the third module chamber 214. The first module chamber 212, the second module chamber 213, and the third module chamber 214 may be formed at different positions in a plan view. That is, the blowing part 310, the heating part 320, and the dehumidifying part 330 may be disposed at different positions in the module housing 200. According to one embodiment of the present invention, the blowing part 310, the heating part 320, and the dehumidifying part 330, which are main means for drying the air inside the inner cabinet 100 and main means for regenerating the dehumidifying part 330, are disposed together in the module chamber 210 of the module housing 200. Accordingly, the blowing part 310, the heating part 320, and the dehumidifying part 330 are disposed at positions considerably close to each other.

In one embodiment of the present invention, the module case 201 of the module housing 200 may be integrally formed by injection molding. In this case, the bottom parts of the module housing 200 may be integrally formed, the bottom parts may not be assembled with each other, and no gaps may be formed in the bottom parts.

In the arrangement explained above, the condensed water can be effectively prevented from leaking from the module housing 200. In addition, a vertical height of the module housing 200 can be minimized.

When moisture remains at an unintended part inside the shoes care device 1, such moisture may reproduce bacteria or cause odors. This is why there is need for countermeasures to solve the problems, and the shoes care device 1 according to one embodiment of the present invention can effectively prevent water from leaking in consideration of such problems.

In the shoes care device 1 according to one embodiment of the present invention, the air in the module chamber 210 may sequentially move the first module chamber 212, the second module chamber 213, and the third module chamber 214. Accordingly, since the third module chamber 214 and a drying flow path F10b may be connected in the shortest distance, which provides excellent drying efficiency by the dehumidifying part 330, and air heated by the heating part 320 moves directly to the dehumidifying part 330 to form the shoes care device 1 with excellent regeneration efficiency.

In the shoes care device 1 according to one embodiment of the present invention, the module housing 200 includes the suction module chamber 211, and the bottom surface of the suction module chamber 211 may be inclined downwardly toward the first module chamber 212. Accordingly, the air introduced through the outlet 203 moves naturally to the first module chamber 212 by hitting the bottom surface of the suction module chamber 211, and the condensed water introduced into the outlet 203 moves to the first module chamber 212 such that the condensed water can be easily drained.

The shoes care device 1 according to one embodiment of the present invention may include the condenser 400, and the module case 201 may include the first condensed water discharge hole 233. In addition, the module case 201 may include the second condensed water discharge hole 234. Accordingly, the dehumidifying part 330 may be effectively regenerated, and condensed water inside the module housing 200 may be easily discharged to the condenser 400.

In the shoe care device 1 according to one embodiment of the present invention, steam generated by the steam generator 700 may be supplied to the accommodation space 101 in the inner cabinet 100. In this case, the steam supplied to the inner cabinet 100 may not only be sprayed to the exteriors of the shoes through the upper discharge port 811 of the nozzle duct 810, but also to the interiors of the shoes through the lower discharge port 821 of the nozzle 820.

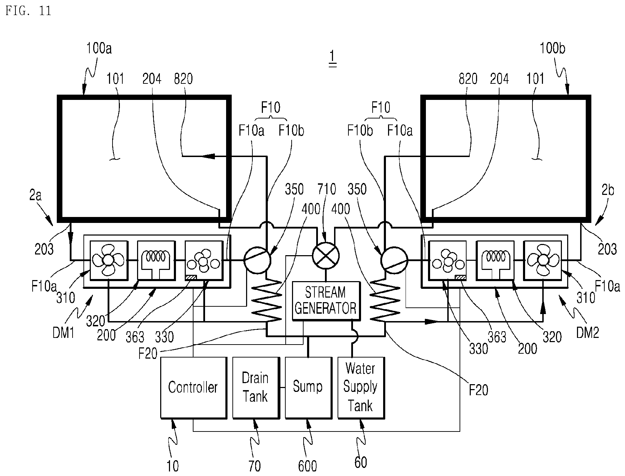

FIG. 11 is a diagram illustrating a connection relationship between components and a flow of fluid in the shoes care device 1 according to one embodiment of the present invention.

The connection path F10 forms a movement path of air connected from the outlet 203 to the nozzle 820. That is, the outlet 203 may form an inlet of the connection path F10, and the nozzle 820 may form an outlet of the connection path F10.

The outlet 203 may be coupled to communicate with the inner cabinet 100, and the nozzle 820 may be provided inside the inner cabinet 100. Except the outlet 203 and the nozzle 820, one part of the connection path F10 may be provided inside the inner cabinet 100, and the other part may be provided outside the inner cabinet 100.

The air inside the inner cabinet 100 moves to the connection path F10 through the outlet 203, and the air passing through the connection path F10 moves back into the inner cabinet 100 through the nozzle 820. As such air flow is repeated, the air circulation is performed in the shoes care device 1.

In the nozzle 820, a hole through which air is discharged is formed in the accommodation space 101 of the inner cabinet 100, and the nozzle 820 may form a last part of the connection path F10.

In the shoes care device 1 according to one embodiment of the present invention, since the nozzle 820 is configured to be movable to various positions inside the inner cabinet 100, the shoes may be managed in various positions.

As described above, the dehumidifying part 330 is disposed in the connection path F10. The air moving through the connection path F10 passes through the dehumidifying part 330, and the dehumidifying part 330 absorbs moisture from the air moving through the connection path F10 such that the air from which moisture has been removed may be supplied into the inner cabinet 100.

The connection path F10 may be divided into a conversion flow path FlOa and a drying flow path F10b. The conversion flow path FlOa and the drying flow path FlOb form a movement path of air sequentially connected to each other. The air in the connection path F10 may sequentially move through the conversion flow path FlOa and the drying flow path F10b.

The conversion flow path FlOa forms an upstream section of the connection path F10, which is connected to the outlet 203. The conversion flow path FlOa may be a section in which the blowing part 310, the heating part 320, and the dehumidifying part 330 are disposed. The conversion flow path FlOa may be formed by the module housing 200, and the module chamber 210 inside the module housing 200 may form the conversion flow path F10a.

The conversion flow path FlOa may be a section in which humid air moves and dries. The conversion flow path FlOa may be a section in which air is dehumidified by the dehumidifying part 330.

Meanwhile, the conversion flow path FlOa may be a section in which the dehumidifying part 330 (the dehumidifying material 331) are regenerated.

The drying flow path FlOb forms a downstream section of the connection path F10, which connects the conversion flow path FlOa to the nozzle 820. A flow path formed by the drying air duct 370, the nozzle duct 810, and the nozzle 820 may form the drying flow path F10b.

The drying passage FlOb may be a section in which dry air with moisture removed therefrom moves.

When the drying module DM operates in the moisture absorption mode, the drying flow path FlOb communicates with the conversion flow path FlOa, and when the drying module DM operates in the regeneration mode, the drying flow path FlOb and the conversion flow path FlOa may not communicate with each other such that the drying flow path FlOb and the conversion flow path FlOa block each other

Accordingly, when air is dehumidified by the dehumidifying part 330 in the conversion flow path FlOa, the dried air moves through the drying flow path F10b.

The dry air duct 370 may be fixedly coupled to an outer wall surface of the inner cabinet 100, and the nozzle duct 810 may be provided inside the inner cabinet 100.

As the dry air duct 370 is tightly coupled to an inner rear plate 110 of the inner cabinet 100, a flow path may be formed between the dry air duct 370 and the inner cabinet 100 (the inner rear plate 110), and such a flow path may form a part of the drying flow path FlOb. A lower part of the dry air duct 370 communicates with the dry air outlet 231 of the module housing 200, an upper part thereof communicates with the nozzle duct 810, which connect the interior of the module housing 200 and the interior of the nozzle duct 810 for mutual communication.

As described above, after humid air in the accommodation space 101 of the inner cabinet 100 flows into the conversion flow path F10a, the air is dehumidified by the dehumidifying part 330 and converted into dry air, and the dry air can be resupplied to the accommodation space 101 of the inner cabinet 100 through the drying flow path F10b.

The regeneration path F20 forms a movement path of a fluid.

The regeneration path F20 forms a passage through which air and/or condensed water inside the shoes care device moves

The regeneration path F20 forms a path through which air and/or condensed water passing through the dehumidifying part 330 moves when the dehumidifying material 331 is regenerated. The regeneration path F20 may be entirely or partially formed of a pipe, a hose, a tube, a duct, a housing, or a combination thereof.

Moisture generated during the regeneration process of the dehumidifying material 331 needs to be discharged through a separate flow path separated from the drying flow path F10b, which is a flow path through which dry air moves. Accordingly, the shoes care device 1 according to one embodiment of the present invention includes the regeneration path F20, and when the dehumidifying material 331 is regenerated, air passing through the dehumidifying part 330 is not ventilated to the nozzle 820 but moves through the regeneration path F20.

The regeneration path F20 is a flow path branched from the connection path F10. The regeneration path F20 may be branched from the connection path F10 to form a path different from that of the drying flow path FlOb of the connection path F10. The regeneration path F20 is connected to the sump 600.

The regeneration path F20 may be a section that connects the conversion flow path F10a and the sump 600.

The regeneration path F20 may be a section in which humid air separated from the dehumidifying part 330 moves.

The condenser 400 according to one embodiment of the present invention forms a regeneration path F20. Moisture separated from the dehumidifying material 331 may be condensed after moving to the condenser 400 along with air moving along the regeneration path F20. In addition, condensed water condensed in the condenser 400 may be moved to the sump 600 through the regeneration path F20, collected from a lower part of the sump 600, and then discharged to the drain tank 70, discharged to the outside, or pressed to the steam generator 700.

In the shoes care device 1 according to one embodiment of the present invention, when the drying module DM operates in the regeneration mode, the regeneration path F20 communicates with the conversion flow path FlOa, and when the drying module DM operates in the moisture absorption mode, the regeneration path F20 and the conversion flow path F10 may not communicate with each other such that the regeneration path F20 and the conversion flow path F10 block each other.

Accordingly, when the dehumidifying part 330 is regenerated in the conversion flow path F10a, humid air containing moisture separated from the dehumidifying part 330 moves through the regeneration path F20.

In one embodiment of the present invention, the damper 350 may be formed in the form of a damper valve.

The damper 350 may be rotatably coupled to the module housing 200. The damper 350 may be coupled to the module housing 200 in a form accommodated in the module housing 200.

As described above, in the module housing 200, the dry air outlet 231 forming an inlet of the drying flow path F10b is formed as a passage of the connection path F10, and the wet air outlet 232 forming an inlet of the regeneration path F20 is formed.

The damper 350 controls a movement path of air passing through the dehumidifying material 331 in the module housing 200. Depending on the operation of the damper 350, the air passing through the dehumidifying material 331 may move into the inner cabinet 100 through the nozzle 820 or may move into the regeneration path F20.

The damper 350 may be configured to open the regeneration path F20 while blocking the drying flow path F10b, or to open the drying flow path FlOb while blocking the regeneration path F20.

The damper 350 may be configured to selectively shield the dry air outlet 231 and the wet air outlet 232. The damper 350 may be configured to selectively seal the dry air outlet 231 and the wet air outlet 232.

The damper 350 may selectively block one of the dry air outlet 231 and the wet air outlet 232. When the damper 350 opens the dry air outlet 231 while blocking the wet air outlet 232, the air passing through the dehumidifying material 331 may move into the inner cabinet 100 through the nozzle 820, and when the damper 350 opens the wet air outlet 232 while blocking the dry air outlet 231, the air passing through the dehumidifying material 331 may be condensed while moving through the regeneration path F20.

In the shoes care device 1 according to one embodiment of the present invention, the damper 350 may be configured to be hinge-rotatable around a hinge axis 350a formed on one side. The hinge axis 350a of the damper 350 may be parallel to the third direction (Z direction). In addition, the shoes care device 1 may include a damper motor 351 configured to rotate the damper 350 around the hinge axis 350a of the damper 350. The damper motor 351 may be formed as an electric motor and may be configured to rotate the damper 350 bidirectionally.

When the damper 350 opens the dry air outlet 231 and seals the wet air outlet 232, the air inside the inner cabinet 100 moves along the connection path F10 and is circulated by sequentially passing through the inlet 203, the module housing 200 (the blowing part 310 and the dehumidifying part 330, the dry air outlet 231, the dry air duct 370, the nozzle duct 810, and the nozzle 820.

When the damper 350 seals the dry air outlet 231 and opens the wet air outlet 232, the air moves along the conversion flow path FlOa and the regeneration path F20 and is circulated by sequentially passing through the module housing 200 (the blowing part 310, the heating part 310, and the dehumidifying part 330), the wet air outlet 232, and the condenser 400.

In one embodiment of the present invention, the controller 10 may control the damper motor 351 such that the damper 350 closes the dry air outlet 231 and opens the wet air outlet 232 when the heating part 320 is turned on. In addition, the controller 10 may control the damper motor 351 such that the damper 350 opens the dry air outlet 231 and closes the wet air outlet 232 when the heating part 320 is turned off.

Accordingly, by controlling the damper motor 351 by the controller 10, the damper 350 may open the drying flow path F10b and close the regeneration path F20 when the heating part 320 is turned off, and may close the drying flow path FlOb and open the regeneration path F20 when the heating part 320 is turned on.

The damper motor 351 may be controlled individually in each of the first management device 2a and the second management device 2b.

Referring to FIG. 11, in the drying module A (DM1) of the first management device 2a, the damper 350 seals the wet air outlet 232 and opens the dry air outlet 231 of the second management device 2b, and in the drying module B (DM2) of the second management device 2b, when the damper 350 opens the wet air outlet 232 and seals the dry air outlet 231, the air in the conversion flow path FlOa of the first management device 2a may flow through the drying flow path F10b, and the air in the conversion flow path FlOa of the second management device 2b may flow through the regeneration path F20. Furthermore, in this case, the drying module A (DM1) of the first management device 2a may operate in the moisture absorption mode, and the drying module B (DM2) of the second management device 2b may operate in the regeneration mode.

In contrast, when in the drying module A (DM1) of the first management device 2a, the damper 350 opens the wet air outlet 232 and seals the dry air outlet 231, and in the drying module B (DM2) of the second management device 2b, the damper 350 seals the wet air outlet 232 and opens the dry air outlet 231, the air in the conversion flow path F10a of the first management device 2a may flow along the regeneration path F20, and the air in the conversion flow path FlOa of the second management device 2b may flow along the drying flow path F10b. Furthermore, in this case, the drying module A (DM1) of the first management device 2a may operate in the regeneration mode, and the drying module B (DM2) of the second management device 2b may operate in the moisture absorption mode.

In both the drying module A (DM1) of the first management device 2a and the drying module B (DM2) of the second management device 2b, when the damper 350 seals the wet air outlet 232 and opens the dry air outlet 231, both the drying module A DM1 and the drying module B (DM2) may operate in the moisture absorption mode.

In both the drying module A (DM1) of the first management device 2a and the drying module B (DM2) of the second management device 2b, when the damper 350 seals the dry air outlet 231 and opens the wet air outlet 232, both the drying module A (DM1) and the drying module B (DM2) may operate in the regeneration mode.

In the shoes care device 1 according to one embodiment of the present invention, the first management device 2a and the second management device 2b individually include the inner cabinet 100, the connection path F10, the blowing part 310, and the dehumidifying part 330. In addition, the shoes care device 1 includes the steam generator 700 and the steam valve 710. Accordingly, the degree of the supply of steam, the degree of dehumidification by the dehumidifying part 330, and the flow of air circulated along the connection path F10 may be different in each of the first management device 2a and the second management device 2b, and the first management device 2a and the second management device 2b may manage shoes under different conditions.

In addition, the first management device 2a and the second management device 2b include the module housing 200, the blowing part 310, the heating part 320, the dehumidifying part 330, and the drying flow path F10b, respectively. The air and condensed water moving in the first management device 2a and the air and condensed water moving in the second management device 2b move along different paths, thereby achieving accurate control intended in each of the first management device 2a and the second management device 2b. In this case, since the first management device 2a and the second management device 2b share and use the steam generator 700, the steam generator 700 can be efficiently utilized in the shoes care device 1, and the shoes care device 1 can efficiently utilize the space.

In addition, as described above, when the shoes are dried in one of the first management device 2a and the second management device 2b, the dehumidifying part 330 may be regenerated in the other, and the efficient management of the shoes and efficient use of the shoes care device 1 can be achieved.

In the shoes care device 1 according to one embodiment of the present invention, the first management device 2a and the second management device 2b each include the regeneration path F20 and the damper 350 individually.

The controller 10 may control the heating part 320 (the heater 321) and the damper 350 to interwork with each other.

The controller 10 may control the damper 350 to open the drying flow path FlOb and close the regeneration path F20 when the heating part 320 is turned off, and may control the damper 350 to close the drying flow path FlOb and open the regeneration path F20 when the heating part 320 is turned on.

The controller 10 may control each component of the shoes care device 1 such that air flowing into the module chamber 210 from the accommodation space 101 and passing through the dehumidifying part 330 moves along the drying flow path FlOb when the heating part 320 is turned off (when the heater 321 of the heating part 320 is turned off), and the air moves along the regeneration path F20 when the heating part 320 is turned on (when the heater 321 of the heating part 320 is turned on).

Such control may be performed individually in each of the first management device 2a and the second management device 2b. Accordingly, since the movement path of air inside the module chamber 210 is changed depending on an operation of the heating part 320, the drying of the shoes and the regeneration of the dehumidifying part 330 can be effectively achieved.

The controller 10 may control the steam valve 710 and the heating part 320 to interwork with each other.

The controller 10 ma control the steam valve 710 such that when the heating part 320 of the first management device 2a is turned off, the valve disk 715 closes or opens the first valve outlet 713, when the heating part 320 of the first management device 2a is turned on, the valve disk 715 closes the first valve outlet 713, when the heating part 320 of the second management device 2b is turned off, the valve disk 715 closes or opens the second valve outlet 714, and when the heating part 320 of the second management device 2b is turned on, the valve disk 715 closes the second valve outlet 714.

In this way, the supply of steam to the accommodation space 101 of the inner cabinet 100 and the operation of the heating part 320 inside the module housing 200 may interwork with each other to effectively perform the drying of the shoes and the regeneration of the dehumidifying part 330.

The shoes care device 1 according to one embodiment of the present invention may include a first sensor 361 and a second sensor 362 (see FIG. 9).

The first sensor 361 may be installed in the second module chamber 213 of the module housing 200, and the second sensor 362 may be installed in the third module chamber 214 of the module housing 200. The first sensor 361 may be configured to measure the temperature and/or humidity of the second module chamber 213, and the second sensor 362 may measure the temperature and/or humidity of the third module chamber 214.

The first sensor 361 measures the temperature and/or humidity of air before passing through the dehumidifying part 330, and the second sensor 362 measures the temperature and/or humidity of air after passing through the dehumidifying part 330.

The controller 10 may compare the temperature and/or humidity of the second module chamber 213 measured by the first sensor 361 with the temperature and/or humidity of the third module chamber 214 measured by the second sensor 362 to recognize a state and a change of the temperature and/or humidity inside the module housing, and may also check an operation state of the drying module DM.

The controller 10 may recognize the temperature and humidity of the second module chamber 213 measured by the first sensor 361, and the temperature and humidity of the third module chamber 214 measured by the second sensor 362 to recognize a change in the humidity inside the module housing 200. Accordingly, the degree of dehumidification by the dehumidifying part 330 may be checked, and the degree of regeneration of the dehumidifying part 330 may be checked.

In one embodiment, when the drying module DM operates in the moisture absorption mode, the controller 10 may control the drying module DM to stop operating in the moisture absorption mode and operate in the regeneration mode if a humidity change amount of the second module chamber 213 recognized by the first sensor 361 and a humidity change amount of the third module chamber 214 recognized by the second sensor 362 is less than or equal to a reference value.

In one embodiment, when the drying module DM operates in the regeneration mode, the controller 10 may control the drying module DM to stop operating in the regeneration mode if the humidity change amount of the second module chamber 213 recognized by the first sensor 361 and the humidity change amount of the third module chamber 214 recognized by the second sensor 362 is less than or equal to the reference value.

The shoes care device 1 according to one embodiment of the present invention further includes a third sensor 363 capable of measuring the amount of moisture adsorbed to the dehumidifying material 331, and the controller 10 may control the drying module DM to operate the regeneration mode until the amount of moisture measured by the third sensor 363 is less than or equal to a set value.

Specifically, the controller 10 may control all the heating parts 320 to operate until the amount of moisture measured by the third sensor 363 is less than or equal to the set value.

In this case, as illustrated in FIG. 11, the third sensor 363 may include a moisture sensor installed adjacent to the dehumidifying material 331 to measure the amount of moisture adsorbed to the dehumidifying material 331, and the type and number thereof may vary as necessary.

In this way, when the moisture adsorbed on the dehumidifying material 331 is sensed to exceed the reference value, the shoes care device 1 according to one embodiment of the present invention first regenerates all dehumidifying materials 331 until the moisture is less than or equal to the reference value. Accordingly, the dehumidifying material 331 can maintain an appropriate state for dehumidification all the times even when the shoes care device 1 operates to refresh the shoes.

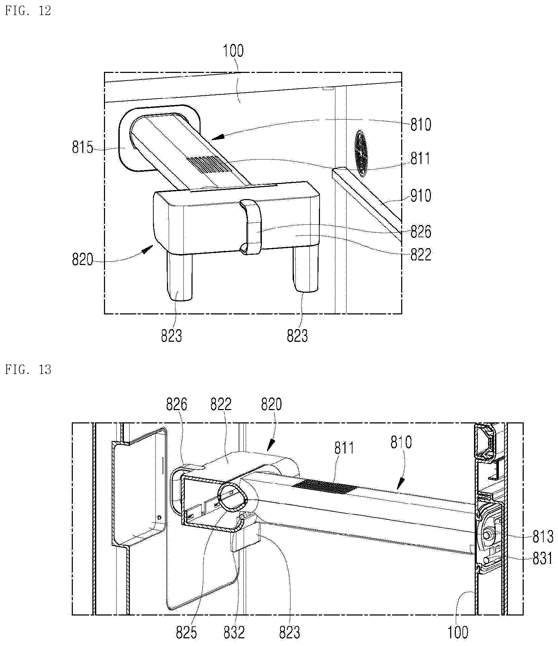

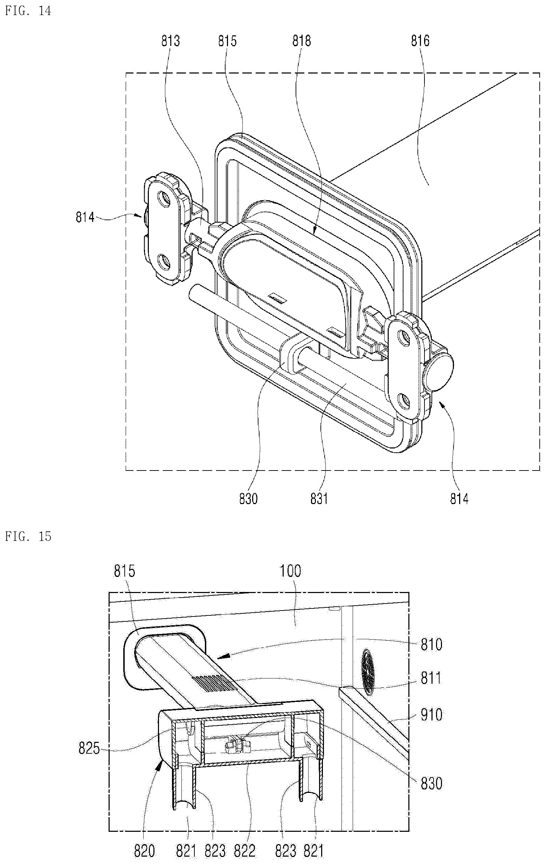

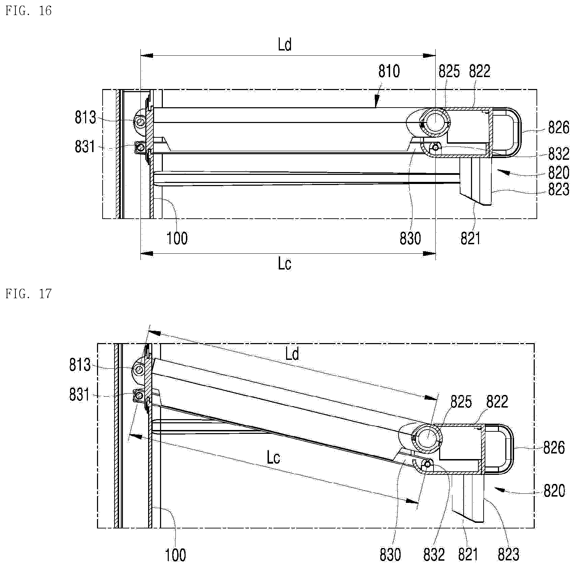

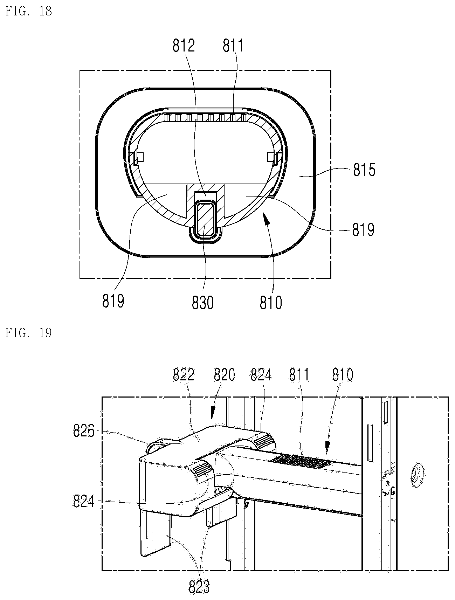

FIG. 12 is a view illustrating in more detail a nozzle duct 810 and a nozzle 820 installed in the inner cabinet 100 in the shoe care device 1 according to one embodiment of the present invention. FIG. 13 is a cross-sectional view illustrating coupling structures at one end and the other end of the nozzle duct 810 illustrated in FIG. 12. FIG. 14 is a cross-sectional view illustrating a portion where the nozzle duct 810 in FIG. 12 is coupled to the inner cabinet 100. FIG. 15 is a cross-sectional view illustrating a portion where the nozzle duct 810 of FIG. 12 is coupled to the nozzle 820. FIGS. 16 and 17 are views illustrating the state in which the angle of the nozzle duct 810 of FIG. 12 is adjusted. FIG. 18 is a cross-sectional view illustrating the interior of the nozzle duct 810 of FIG. 12. FIG. 19 is a view illustrating the nozzle duct 810 and the nozzle 820 of FIG. 12 viewed in another direction.

The shoe care device 1 according to one embodiment of the present invention may include an inner cabinet 100, an outlet 203 , a nozzle duct 810, a nozzle 820, a nozzle connector 830, a connection path F10, a blowing part 310, and a dehumidifying part 330.

The nozzle duct 810 is a part that is hinged to the inner cabinet 100 at one end thereof and protrudes into the inner cabinet 100 to form an air passage. The nozzle 820 is coupled to the other end of the nozzle duct so that the nozzle duct may form a passage through which air moves to the nozzle 820.

Since the nozzle duct 810 is installed to protrude into the inner cabinet 100, the position where the nozzle 820 is disposed inside the inner cabinet 100 may be varied.

In particular, the one end of the nozzle duct 810 may be hinged to the inner cabinet 100. That is, a nozzle supporter hinge axis 813 provided at one end of the nozzle duct 810 may be coupled to the inner cabinet 100 to make the nozzle duct 810 rotatable around the nozzle supporter hinge axis 813.

The nozzle 820 is a part that is hinged to the other end of the nozzle duct 810 to be insertable into a shoe from inside the inner cabinet 100 to spray air into the shoe. The air passing through the connection path F10 may be sprayed to the shoe inside the inner cabinet 100 through the nozzle 820.

The nozzle 820 sprays air in the state of being inserted into the shoe, thereby ensuring that air is smoothly introduced into the shoe.

In particular, the nozzle 820 may be hinged to the other end of the nozzle duct 810. That is, since the nozzle 820 is coupled to the nozzle hinge axis 825 formed at the other end of the nozzle duct 810, the nozzle 820 is rotatable around the nozzle hinge axis 825.

The nozzle connector 830 is a part that is installed separately from the nozzle duct 810, hinged to the inner cabinet 100 at one end, and hinged to the nozzle 820 at the other end. The nozzle connector may ensure that the angle of the nozzle 820 is maintained constant even when the nozzle duct 810 performs hinge rotation.

In relation to this, when the nozzle duct 810 performs hinge rotation around the nozzle supporter hinge axis 813 in a hinged manner, the position of the nozzle 820 coupled to the other end of the nozzle duct 810 is lowered.

In this case, when the angle of the nozzle 820 is also constrained by the angle of the nozzle duct 810, the nozzle 820 is also inclined integrally with the nozzle duct 810, making it difficult for the nozzle 820 to be inserted into the upper surface of a shoe.

Therefore, even when the nozzle duct 810 is disposed to be inclined, it may be desirable to adjust the angle of the nozzle 820 such that the nozzle is not inclined by a predetermined angle or more.

However, since the nozzle 820 is also hinged to the other end of the nozzle duct 810, a user may arbitrarily adjust the angle of the nozzle 820 such that the nozzle 820 is not inclined by a predetermined angle or more.

However, when the user performs operations as described above, there are problems in that it may be very inconvenient from the user's point of view and that the angle of the nozzle duct 810 may also be changed in the process of adjusting the angle of the nozzle 820.

Accordingly, with the nozzle connector 830 installed separately from the nozzle duct 810, even when the user does not separately adjust the angle of the nozzle 820, it may be desirable for the nozzle 820 to always maintain its angle constant in the process of adjusting only the angle of the nozzle duct 810.

Specifically, it may be desirable for the nozzle 820 to maintain the angle at which the lower discharge port 821 discharges air in the vertical direction.

In this case, the first connection hinge axis 831 formed at one end of the nozzle connector 830 is coupled to the inner cabinet 100, so that the nozzle connector 830 can also be rotated around the first connection hinge axis 831.

In addition, the nozzle 820 is coupled to the second connection hinge axis 832 formed at the other end of the nozzle connector 830, so that the nozzle 820 can also be rotated around the second connection hinge axis 832.

In addition, when the angle of the nozzle duct 810 is changed, the angle of the nozzle connector 830 may be changed correspondingly.

In this case, since the gap and lateral displacement between the nozzle supporter hinge axis 813 and the first connection hinge axis 831 are constrained to the inner cabinet 100, the nozzle supporter hinge axis 813 and the first connection hinge axis 831 may maintain the distance therebetween even when the nozzle duct 810 and the nozzle connector 830 rotate by the same angle.

Meanwhile, since the distance between the nozzle hinge axis 825 coupled to the nozzle 820 and the second connection hinge axis 832 is also constrained to the nozzle 820, even when the nozzle duct 810 and the nozzle connector 830 are rotated by the same, the nozzle hinge axis 825 and the second connection hinge axis 832 may also maintain the distance therebetween.

However, depending on the rotation of the nozzle duct 810, the lateral position of the nozzle hinge axis 825 may move toward the nozzle supporter hinge axis 813, and the longitudinal position of the nozzle hinge axis 825 may move downward.

At the same time, the lateral and longitudinal positions of the second connection hinge axis 832 may be moved to correspond to the lateral and longitudinal positions of the nozzle hinge axis 825.

Accordingly, since the nozzle hinge axis 825 and the second connection hinge axis 832 may move the lateral and longitudinal positions thereof equally while maintaining the same distance therebetween, even when the nozzle duct 810 and the nozzle connector 830 are changed to any angle, the angle of the nozzle 820 may be maintained constant.

In this way, in the shoe care device 1 according to the present embodiment, one end and the other end of the nozzle duct 810 are hinged to the inner cabinet 100 and the nozzle 820, respectively, and separately from the nozzle duct 810, one end and the other end of the nozzle connector 830 are hinged to the inner cabinet 100 and the nozzle 820, respectively, whereby even when the angle of the nozzle duct 810 is changed, the angle of the nozzle 820 is maintained constant. Therefore, shoe processing efficiency can be further improved by stably supplying air to a shoe.

In the shoe care device 1 according to one embodiment of the present invention, the nozzle 820 may include a nozzle body 822 hinged to the nozzle duct 810, and a nozzle protrusion 823 protruding downward from the nozzle body 822 and having a lower discharge port 821 provided at an end thereof.

Specifically, the nozzle body 822 is a part that is coupled to the nozzle duct 810 by the nozzle hinge axis 825 and is also coupled to the nozzle connector 830 by the second connection hinge axis 832.

Accordingly, even when the angle of the nozzle duct 810 is changed, the angle of the nozzle body 822 may be maintained constant.

Meanwhile, a nozzle handle 826 may be provided on the nozzle body 822. Thus, in order to adjust the angle of the nozzle duct 810, the user may easily adjust the angle of the nozzle duct 810 by holding the nozzle handle 826 and applying force without touching the nozzle duct 810.

The nozzle protrusion 823 is a part that protrudes from the nozzle body 822 to be easily inserted into a shoe, and may have a lower discharge port 821 that is open downward toward the shoe.

In this case, it may be desirable for the nozzle protrusion 823 to maintain a vertical angle such that the lower discharge port 821 discharges air in the vertical direction.

In addition, it is desirable to configure the end portion of the nozzle protrusion 823 to be inserted into an open surface of a shoe corresponding to an ankle of a shoe wearer and then to introduce air into the toe of the shoe smoothly.

To this end, the end portion of the nozzle protrusion 823 may be partially inclined.

As described above, in the shoe care device 1 according to the present embodiment, since the nozzle 820 includes the nozzle body portion 822 and the nozzle protrusion 823, the lower discharge port 821 provided in the nozzle protrusion 823 may be more easily inserted into the interior of the shoe.

In the shoe care device 1 according to one embodiment of the present invention, the length of the nozzle connector 830 may correspond to that of the nozzle duct 810.

As described above, since it is desirable for the nozzle 820 to maintain the angle at which the lower discharge port 821 discharges air in the vertical direction, the length Ld of the nozzle duct 810 and the length Lc of the nozzle connector 830 Lc are preferably equal to each other.

When the lengths of the nozzle duct 810 and the nozzle connector 830 are different, the lateral positions of the nozzle hinge axis 825 and the second connection hinge axis 832 are different. Therefore, it may be difficult for the nozzle 820, which is coupled to both the nozzle duct 810 and the nozzle connector 830, to maintain the angle at which air is discharged in the vertical direction.

In this way, in the shoe care device 1 according to the present embodiment, since the lengths of the nozzle connector 830 and the nozzle duct 810 correspond to each other, the nozzle 820, which is hinged to both the nozzle duct 810 and the nozzle connector 830, may always maintain the same angle.

In the shoe care device 1 according to one embodiment of the present invention, the nozzle duct 810 may have a nozzle groove 812 extending along the longitudinal direction, and the nozzle connector 830 may be installed by being inserted into the nozzle groove 812.

As described above, in order to make the angle of the nozzle duct 810 adjustable while the nozzle 820 maintains a constant angle, it is necessary to install the nozzle connector 830 separately from the nozzle duct 810.

However, although the nozzle connector 830 is a necessary member for the function of the shoe care device 1, it may be perceived as a secondary member from a user's point of view. In particular, when the nozzle connector 830 is exposed to the user, its appearance may not be good, and the structure of the shoe care device 1 may be perceived as complicated.

Accordingly, it is desirable for the nozzle connector 830 to perform the above-described functions without problems while being as little exposed to the user as possible.

Accordingly, the nozzle groove 812 may be provided in the nozzle duct 810 to correspond to the shape of the nozzle connector 830. In addition, the nozzle connector 830 may be coupled to the inner cabinet 100 and the nozzle 820 in the state of being inserted into the nozzle groove 812.

Through this, the nozzle connector 830 may perform the function of maintaining the nozzle 820 at a constant angle without problems while being minimally exposed to the user

In this way, in the shoe care device 1 according to the present embodiment, since the nozzle connector 830 is installed by being inserted into the nozzle groove 812 provided along the longitudinal direction of the nozzle duct 810, exposure of the nozzle connector 830 may be minimized while the user changes the nozzle duct 810.

In the shoe care device 1 according to one embodiment of the present invention, the nozzle duct 810 may have an upper discharge port 811 that is open upward. For example, the upper discharge port 811 is provided in a portion of the upper surface of the nozzle duct 810 so that some of the air moving into the nozzle duct 810 may be discharged to the accommodation space 101 through the upper discharge port 811.

Accordingly, since the air is discharged within the inner cabinet 100 through the upper discharge port 811 provided in the nozzle duct 810 as well as the lower discharge port 821 provided in the nozzle 820, the air may be discharged to various portions within the inner cabinet 100 , so that the processing efficiency of a shoe can be further improved.

In the shoe care device 1 according to one embodiment of the present invention, an auxiliary discharge port 824 may be provided in the upper surface of the nozzle body 822. The auxiliary discharge port 824 is provided in a portion of the upper surface of the nozzle body 822, so that some of the air moving inside the nozzle duct 810 may be discharged into the accommodation space 101 through the auxiliary discharge port 824.

In this case, the auxiliary discharge port 824 may be provided in the upper surface of the nozzle body 822 toward the rear wall of the inner cabinet 100.

Inside the inner cabinet 100, moisture generated during a shoe processing process may be condensed on the inner wall or the like of the inner cabinet 100. In particular, when steam is supplied to the accommodation space 101, a relatively large amount of moisture may be condensed, and it is necessary to effectively remove the condensed moisture for the function of the shoe care device 1.

Meanwhile, a door 30 may be installed on the front surface of the inner cabinet 100, and, if necessary, an outlet 203 may be placed on the front side of the inner cabinet 100.

Accordingly, the front surface side in the interior of the inner cabinet 100 is relatively advantageous for air circulation, and the generated moisture may be effectively removed in the front surface side.

On the other hand, the rear wall side in the interior of the inner cabinet 100 is relatively disadvantageous for air circulation, and the generated moisture may not be effectively removed in the rear wall side.

Accordingly, by discharging air from the upper surface of the nozzle body 822 through the auxiliary discharge port 824 toward the rear wall of the inner cabinet 100, air may be circulated to the portion that is relatively vulnerable to air circulation.

In this way, since the shoe care device 1 according to the present embodiment also discharges air through the auxiliary discharge port 824 provided in the nozzle body 822 within the inner cabinet 100, dry air may be supplied to a portion of the interior of the inner cabinet 100 where moisture removable is disadvantageous.

In addition, in the shoe care device 1 according to the present embodiment, since the steam is also sprayed through the auxiliary discharge port 824 provided in the nozzle body 822 within the inner cabinet 100, the steam can be sprayed to various portions of the shoes within the inner cabinet 100.

In the shoe care device 1 according to one embodiment of the present invention, the nozzle body 822 may be attached to the ceiling surface of the inner cabinet 100 using magnetic force.

As described above, in the case where the nozzle duct 810 is installed to protrude to the interior of the inner cabinet 100, while the nozzle 820 is not in use, it may be desirable to position the nozzle 820 in the upper portion of the inner cabinet 100 so that the nozzle does not occupy the internal space of the inner cabinet 100.

In this case, the hinge axis resistance part 814 may maintain the nozzle 820 in the state of being positioned in the upper portion of the inner cabinet 100.

However, when an unintended external force is applied to the nozzle 820 or the performance of the hinge axis resistance part 814 deteriorates due to long-term use, there is a risk that the nozzle 820 may sag downward even when it is not in use.

Therefore, when the nozzle 820 is not used, the nozzle 820 may be attached to the ceiling surface of the inner cabinet 100 using magnetic force to hold and may be held on the ceiling surface of the inner cabinet 100.

In this case, a magnetic material may be disposed on at least one of the upper surface of the nozzle 820 and the ceiling surface of the inner cabinet 100, and the upper surface of the nozzle 820 and the ceiling of the inner cabinet 100 may be made of a material that can be attached by magnetic force.

In this way, in the shoe care device 1 according to the present embodiment, since the nozzle 820 is attached to the ceiling surface of the inner cabinet 100, the nozzle 820 and the nozzle duct 810, which are not in use, may be prevented from occupying the internal space of the inner cabinet 100 unnecessarily.

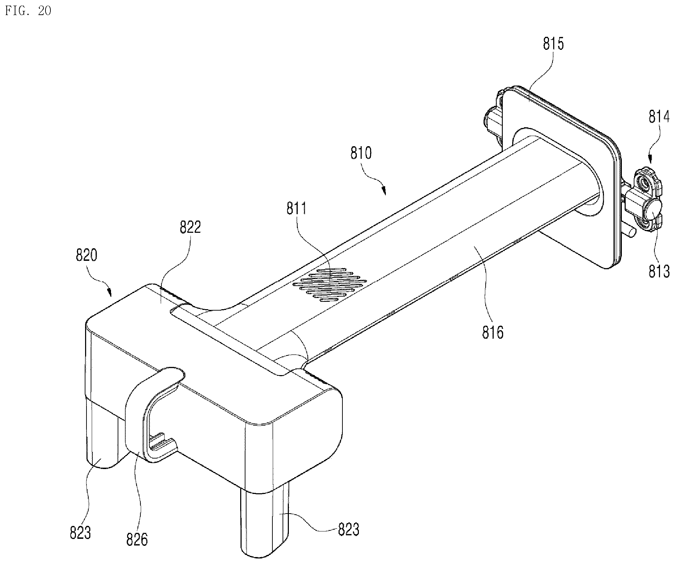

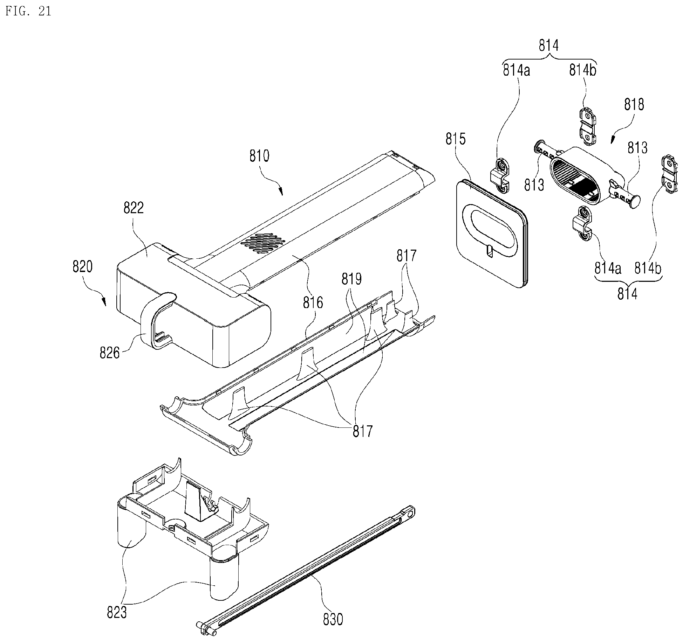

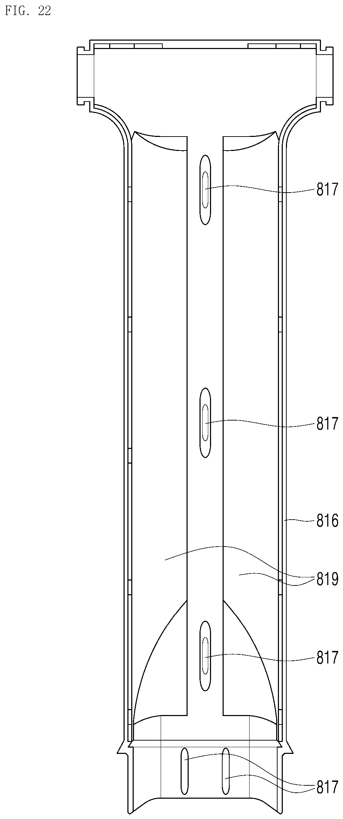

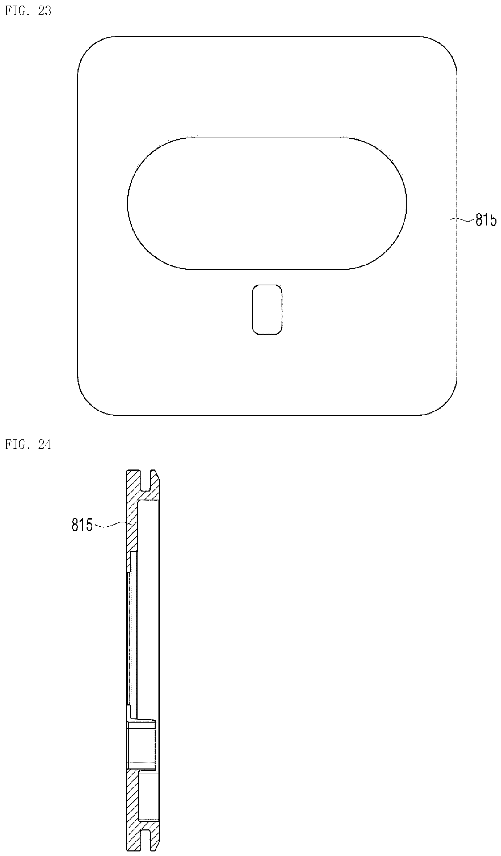

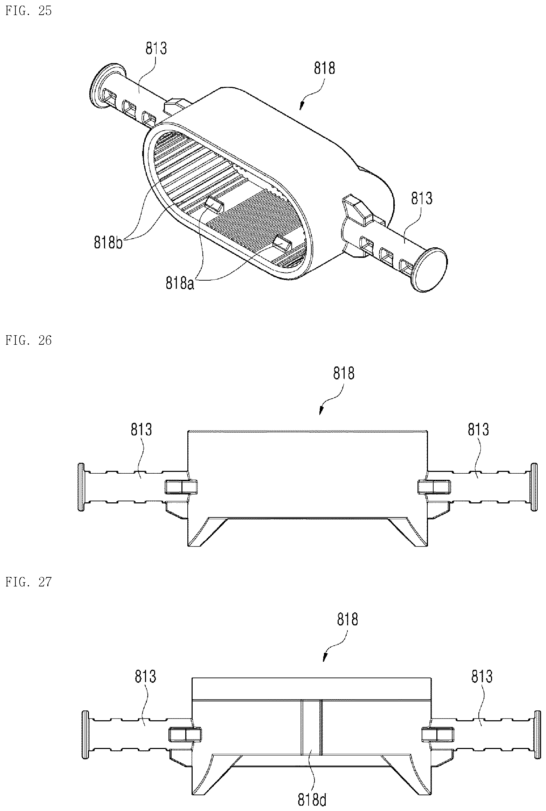

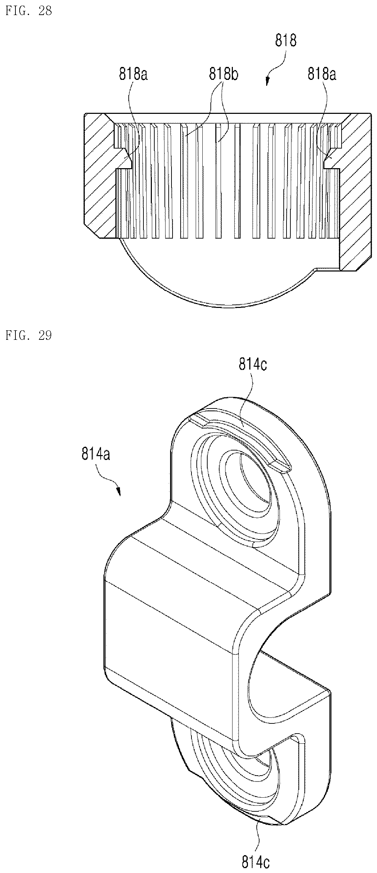

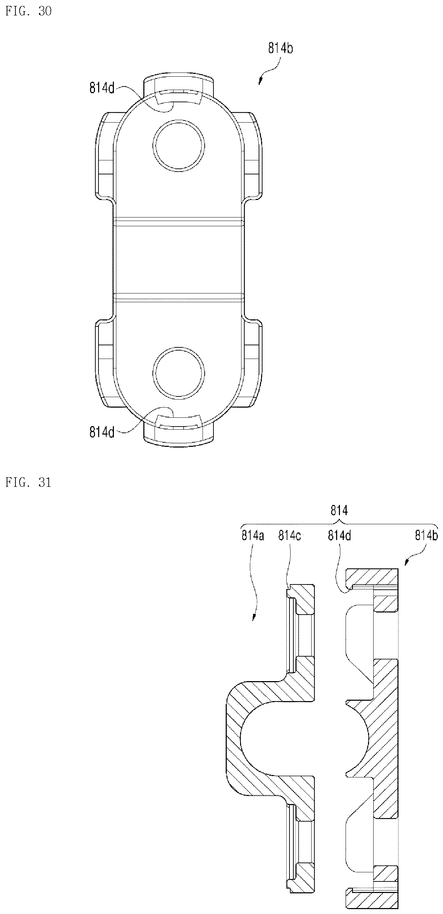

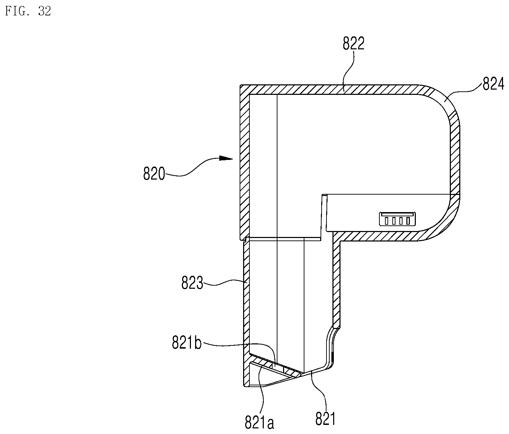

FIG. 20 is a view illustrating the nozzle duct 810 and the nozzle 820 of FIG. 12 viewed in another direction. FIG. 21 is an exploded view illustrating the nozzle duct 810 and the nozzle 820 of FIG. 20. FIG. 22 is a cross-sectional view illustrating the interior of the nozzle duct 810 of FIG. 20. FIGS. 23 and 24 are views illustrating the nozzle sealing part 815 of FIG. 20 in more detail. FIGS. 25 to 28 are views illustrating the nozzle supporter 818 of FIG. 20 in more detail. FIGS. 29 to 31 are views illustrating the hinge axis resistance part 814 of FIG. 20 in more detail. FIG. 32 is a cross-sectional view illustrating the interior of the nozzle 820 illustrated in FIG. 20.

The shoe care device 1 according to one embodiment of the present invention may include an inner cabinet 100, an outlet 203, a nozzle duct 810, a nozzle 820, a connection path F10, a blowing part 310, and a dehumidifying part 330. In this case, the nozzle duct 810 may include a nozzle bar 816 and a nozzle rib 817.

The inner cabinet 100 is a part that provides an accommodation space 101 configured to accommodate shoes, and have an outlet 203 and a nozzle 820 disposed therein.

The outlet 203 is a part provided in a portion of the inner cabinet 100 to allow the air inside the inner cabinet 100 to be sucked, and may form the beginning of the connection path F10.

In the outlet 203, a net, such as a grid or mesh, may be provided.

The outlet 203 may be configured in a shape extending to opposite side surfaces of the inner cabinet 100. That is, the outlet 203 may be provided in the form of a long hole extending from the bottom surface to the opposite side surfaces of the inner cabinet 100.

In this case, the bottom surface of the inner cabinet 100 may be inclined downward toward the outlet 203. That is, the portion where the outlet 203 is provided in the bottom surface of the inner cabinet 100 may be disposed at the lowest level. Accordingly, when there is water on the bottom surface of the inner cabinet 100, the water may flow along the bottom surface of the inner cabinet 100 due to gravity and flow into the outlet 203.

The nozzle duct 810 is a part that is hinged to the inner cabinet 100 at one end thereof and protrudes into the inner cabinet 100 to form an air passage. The nozzle 820 is coupled to the other end of the nozzle duct so that the nozzle duct may form a passage through which air moves to the nozzle 820.

The nozzle 820 is a part that is hinged to the other end of the nozzle duct 810 to be insertable into a shoe from inside the inner cabinet 100 to spray air into the shoe. The air passing through the connection path F10 may be sprayed to the shoe inside the inner cabinet 100 through the nozzle 820.

The connection path F10 is a part that forms a flow path through which air in the accommodation space 101 is discharged from the inner cabinet 100 and then flows back to the accommodation space 101. The connection path may be an air flow path through which the air inside the inner cabinet 100 is dehumidified while being suctioned into the module chamber 210 and then blown to pass through the dehumidifying part 330, and is supplied back into the inner cabinet 100.

In this case, the connection path F10 is a part where air circulates between the outlet 203 and the nozzle 820. The outlet 203 may form the inlet of the connection path F10, and the nozzle 820 may form the outlet of the connection path F10.

The blowing part 310 is a part disposed in the connection path F10 to blow air. When the blowing part 310 is operated, air may be suctioned from the inner cabinet 100, and the suctioned air may be blown from the connection path F10.

In this case, the blowing part 310 may be installed on the connection path F10 and blow air from the outlet 203 toward the nozzle 820.

The dehumidifying part 330 is a part that is installed on the connection path F10 and dehumidifies air. With the heating of the dehumidifying material 430, the moisture adsorbed on the dehumidifying material 430 may be separated and the dehumidifying material 430 may be regenerated to the state in which the dehumidifying function can be performed.

The module chamber 210 is a part that blows air from the accommodation space 101 and is capable of heating the dehumidifying part 330, which is disposed on the path of the blown air

In this case, the dehumidifying part 330 is heated, whereby the moisture adsorbed on the dehumidifying part 330 is separated, so that the dehumidifying part 330 can be regenerated to the state in which the dehumidifying function can be performed.

To this end, the module chamber 210 may include a dehumidifying part 330, a blowing part 310, a heating part 320, a damper 350, and the like.

In particular, the module chamber 210 may form a portion of the connection path F10 through which air circulates between the outlet 203 and the nozzle 820, and the regeneration path F20, which is branched from the connection path F10 passing through the dehumidifying part 330, so that blowing is performed therethrough.

Accordingly, the air in the accommodation space 101 may move to the connection path F10 or the regeneration path F20 in the process of being blown to the module chamber 210 to pass through the dehumidifying part 330.

The controller 10 is a part that controls the shoe care device 1, and may control the module chamber 210 to selectively open/close the connection path F10 and the regeneration path F20 depending on whether the dehumidifying part 330 is heated.

That is, the controller 10 may selectively open/close the connection path F10 and the regeneration path F20 depending on whether the dehumidifying part 330 is in a regenerated state.

The connection path F10 is a part where air circulates between the outlet 203 and the nozzle 820. The outlet 203 may form the inlet of the connection path F10, and the nozzle 820 may form the outlet of the connection path F10.

That is, the connection path F10 may be an air flow path through which the air inside the inner cabinet 100 is dehumidified in the process of being sucked into the module chamber 210 and then blown to pass through the dehumidifying part 330, and is then returned again to the interior of the inner cabinet 100.

In this way, the shoe care device 1 according to the present embodiment may always maintain appropriate shoe processing performance since the dehumidifying part 330 is disposed in the module chamber 210 not only to collect moisture and bacteria in the blown air, but also to heat and regenerate the dehumidifying part 330 in the module chamber 210.

In addition, the shoe care device 1 according to the present embodiment may prevent a user from being exposed to the air used to dehumidify and deodorize shoes since the connection path F10 through which air circulates is provided between the outlet 203 and the nozzle 820 each of which is disposed inside the inner cabinet 100.

The nozzle bar 816 is a part that is provided in a tubular shape extending along the longitudinal direction through which air is movable along the internal space. The nozzle bar may connect the inner cabinet 100 and the nozzle 820 to provide an air moving passage.

In this case, since the nozzle bar 816 has a hollow tubular structure, sufficient rigidity against an external force or the like may not be ensured. When the rigidity of the nozzle bar 816 is insufficient, the nozzle duct 810 may be deformed or damaged when manufacturing the shoe care device 1.

In addition, when the nozzle bar 816 is not sufficiently rigid, the function of the nozzle duct 810 may not be performed smoothly in the process of using the shoe care device 1, and problems in usability may occur.

Therefore, in order to appropriately reinforce the rigidity of the nozzle bar 816, it may be desirable to form the nozzle rib 817 inside the nozzle bar 816.

The nozzle rib 817 is a portion provided inside the nozzle bar 816 to connect the upper and lower surfaces to each other, and may reinforce the rigidity of the nozzle bar 816 to minimize deformation and damage even against an external force.

Nozzle ribs 817 may be disposed at a plurality of points with a predetermined length along the longitudinal direction of the nozzle bar 816 so as not to impede the movement of air inside the nozzle bar 816.

In addition, the nozzle ribs 817 may be arranged in parallel at portions where the nozzle supporter 818 and the nozzle bar 816 are coupled to further reinforce rigidity.

In this way, since the shoe care device 1 according to the present embodiment are provided with the nozzle ribs 817 that connect the upper and lower surfaces inside the tubular nozzle bar 816 to reinforce rigidity, the nozzle duct 810 may have sufficient rigidity to prevent functional deterioration or inconvenience in use in the process of manufacturing and using the shoe care device 1.

The shoe care device 1 according to one embodiment of the present invention may further include a steam generator 700 configured to supply steam to the inner cabinet 100.

That is, since steam treatment of shoes is performed by supplying steam into the inner cabinet 100 through the steam generator 700, a refreshing effect through swelling of the shoe material or the like may be achieved in addition to a sterilization effect by the high temperature of steam.

In the shoe care device 1 according to one embodiment of the present invention, the nozzle duct 810 may be installed to protrude forward from the rear wall of the inner cabinet 100.

That is, since the nozzle duct 810 is installed to be directed forward from the rear wall of the inner cabinet 100, the internal space of the inner cabinet 100 may be effectively utilized.

In the shoe care device 1 according to one embodiment of the present invention, one end of the nozzle duct 810 may be coupled while penetrating the inner cabinet 100.

As described above, the air in the accommodation space 101 may move to the connection path F10 or the regeneration path F20 in the process of being discharged to the exterior of the inner cabinet 100 through the outlet and then blown into the module chamber 210 to pass through the dehumidifying part 330.

During this, the air moving to the connection path F10 may be discharged back into the accommodation space 101 through the nozzle duct 810 and the nozzle 820 installed inside the inner cabinet 100.

Therefore, in order for air to circulate along the connection path F10, a portion connected to the nozzle duct 810 from the exterior of the inner cabinet 100 to allow air to move therethrough is required.

Meanwhile, since one end of the nozzle duct 810 is hinged to the inner cabinet 100 as described above, it may be desirable for air to move from the exterior of the inner cabinet 100 to the nozzle duct 810 through the portion coupled to the inner cabinet 100 as described above.

Accordingly, one end of the nozzle duct 810 hinged to the inner cabinet 100 may be coupled while penetrating the inner cabinet 100, allowing air outside the inner cabinet 100 to flow into the nozzle duct 810.

In this way, in the shoe care device 1 according to the present embodiment, since the nozzle duct 810 is coupled to the inner cabinet 100 in a penetrating structure, air may move smoothly from the connection path F10 to the nozzle duct 810.

In addition, the shoe care device 1 according to the present embodiment, since the nozzle duct 810 is coupled to the inner cabinet 100 in a penetration structure, steam can be smoothly moved from the dry air duct 370 to the nozzle duct 810.

In the shoe care device 1 according to one embodiment of the present invention, the nozzle duct 810 may include a nozzle sealing part 815 interposed to surround the outer peripheral surface of the nozzle duct 810 in the portion coupled with the inner cabinet 100.

As described above, when the nozzle duct 810 is coupled by causing one end thereof to penetrate the inner cabinet 100, an airtightness problem may occur at this coupled portion.

In particular, since the nozzle duct 810 is coupled to the inner cabinet 100 to be capable of hinge rotation about one end, a structure that is capable of maintaining airtightness without interfering with the hinge rotation of the nozzle duct 810 is required.

Accordingly, the nozzle sealing part 815 may be coupled to surround the outer peripheral surface of the nozzle duct 810 to prevent air from moving to the outer peripheral surface of the nozzle duct 810.

In addition, the nozzle sealing part 815 may be made of a certain elastic material member to minimize interference with the hinge rotation of the nozzle duct 810.

In this way, in the shoe care device 1 according to the present embodiment, since the nozzle duct 810 is sealed in the coupling surface with the inner cabinet 100 other than the penetration portion by the nozzle sealing part 815, it is possible to prevent air from leaking to parts other than the connection path F10 while ensuring a certain degree of mobility of the nozzle duct 810 with respect to the inner cabinet 100.

In addition, in the shoe care device 1 according to the present embodiment, since the nozzle duct 810 is sealed in the coupling surface with the inner cabinet 100 other than the penetration portion by the nozzle sealing part 815, it is possible to prevent steam from leaking to parts other than the connection path F10 while ensuring a certain degree of mobility of the nozzle duct 810 with respect to the inner cabinet 100.

In the shoe care device 1 according to one embodiment of the present invention, the nozzle sealing part 815 may be made of a heat-resistant material.

In the process in which the steam supplied from the steam generator 700 moves to the nozzle duct 810, thermal deformation or thermal damage to the nozzle sealing part 815 may occur due to steam corresponding to a relatively high temperature.

When thermal deformation or thermal damage occurs in the nozzle sealing part 815, there is a risk that the performance of the nozzle sealing part 815 may deteriorate and airtightness may not be effectively achieved.

Therefore, the nozzle sealing part 815 may be preferably made of a heat-resistant material so that thermal deformation or thermal damage does not occur even when exposed to steam corresponding to a relatively high temperature.

In this way, in the shoe care device 1 according to the present embodiment, since the nozzle sealing part 815 is made to have heat-resistant performance, it is possible to prevent the performance of the nozzle sealing part 815 from being deteriorated by steam corresponding to a relatively high temperature.

Meanwhile, during the hinge rotation of the nozzle duct 810, the nozzle sealing part 815 may press the nozzle duct 810 using an elastic restoring force. Accordingly, a force to return the nozzle duct 810 to a horizontal state may be applied to the nozzle duct 810 by the nozzle sealing part 815.

However, as described above, when a frictional force generated by the hinge axis resistance part 814 is greater than the elastic restoring force of the nozzle sealing part 815, the nozzle duct 810 may maintain its current angle state without returning to the horizontal state.

In the shoe care device 1 according to one embodiment of the present invention, the portion of the nozzle sealing part 815 that is in contact with the outer peripheral surface of the nozzle bar 816 may be formed relatively thinner than the remaining portion.

As described above, it is necessary for the nozzle sealing part 815 to have a structure that is capable of maintaining airtightness without interfering with the hinge rotation of the nozzle duct 810.

In this case, the nozzle sealing part 815 may be made of an elastic material member to allow the hinge rotation of the nozzle duct 810 to a certain extent.

However, in order to allow the hinge rotation of the nozzle duct 810 to be performed more smoothly, it may be desirable to make the thickness of the nozzle sealing part 815 at the portion in contact with the outer peripheral surface of the nozzle bar 816 as thin as possible within the limit of maintaining airtightness.

In this way, in the shoe care device 1 according to the present embodiment, since the nozzle sealing part 815 in contact with the outer peripheral surface of the nozzle bar 816 is made relatively thin, interference with the nozzle sealing unit 815 may be minimized when adjusting the angle of the nozzle duct 810 while airtightness is maintained.

In the shoe care device 1 according to one embodiment of the present invention, the nozzle duct 810 may further include a nozzle supporter 300, which is fastened to the end portion of the nozzle bar 816 that penetrates the nozzle sealing part 815, to support the nozzle bar 816 with respect to the inner cabinet 100.

That is, the end portion of the nozzle bar 816 may be coupled to the nozzle supporter 818, and the nozzle supporter 818 may be coupled to the inner cabinet 100 to support the nozzle bar 816.

As described above, since the nozzle bar 816 is made of a hollow tubular member, it may not be desirable in terms of rigidity for the end portion of the nozzle bar 816 to be directly coupled to the inner cabinet 100.

Therefore, when the nozzle bar 816 is indirectly coupled to the inner cabinet 100 via the nozzle supporter 818 having a predetermined rigidity, the nozzle duct 810 may be more stably and firmly coupled to the inner cabinet 100.

In this way, in the shoe care device 1 according to the present embodiment, since the nozzle duct 810 is supported with respect to the inner cabinet 100 via the nozzle supporter 818, the nozzle duct 810 may be coupled to the inner cabinet 100 more stably.

In the shoe care device 1 according to one embodiment of the present invention, the nozzle supporter 818 may include a nozzle supporter hooks 818a that protrude inward from the upper and lower surfaces of the portion fastened to the nozzle bar 816, respectively.

When the end portion of the nozzle bar 816, which is a tubular member extending along the longitudinal direction, is simply fitted into the nozzle supporter 818, there is a risk that the nozzle bar 816 may be separated from the nozzle supporter 818 by an external force in the longitudinal direction.

Therefore, it may be desirable to provide the nozzle supporter hooks 818a at the coupling portion between the nozzle bar 816 and the nozzle supporter 818 to resist the external force in the longitudinal direction.

Accordingly, when the outer peripheral surface of the nozzle bar 816 is fitted into the inner peripheral surface of the nozzle supporter 818, the nozzle supporter hooks 818a provided on the nozzle supporter 818 may be fitted and fastened to the outer peripheral surface of the nozzle bar 816.

In this way, in the shoe care device 1 according to the present embodiment, since the nozzle supporter hooks 818a provided on the nozzle supporter 818 are fastened to the outer peripheral surface of the nozzle bar 816, the coupling between the nozzle bar 816 and the nozzle supporter 818 may be maintained more stably.

In the shoe care device 1 according to one embodiment of the present invention, the nozzle supporter 818 may further include a nozzle supporter rib 818b provided along the longitudinal direction of the nozzle bar 816 on the inner surface of the portion fastened to the nozzle bar 816.

As described above, since it is necessary for the nozzle duct 810 to perform hinge rotation in the state in which the nozzle bar 816 is coupled to the nozzle supporter 818, relatively large stress and deformation may occur in the portion where the nozzle bar 816 and the nozzle supporter 818 are coupled.

Therefore, it may be desirable to structurally reinforce the portion where the nozzle bar 816 and the nozzle supporter 818 are coupled to appropriately respond to applied stress and deformation.

Accordingly, by providing the nozzle supporter rib 818b in a concavo-convex structure on the inner surface of the nozzle supporter 818 coupled to the nozzle bar 816, greater structural rigidity may be achieved.

In this way, in the shoe care device 1 according to the present embodiment, since the nozzle supporter rib 818b is provided on the inner surface of the nozzle supporter 818 along the longitudinal direction of the nozzle bar 816, the rigidity of the nozzle supporter 818 coupled to the nozzle bar 816 may be reinforced.

In the shoe care device 1 according to one embodiment of the present invention, the nozzle supporter 818 may include a nozzle supporter hinge axis 813 and a hinge axis resistance part 814.

Specifically, the nozzle supporter hinge axis 813 is a part installed to enable hinge rotation at a portion coupled with the inner cabinet 100. The nozzle supporter hinge axis 813 provided in the nozzle supporter 818 is coupled to the inner cabinet 100 to make the nozzle duct 810 rotatable about the nozzle supporter hinge axis 813.

In this case, the angle of the nozzle duct 810 may be easily changed via the nozzle supporter hinge axis 813, but in order to maintain the nozzle duct 810 at the angle desired by the user, a configuration that limits the rotation of the nozzle supporter hinge axis 813 may be required.

When this configuration is not present, the nozzle duct 810 may not be maintained at the angle desired by the user, which may cause the nozzle 820 to sag downward due to gravity.

In addition, it may be impossible to hold the nozzle 820 at the uppermost portion within the accommodation space 101 when the nozzle 820 is not in use.

In particular, the nozzle duct 810 is has a structure in which one end is hinged to the inner cabinet 100, but the other end does not have a separate support member, in which since sagging due to the own weight of the nozzle duct inevitably increases toward the other end, it may be said that a separate configuration is required to prevent such sagging.

The hinge axis resistance part 814 is a part installed to limit the rotation of the nozzle supporter hinge axis 813, and may restrict the nozzle supporter hinge axis 813 from rotating at a predetermined pressure or lower.

Therefore, only when the user applies an external force of the predetermined pressure or lower, the nozzle supporter hinge axis 813 may rotate so that the angle of the nozzle duct 810 may be changed. In addition, when an external force of the predetermined pressure or lower is not applied, the rotation is limited by the hinge axis resistance part 814 so that the angle of the nozzle duct 810 may be maintained.

As described above, in the shoe care device 1 according to the present embodiment, the nozzle supporter 818 is hinged to the inner cabinet 100 via the nozzle supporter hinge axis 813, and the nozzle supporter 818 is supported on the inner cabinet 100 via the hinge axis resistance part 814. Thus, if necessary, the angle of the nozzle duct 810 may be adjusted through hinge rotation relative to the inner cabinet 100, and the angle of the nozzle duct 810 may be maintained constant when the angle change of the nozzle duct 810 is not intended.

In the shoe care device 1 according to one embodiment of the present invention, the hinge axis resistance part 814 may cover the nozzle supporter hinge axis 813 and may be coupled to the inner cabinet 100 to generate a frictional force on the nozzle supporter hinge axis 813.

That is, the hinge axis resistance part 814 may be coupled to the inner cabinet 100, and the nozzle supporter hinge axis 813 may be interposed between the coupling surfaces of the hinge axis resistance part 814 and the inner cabinet 100.

Therefore, the frictional force may be generated between the nozzle supporter hinge axis 813 and the hinge axis resistance part 814 due to the close contact force with which the hinge axis resistance part 814 is coupled to the inner cabinet 100.

As a result, in the case where a pressure smaller than the frictional force generated by the hinge axis resistance part 814 is applied when the nozzle supporter hinge axis 813 is about to rotate, the rotation of the nozzle supporter hinge axis 813 may be limited.

In this case, the hinge axis resistance part 814 may be made of a member containing an elastic material, and may be made of a material with excellent strength and elasticity, such as acetal (POM).

In this way, in the shoe care device 1 according to the present embodiment, since the hinge axis resistance part 814 generates a frictional force on the nozzle supporter hinge axis 813, when an external force smaller than the generated frictional force is applied, the angle of the nozzle duct 810 may be maintained to achieve a stable state.

In the shoe care device 1 according to one embodiment of the present invention, a pair of nozzle supporter hinge axes 813 may be installed symmetrically on opposite side surfaces of the nozzle supporter 818.

As described above, the nozzle duct 810 has a structure in which one end is hinged to the inner cabinet 100, but the other end does not have a separate support member, in which sagging due to the own weight of the nozzle duct inevitably increases toward the other end.

In particular, when a torsional deformation occurs toward the other end of the nozzle duct 810 in addition to the sagging, there is a risk that the performance of the shoe care device 1 may deteriorate.

Therefore, in order to prevent torsional deformation, it is necessary to arrange the nozzle supporter hinge axes 813 symmetrically to prevent eccentricity from occurring toward the opposite side surfaces of the nozzle duct 810.

In this way, in the shoe care device 1 according to the present embodiment, since a pair of nozzle supporter hinge axes 813 are installed on the opposite side surfaces of the nozzle supporter 818, eccentricity that occurs when the hinge of the nozzle duct 810 rotates may be minimized.

In the shoe care device 1 according to one embodiment of the present invention, the hinge axis resistance part 814 may be installed on each nozzle supporter hinge axis 813. That is, the hinge axis resistance part 814 may be installed on each nozzle supporter hinge axis 813 to generate a frictional force.

When a pair of nozzle supporter hinge axes 813 are installed on the opposite side surfaces of the nozzle supporter 818, the frictional force that limits the rotation of the nozzle supporter hinge axes 813 also needs to be generated uniformly on the opposite side surfaces.

Otherwise, there is a risk that eccentricity may occur as described above and cause torsional deformation in the nozzle duct 810.

In this way, in the shoe care device 1 according to the present embodiment, since the hinge axis resistance part 814 is installed on each nozzle supporter hinge axis 813, a greater support force for the nozzle duct 810 may be ensured.

In the shoe care device 1 according to one embodiment of the present invention, the hinge axis resistance part 814 may include a first hinge axis holder 814a and a second hinge axis holder 814b.

Specifically, the first hinge axis holder 814a is a part that covers one surface of the nozzle supporter hinge axis 813, and may be in close contact with a portion of the outer peripheral surface of the nozzle supporter hinge axis 813.

The second hinge axis holder 814b is a part that covers the other surface of the nozzle supporter hinge axis 813 and is coupled to the first hinge axis holder 814a, and may be in close contact with the remaining portion of the outer peripheral surface of the nozzle supporter hinge axis 813.

That is, the hinge axis resistance part 814 may be coupled to the nozzle supporter hinge axis 813 by coupling the first hinge axis holder 814a and the second hinge axis holder 814b to each other in the state in which the nozzle supporter hinge axis 813 is interposed therebetween.

In this case, the first hinge axis holder 814a and the second hinge axis holder 814b may be coupled using separate fastening members or the like, and the first hinge axis holder 814a and the second hinge axis holder 814b may be formed in a shape corresponding to the axial cross-sectional shape of the nozzle supporter hinge axis 813.

In this way, in the shoe care device 1 according to the present embodiment, since the hinge axis resistance part 814 includes the first hinge axis holder 814a and the second hinge axis holder 814b, the nozzle supporter hinge axis 813 and the hinge axis resistance part 814 may be coupled more easily.

In the shoe care device 1 according to one embodiment of the present invention, a hinge axis holder groove 814c may be provided on the outer surface of the first hinge axis holder 814a, and a hinge axis holder hook 814d, which is fastenable to the hinge axis holder groove 814c, may be provided on the outer surface of the second hinge axis holder 814b.

As described above, when the first hinge axis holder 814a and the second hinge axis holder 814b are coupled using separate fastening members or the like, the number of fastening members is inevitably limited, and there is a risk that a gap may occur between the first hinge axis holder 814a and the second hinge axis holder 814b in the portion where the fastening members are not disposed.

When a gap occurs between the first hinge axis holder 814a and the second hinge axis holder 814b as described above, the frictional force between the hinge axis resistance part 814 and the nozzle supporter hinge axis 813 may decrease, making it difficult for the function of the hinge axis resistance part 814 to be performed properly.

Accordingly, it may be desirable to further improve the fastening force by providing hook and groove structures, which are fastenable to each other, on the outer surfaces of the first hinge axis holder 814a and the second hinge axis holder 814b.

In this way, in the shoe care device 1 according to the present embodiment, since the hinge axis holder hook 814d provided on the second hinge axis holder 814b is fastened to the hinge axis holder groove 814c provided on the first hinge axis holder 814a, the coupling between the first hinge axis holder 814a and the second hinge axis holder 814b may be maintained more stably.

The shoe care device 1 according to one embodiment of the present invention may include a nozzle connector 830 that is installed separately from the nozzle duct 810, hinged to the inner cabinet 100 at one end, and hinged to the nozzle 820 at the other end.

That is, one end and the other end of the nozzle duct 810 are hinged to the inner cabinet 100 and the nozzle 820, respectively, and, separately from the nozzle duct 810, one end and the other end of the nozzle connector 830 are hinged to the inner cabinet 100 and the nozzle 820, respectively. Thus, the angle of the nozzle 820 may be maintained constant even when the angle of the nozzle duct 810 is changed.

In the shoe care device 1 according to one embodiment of the present invention, the nozzle connector 830 may be disposed under the nozzle supporter 818, and the nozzle supporter 818 may have a nozzle supporter groove 818d provided on the lower surface thereof to correspond to the shape of the upper surface shape of the nozzle connector 830.

As described above, it is desirable for the nozzle connector 830 to perform the above-described functions without problems while being as little exposed to the user as possible.

Accordingly, the nozzle connector 830 may be disposed under the nozzle bar 816, and accordingly, the nozzle connector 830 may be disposed under the nozzle supporter 818.

In this case, depending on the hinge rotation of the nozzle duct 810, the nozzle connector 830 and the nozzle supporter 818 also move to a predetermined angle, and in that process, there is a risk that interference between the nozzle supporter 818 and the nozzle connector 830 may occur.

Therefore, it may be desirable to form the nozzle supporter groove 818d in the lower surface of the nozzle supporter 818 that is to come into contact with the upper surface of the nozzle connector 830 to correspond to the shape of the nozzle connector 830, and to ensure that the upper surface of the nozzle connector 830 is engaged with the nozzle supporter groove 818d.

In this way, in the shoe care device 1 according to the present embodiment, since the upper surface of the nozzle connector 830 is engaged with the nozzle supporter groove 818d provided in the lower surface of the nozzle supporter 818, interference between the nozzle connector 830 and the nozzle supporter 818 may be minimized.

In the shoe care device 1 according to one embodiment of the present invention, the lower discharge port 821 may have a discharge slope 821a configured to guide sprayed air with a shape that decreases in height from the front to the rear of the inner cabinet 100.

In the nozzle 820, the lower discharge port 821, which opens downward, may be provided, and in the state in which the nozzle 820 is inserted into a shoe, air and/or steam may be sprayed into the shoe through the lower discharge port 821.

In this case, since the nozzle 820 is inserted into the interior of a shoe from above the heel of the shoe, air and/or steam may be smoothly sprayed onto the heel of the shoe, but it may be difficult for the sprayed air and/or steam to move evenly to the toe of the shoe.

Therefore, as illustrated in FIG. 32, it may be desirable to provide the discharge slope 821a at the lower discharge port 821 such that the sprayed air and/or steam is guided along the discharge slope 821a.

In this way, in the shoe care device 1 according to the present embodiment, since the discharge slope 821a is configured to guide the steam sprayed from the lower discharge port 821 toward the rear side of the inner cabinet 100, the steam may be sprayed to the toe of the shoe in the state in which the lower discharge port 821 is inserted into the interior of the shoe.

In the shoe care device 1 according to one embodiment of the present invention, the lower discharge port 821 may have a discharge through hole 821b in a portion of the discharge slope 821a.

When the sprayed steam is guided to the toe of the shoe through the discharge slope 821a as described above, the steam sprayed to the heel of the shoe may be insufficient.

Therefore, as illustrated in FIG. 32, it may be desirable to provide the discharge through hole 821b in a portion of the discharge slope 821a such that, when guiding the steam to the toe of the steam, some of the sprayed steam is sprayed onto the heel of the shoe through the discharge through hole 821b.

In this way, in the shoe care device 1 according to the present embodiment, since the discharge through-hole 821b is provided in a portion of the discharge slope 821a, steam may also be sprayed onto the heel of the shoe to a certain extent in the state in which the lower discharge port 821 is inserted into the interior of the shoe.

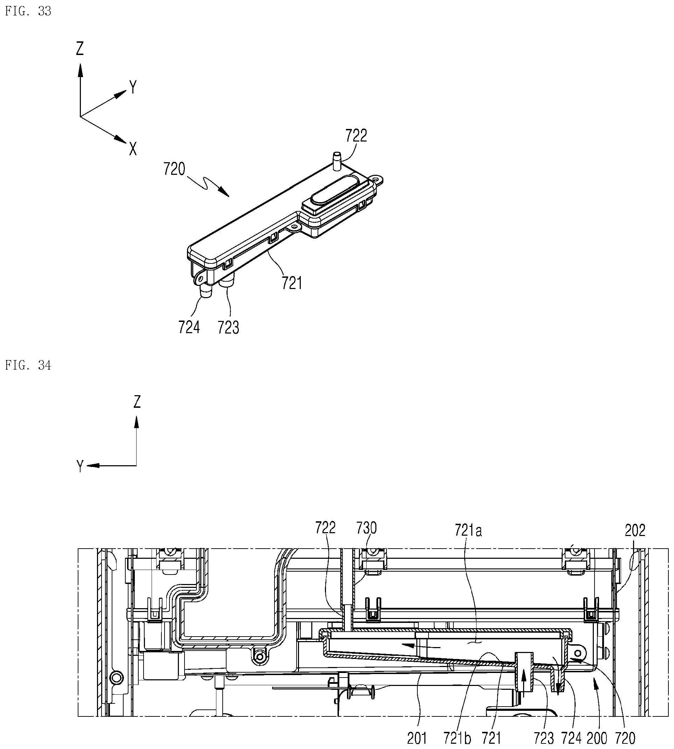

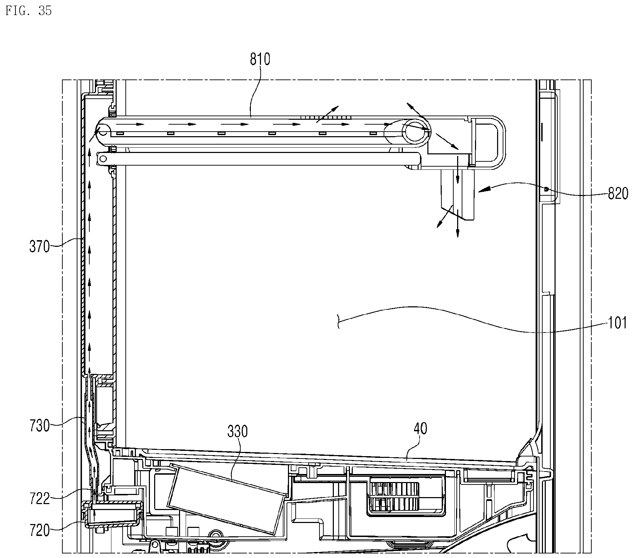

FIG. 33 is a perspective view illustrating a steam separator 720 according to one embodiment of the present invention. FIG. 34 is a cross-sectional view illustrating a separating inlet 723 of the steam separator 720 in the shoe care device 1 according to one embodiment of the present invention. FIG. 35 is a view schematically illustrating the flow of steam supplied to the inner cabinet 100 in the shoe care device according to one embodiment of the present invention.

The shoe care device 1 according to one embodiment of the present invention may further include a steam separator 720.

Most of the steam supplied from the steam generator 700 to the accommodation space 101 in the inner cabinet 100 is in a gaseous state, but may be condensed during movement and generate condensed water in a liquid state.

As described above, measures are needed to prevent moisture from remaining in unintended locations inside the shoe care device 1.

This also applies to the flow path through which steam is supplied, and it is necessary to prevent condensed water in steam from being adsorbed and remaining on the inner surface of the flow path through which steam is supplied.

Even when the condensed water in the steam is supplied into the inner cabinet 100 as is, since the condensed water is not provided to the shoe in the form of steam, it is difficult to properly perform shoe care (sterilization with high-temperature steam, swelling of the shoe material, and the like).

Therefore, it is desirable to remove the condensed water in the steam by the steam separator 720 before the condensed water in the steam flows into the inner cabinet 100.

In the shoe care device 1 according to one embodiment of the present invention, the steam separator 720 may include a separating base 721, a separating connector 722, a separating inlet 723, and a separating outlet 724.

The separating base 721 may be in the form of a housing that defines a predetermined internal space 721a.

The separating inlet 723 provides an inlet through which steam flows into the steam separator 720. The separating inlet 723 may be connected to a steam valve 710, and the steam generated in the steam separator 720 may pass through the steam valve 710 and then flow into the steam separator 720 through the separating inlet 723. The separating inlet 723 may be located lower than the separating connector 722. The separating inlet 723 may be provided in a form vertically opened at the lower side of the separating base 721.

The separating inlet 723 may be provided in the form of a vertical tube, and its upper end may be provided to be higher than the bottom surface 721b of the separating base 721. The separating inlet 723 may protrude upward from the bottom surface 721b of the separating base 721. Accordingly, even when condensed water is generated inside the separating base 721 and flows to the bottom surface 721b of the separating base 721, the condensed water can be prevented from flowing into the separating inlet 723, and as will be described later, all the condensed water can be discharged through the separating outlet 724.

The separating connector 722 provides an outlet through which the steam inside the steam separator 720 is discharged. The steam inside the steam separator 720 may move to a steam connection pipe 730 through the separating connector 722. The separating connector 722 may be provided on the upper side of the separating base 721. The separating connector 722 may be provided in the form opened vertically upward from the upper side of the separating base 721, and may be directly connected to and communicate with the steam connection pipe 730.

The separating outlet 724 provides an outlet through which the condensed water inside the steam separator 720 is discharged. The separating outlet 724 may be connected to the sump 600, and the condensed water inside the steam separator 720 may move to the sump 600 through the separating outlet 724. The separating outlet 724 may be located lower than the separating connector 722. The separating outlet 724 may be provided in a form opened vertically downward at the lower side of the separating base 721.

The separating outlet 724 may be provided at the lower end of the bottom of the separating base 721. That is, the separating outlet 724 may be provided at the lowest point of the bottom surface of the separating base 721. The bottom surface of the separating base 721 may be inclined downward toward the separating outlet 724. In one embodiment, the bottom surface of the separating base 721 may be inclined downward along a second direction Y or a direction opposite to the second direction Y, and the separating outlet 724 may be provided in the bottom surface of the separating base 721 at the front or rear end in the second direction Y

The steam flowing into the steam separator 720 may be heated to a predetermined temperature, or may have a temperature not lower than ordinary temperature (e.g., 20 ± 5°C). Since the steam has a strong tendency to rise, the steam may naturally move through the separating connector 722 provided upward from the upper side of the separating base 721.

Some of the steam flowing into the steam separator 720 may be cooled and condensed inside the separating base 721, and the condensed water may flow along the bottom surface of the separating base 721, be discharged to the exterior of the steam separator through the separating outlet 724, and move the sump 600.

As described above, in the shoe care device 1 according to one embodiment of the present invention, since the separating connector 722 and the separating outlet 724 are separately provided in the steam separator 720, when steam and condensed water coexist, the steam and the condensed water can be moved through separate paths, respectively.

In addition, the separating connector 722 is provided on the upper side of the steam separator 720 and the separating outlet 724 is provided on the lower side of the steam separator 720. Thus, steam and condensed water can be discharged in opposite directions depending on the respective properties of steam and condensed water, thereby preventing residual water from being generated in a flow path through which steam is supplied.

In the shoe care device 1 according to one embodiment of the present invention, the steam supplied to the inner cabinet 100 may be sprayed onto a shoe through the upper discharge port 811 and the lower discharge port 821.

When steam is supplied to the inner cabinet 100 to perform steam treatment on a shoe, the steam may be sprayed to the exterior of the shoe, but the steam may not be sprayed directly to the interior of the shoe.

Therefore, in order to ensure that steam is sprayed not only to the exterior of the shoe but also to the interior of the shoe, the steam supplied to the inner cabinet 100 may be moved to the nozzle duct 810 and the nozzle 820.

In this way, the steam moving to the nozzle duct 810 and the nozzle 820 may be sprayed to the exterior of the shoe through the upper discharge port 811 of the nozzle duct 810, and also sprayed to the interior of the shoe through the lower discharge port 821 of the nozzle 820.

In this way, in the shoe care device 1 according to the present embodiment, the steam supplied to the inner cabinet 100 may be sprayed not only to the exterior of the shoe through the upper discharge port 811 of the nozzle duct 810, but also to the interior of the shoe through the lower discharge port 821 of the nozzle 820. Thus, steam treatment of the interior and exterior of the shoe may be performed more appropriately.

The shoe care device 1 according to one embodiment of the present invention may further include a dry air duct 370 that forms a portion of the connection path F10 and guides air passing through the dehumidifying part 330 to the nozzle duct 810.

In this case, the steam generator 700 may supply steam to a portion of the dry air duct 370.

As described above, steam supplied to the inner cabinet 100 needs to be moved to the nozzle duct 810 and the nozzle 820. To this end, when a separate steam flow path interconnecting the steam generator 700 and the nozzle duct 810, the overall structure of the shoe care device 1 may be complicated.

In particular, since the nozzle duct 810 is already connected to the dry air duct 370, which forms a portion of the connection path F10, when steam is supplied to the dry air duct 370, the steam can be moved to the nozzle duct 810 and the nozzle 820.

In this way, in the shoe care device 1 according to the present embodiment, steam from the steam generator 700 is supplied to a portion of the dry air duct 370 and moved to the nozzle duct 810 and the nozzle 820. Thus, steam treatment of the shoe can be smoothly performed without providing a separate flow path for supplying steam to the inner cabinet 100.

The shoe care device 1 according to one embodiment of the present invention may further include a steam separator 720 and a steam connection pipe 730.

The steam separator 720 may be disposed between the steam generator 700 and the dry air duct 370 to remove condensed water in the steam.

The steam connection pipe 730 is a part that interconnects the separating connector 722 configured to discharge the steam inside the steam separator 720 and a portion of the dry air duct 370, and may provide a passage through which steam moves from the steam separator 720 to the dry air duct 370.

In this way, in the shoe care device 1 according to the present embodiment, steam from the steam generator 700 is supplied to the steam separator 720 and then moves to the dry air duct 370 through the steam connection pipe 730. Thus, the condensed water in the steam may be removed in the steam separator 720, and then the steam may be supplied to the inner cabinet 100.

In the shoe care device 1 according to one embodiment of the present invention, the dry air duct 370 may be connected to the steam connection pipe 730 above the steam separator 720.

Since steam generally has a rising nature, it may be desirable for the steam supplied from the steam generator 700 to move along an upward path.

Therefore, it may be desirable to dispose the dry air duct 370 above the steam separator 720 so that rising steam can be naturally moved from the steam separator 720 to the dry air duct 370 through the steam connection pipe.

In this way, in the shoe care device 1 according to the present embodiment, since the dry air duct 370 is disposed above the steam separator 720 and steam moves upward, the steam can be moved more smoothly due to its rising nature.

In the shoe care device 1 according to one embodiment of the present invention, the steam supplied to the inner cabinet 100 may be distributed to and sprayed from the upper discharge port 811 and the auxiliary discharge port 824 and then sprayed to the interior of the shoe through the lower discharge port 821.

As described above, when steam is sprayed to the interior of a shoe through the lower discharge port 821 of the nozzle 820, a certain amount of condensed water may be generated in the nozzle 820, and this condensed water may flow into the interior of the shoe due to the spray pressure of steam.

In this way, when the condensed water flows into the interior of the shoe, it may be difficult to effectively treat the shoe. Thus, it is necessary to minimize the flow of condensed water into the interior of the shoe.

That is, when spraying steam into the interior of the shoe, it is necessary to minimize the spray pressure of steam generated at the lower discharge port 821 to prevent condensed water inside the nozzle 820 from flowing into the shoe due to the spray pressure of steam.

To this end, it may be desirable for the spray pressure of the steam supplied from the steam generator 700 to be lowered to a certain extent before the steam reaches the lower discharge port 821.

In this regard, as illustrated in FIG. 35, in the shoe care device 1 according to the present embodiment, the steam supplied from the steam generator 700 may be distributed at the upper discharge port 811 and the auxiliary discharge port 824 before reaching the lower discharge port 821.

For example, the amounts of steam distributed to the upper discharge port, the auxiliary discharge port, and the lower discharge port may be divided into 3:3.5:3.5.

Accordingly, the spray pressure of the steam sprayed from the lower discharge port 821 is relatively lowered, so that the flow of condensed water into the shoes due to the spray pressure of the steam can be minimized.

In this way, in the shoe care device 1 according to the present embodiment, since the steam supplied to the inner cabinet 100 is distributed to and sprayed from the upper discharge port 811 and the auxiliary discharge port 824 before being sprayed to the interior of the shoe, the spray pressure of steam at the lower discharge port 821 is relatively lowered, so that the flow of condensed water into the interior of the shoe can be minimized.

Meanwhile, in the process of supplying steam from the steam generator 700 to the inner cabinet 100, a certain amount of condensed water may also be generated in the nozzle duct 810. Since it is undesirable for the condensed water to flow into the interior of the shoe through the nozzle 820, it is necessary to prevent the condensed water generated in the nozzle duct 810 from moving to the nozzle 820 as much as possible.

In relation to this, as illustrated in FIGS. 18, 21, and 22, nozzle's water storage portions 819 is provided along the longitudinal direction in the lower portion of the nozzle duct 810. Thus, a certain amount of condensed water generated in the nozzle duct 810 may be stored in the nozzle's water storage portions 819.

These nozzle's water storage portions 819 may be provided at opposite side surfaces of the above-described nozzle groove 812 along the longitudinal direction of the same.

As illustrated in FIGS. 18, 21, and 22, the nozzle's water storage portions 819 are provided at the lowest level on the bottom surface of the nozzle duct 810. Thus, the condensed water inside the nozzle duct 810 can be collected in the nozzle's water storage portions 819.

In this case, steps may be formed in the nozzle's water storage portions 819 where the nozzle duct 810 and the nozzle 820 are connected to each other. Thus, even if the front side of the nozzle duct 810 is lowered to supply steam to the shoe, the condensed water stored in the water storage portions 819 may be blocked by the step not to be moved to the nozzle 820.

In contrast, the nozzle's water storage portions 819 at the portion where the nozzle duct 810 and the dry air duct 370 are connected have no steps or are formed with a relatively gentle slope. Thus, after the supply of steam to the shoe is completed, when the front side of the nozzle duct 810 is raised, the condensed water stored in the nozzle's water storage portions 819 may be moved to the dry air duct 370.

In addition, the condensed water moved to the dry air duct 370 may be discharged to the condenser 400 in the state in which the damper 350 opens the dry air outlet 231.

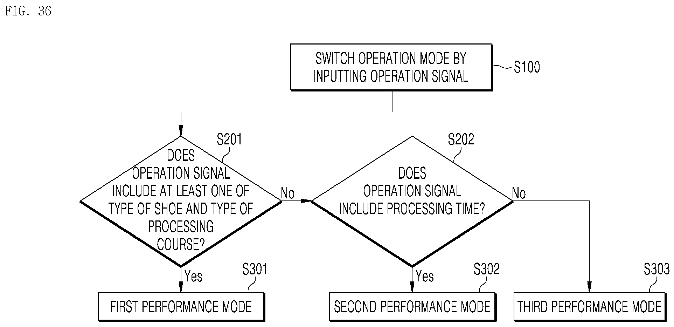

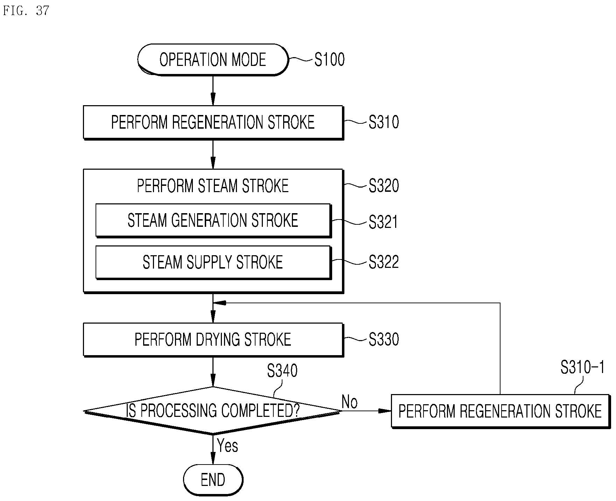

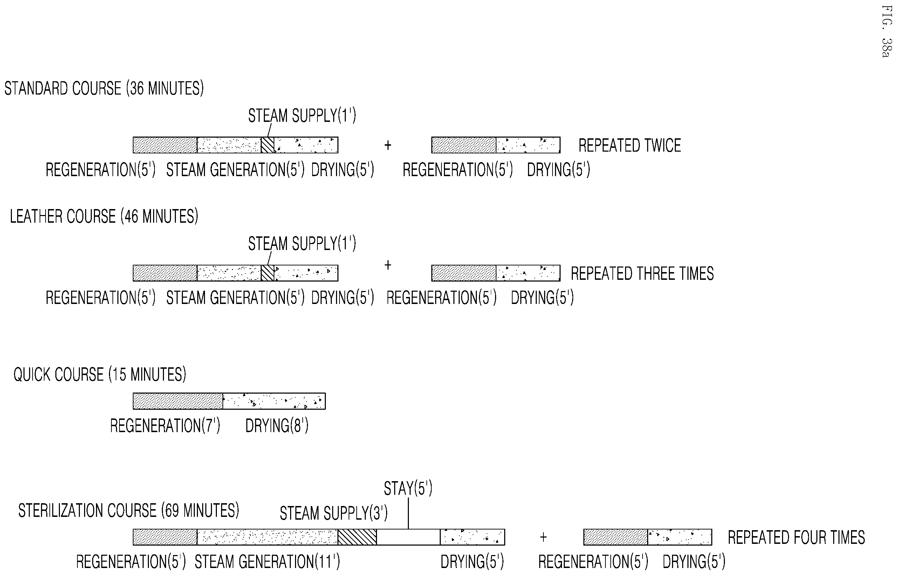

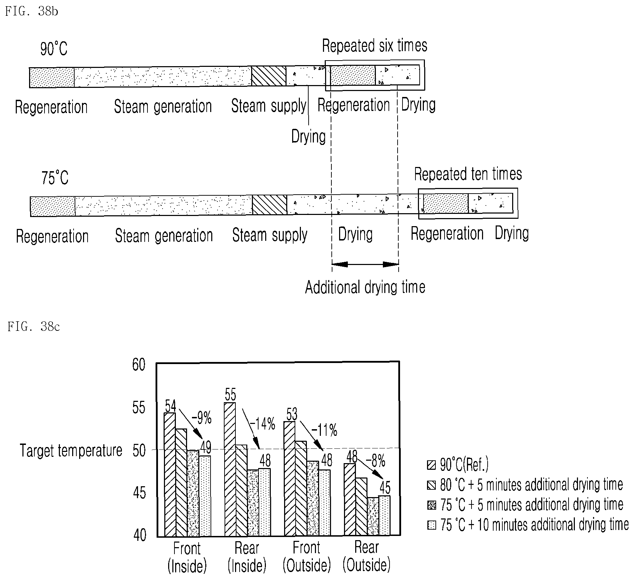

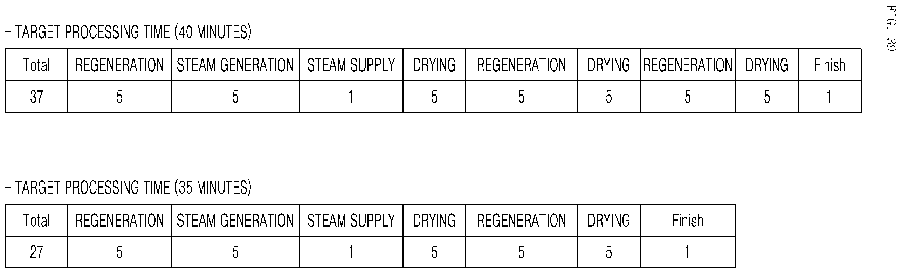

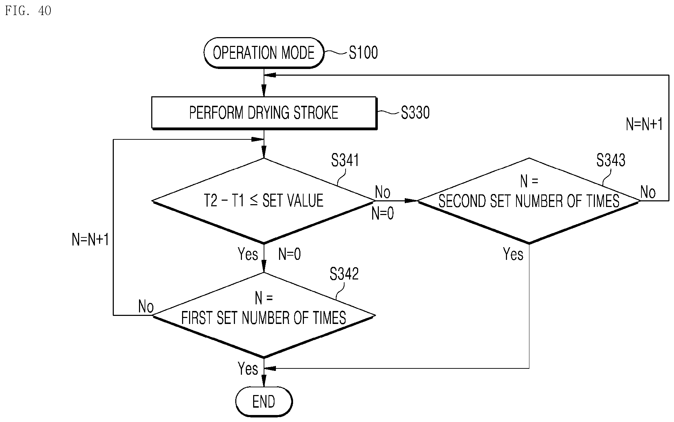

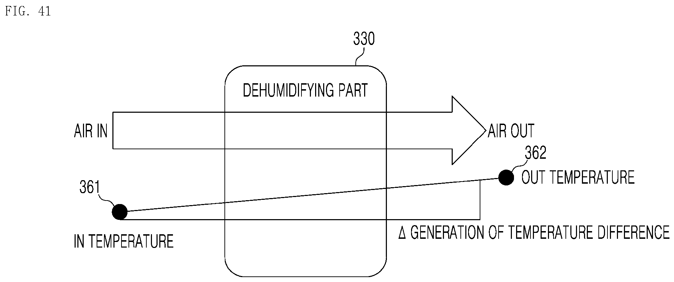

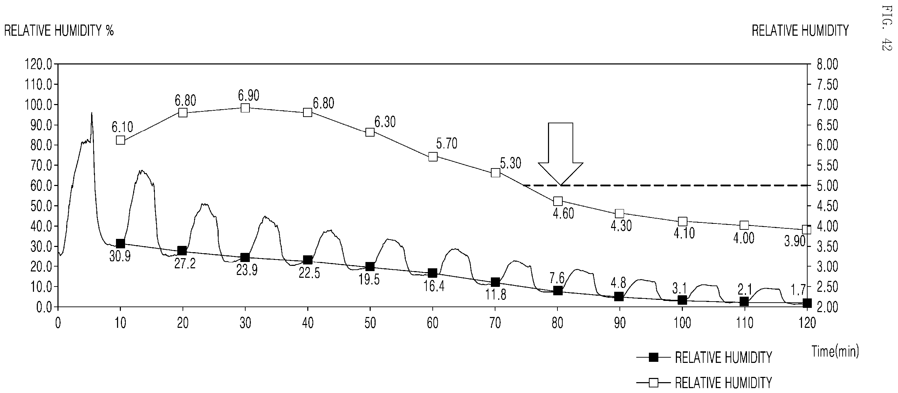

FIG. 36 is a view illustrating the progress of a stroke according to the type of an operation signal in a method of controlling the shoe care device 1 according to one embodiment of the present invention. FIG. 37 is a view showing the progress of strokes after switching to an operation mode in the method of controlling the shoe care device 1 according to one embodiment of the present invention. FIG. 38a is a view illustrating, by way of an example, a first execution mode in the method of controlling the shoe care device 1 according to one embodiment of the present invention. FIG. 38b is a view illustrating a modification of a sterilization course in the first execution mode of the shoe care device according to one embodiment of the present invention. FIG. 38c is a view illustrating temperature changes inside and outside a shoe according to the execution of the sterilization course illustrated in FIG. 38b. FIG. 39 is a view illustrating, by way of an example, a second execution mode in the method of controlling the shoe care device 1 according to one embodiment of the present invention. FIG. 40 is a view illustrating, by way of an example, a third execution mode in the method of controlling the shoe care device 1 according to one embodiment of the present invention. FIG. 41 is a view illustrating a change in temperature of air passing through the dehumidifying part 330 in the shoe care device 1 according to one embodiment of the present invention. FIG. 42 is a view illustrating a change in humidity according to temperature differences of air passing through the dehumidifying part 330 in the shoe care device 1 according to one embodiment of the present invention.

According to an embodiment of the present invention, a shoes care device 1 may be configured to include an inner cabinet 100, a connection path F10, a blowing part 310, a dehumidifying part 330, a heating part 320, a regeneration path F20, a controller 10, and a control panel 33.

In this case, the controller 10 may control a regeneration stroke and a drying stroke according to the type of operation signal input into the control panel 33.

The controller 10 as a part that controls the shoes care device 1 may control a module chamber 210 so that the connection path F10 and the regeneration path F20 are selectively opened/closed according to the dehumidifying part 330 being heated.

That is, the controller 10 may selectively open/close the connection path F10 and the regeneration path F20 according to the dehumidifying part 330 being regenerated.

As a result, the controller 10 may control a drying stroke in which air is moved and dehumidified along the connection path F10 to dry an accommodation space 101 and a regeneration stroke in which the air is moved and heated along the regeneration path F20 to dry the dehumidifying part 330 to be selectively performed.

The control panel is a part into which the operation signal is inputtable by a user, and the user manipulates the control panel 330 to input the operation signal for each component of the shoes care device 1.

In this case, the control panel 33 may be configured to include a touch screen. A control unit (controller 10) linked with the control panel 33 and controlling each component of the shoes care device 1 is provided in an inner space part of the door 30. The controller 10 may be provided inside a machine room 50.

In addition, the user interface (UI) is disposed on the control panel 33, and the user manipulates the UI disposed on the control panel 33 to input enter the operation signal for each component of the shoes care device 1.

Meanwhile, when the user inputs the operation signal into the control panel 33, the user may input a specific operation signal by considering in which stroke order, and/or time to operate the shoes care device 1.

Accordingly, in that a stroke order and/or time desired by the user is reflected to the type of operation signal input into the control panel 33, the shoes care device 1 need to be operated according to such a user's intention.

As a result, the controller 10 may be preferably configured to control the regeneration stroker and the drying stroke by reflecting the user's intention through the type of operation signal input into the control panel 33.

As such, in the shoes care device 1 according to the embodiment, since the regeneration stroke and the drying stroke are made by an optimal stroke to which the user's intention is reflected according to the type of operation signal input by the user, the user may more conveniently and efficiently use the shoes care device 1.

In the shoes care device 1 according to an embodiment of the present invention, when the operation signal is input into the control panel 33, the controller 10 may control the regeneration stroke and the drying stroke to be performed at least once.

That is, if the processing for the shoes reaches an appropriate level through one regeneration and one drying stroke, the operation of the shoes care device 1 may be terminated, but otherwise, the regeneration stroke and the drying stroke may be repeatedly performed until the processing for the shoes reaches the appropriate level.

As such, in the shoes care device 1 according to the embodiment, since the regeneration stroke and the drying stroke are performed at least once in the operation mode, the stroke may be performed repeatedly until appropriate processing for the shoes is achieved.

The shoes care device 1 according to an embodiment of the present invention may further include a steam generator 700 configured to supply the steam to the inner cabinet 100.

In this case, the controller 10 may control a steam stroke of supplying the steam by the steam generator 700.

That is, the controller 10 controls the steam stroke in addition to the regeneration stroke and the drying stroke to allow the regeneration stroke, the drying stroke, and the steam stroke for processing the shoes to be appropriately performed.

As such, in the shoes care device 1 according to the embodiment, since the steam stroke is performed in the operation mode, a refresh effect by puffing of a shoes material can be shown jointly with an effect of sterilization by a high temperature of steam.

In the shoes care device 1 according to the embodiment of the present invention, when the operation signal input into the control panel 33 includes at least one of the type of shoes and the type of processing course, the controller 10 may control the regeneration stroke, the drying stroke, and the steam stroke in a first performance mode of conducting the stroke in a predetermined order to correspond to the operation signal.

Here, the order predetermined to correspond to the operation signal refers to an order of the stroke which is most appropriately set based on experimental data in a design and manufacturing process of the shoes care device 1.

For example, as illustrated in FIG. 38a, when the type of shoes is not general, and special processing is required like leather, the user may input the type of shoes such as 'leather' into the control panel 33.

As a result, the shoes care device 1 may perform processing for the shoes according to the stroke predetermined in a most appropriate order when the type of shoes is 'leather'.

Further, the user may directly select the type of processing course for processing the shoes as 'standard course', `quick course', and `sterilization course', and input the selected type into the control panel 33.

As a result, the shoes care device 1 may perform the processing for the shoes according to the stroke predetermined in the most appropriate order in each course selected by the user

As such, in the shoes care device 1 according to the embodiment, when the operation signal includes at least one of the type of shoes and the type of processing course, the first performance mode in which the stroke is conducted in the predetermined order, a stroke to which the user's intention to conduct the processing for the shoes in a specific order may be performed.

In the shoes care device 1 according to an embodiment of the present invention, when the operation signal input into the control panel 33 includes a processing time, the controller 10 may control the regeneration stroke, the drying stroke, and the steam stroke in a second performance mode of distributing and conducting the stroke within the processing time of the operation signal.

That is, when the user intentionally inputs a time when the processing of the shoes is terminated, the stroke may be performed so that best processing for the shoes is performed within a limit not exceeding the processing time.

For example, as illustrated in FIG. 39, when the user inputs a target processing time as the time when the processing for the shoes is terminated as 40 minutes, the shoes care device 1 may conduct the stroke within the limit not exceeding 40 minutes.

In this case, since the regeneration stroke and the drying stroke are performed at one cycle, and the steam stroke may be preferably performed between one regeneration stroke and one drying stroke, each stroke may be distributed and conducted for a total of 37 minutes within the target processing time of 40 minutes.

In addition, when the user inputs the target processing time as the time when the processing for the shoes is terminated as 35 minutes, each stroke may be distributed and conducted for a total of 27 minutes within the target processing time of 30 minutes by considering such a point.

In this case, when the regeneration stroke and the drying stroke which are one cycle are further performed, the target processing time may be exceeded, the stroke may be controlled to be conducted only up to 27 minutes.

Meanwhile, the stroke may be conducted so that a total stroke time does not exceed the target processing time as described above, but besides, the stroke may also be controlled to be conducted within a limit in which the total stroke time minimally exceeds the target processing time through a case where the user changes setting in advance.

That is, when the target processing time of FIG. 39 is 35 minutes, the regeneration stroke and the drying stroke which are one cycle are performed once more, so the stroke may be conducted up to a total of 37 minutes.

As such, in the shoes care device 1 according to the embodiment, when the operation signal includes the processing time, the second performance mode of distributing and conducting the stroke is performed within the processing time, a stroke to which the user's intention to process the shoes is reflected may be performed within a specific processing time.

In the shoes care device 1 according to an embodiment of the present invention, when the operation signal input into the control panel 33 does not include the type of shoes, the type of processing course, and the processing time, the controller 10 may control the regeneration stroke, the drying stroke, and the steam stroke in a second performance mode of conducting the stroke up to a state of being capable of estimating that the processing for the shoes is completed.

Here, the state of being capable of estimating that the processing for the shoes is completed refers to a state in which it may be judged that appropriate processing is made by sensing temperatures and/or humidities of the shoes, the accommodation space, and the connection path F10 through a separate component such various sensors.

For example, as illustrated in FIG. 40, an air temperature difference (T2 - T1) before and after passing through the dehumidifying part 330 is sensed to judge that the appropriate processing is made.

When the user just operates the shoes care device 1 without inputting a particular operation signal, the user may intend to conduct optimal processing for the shoes without regarding the stroke order or the processing time.

As a result, in the shoes care device 1, the stroke may be conducted until reaching the optimal processing state for the shoes, and then automatically terminated.

As such, in the shoes care device 1 according to the embodiment, when the operation signal does not include the type of shoes, the type of processing course, and the processing time, the third performance mode in which the stroke is conducted up to the state of being capable of estimating that the processing for the shoes is completed, so the stroke to which the user's intention to conduct the optimal processing for the shoes is reflected may be performed regardless of the order or the processing time.

In the shoes care device 1 according to an embodiment of the present invention, the controller 10 may control the regeneration stroke to be performed earlier than the drying stroke.

That is, since the regeneration stroke and the drying stroke are performed at one cycle, and the regeneration cycle is performed before the drying stroke at such a cycle, so the dehumidifying part 330 regenerated before the progress of the drying stroke may continuously perform the dehumidification function in the best state.

In the shoes care device 1 according to an embodiment of the present invention, the controller 10 may control the steam stroke to be performed between one regeneration stroke and one drying stroke.

That is, since the steam stroke is performed between one regeneration stroke and one drying stroke in the operation mode, a humidity which may be relatively high in the steam stroke is repeatedly lowered in a subsequent drying stroke to enhance the processing efficiency for the shoes.

Hereinafter, a control method of the shoes care device 1 according to an embodiment of the present invention will be described.

First, an operation signal is input by the user in a standby mode state, which is switched to an operation mode (S100). In this case, the user may input the operation signal through the control panel 33.

Next, a regeneration stroke S310 in which air is moved and heated along the regeneration path F20 to regenerate the dehumidifying part 330 and a drying stroke S330 in which the air is moved and dehumidified along the connection path F10 to dry the accommodation space 101 are performed.

In this case, steps S310 and S330 may be performed according to the type of operation signal. That is, the type of operation signal is distinguished in steps S201 and S202 illustrated in FIG. 36, and as a result, steps S310 and S330 may be conducted.

As a result, in that the stroker order and/or time desired by the user are reflected to the type of operation signal input by the user, steps S310 and S330 may be performed according to the user's intention.

In the control method of the shoes care device 1 according to an embodiment of the present invention, after being switched to step S100, steps S310 and S330 may be performed at least once.

That is, in the shoes care device 1, steps S310 and S330 are conducted once, and then when it is judged that processing for shoes reaches an appropriate level in step S340, the operation of the shoes care device 1 may be terminated.

On the contrary, when it is judged that the processing for the shoes does not reach the appropriate level, steps S310-1 and S330 may be repeatedly performed until reaching the appropriate level.

The control method of the shoes care device 1 according to an embodiment of the present invention may further include a step S320 of performing the steam stroke of supplying steam to the accommodation space 101.

That is, the shoes care device 1 conducts steps S310, S320, and S330 to appropriately perform the regeneration stroke, the drying stroke, and the steam stroker for processing the shoes.

In the control method of the shoes care device 1 according to an embodiment of the present invention, when the operation signal includes at least one of the type of shoes and the type of processing course, steps S310, S320, and S330 may be performed in a first performance mode S301 of conducting the stroke in an order predetermined to correspond to the operation signal.

That is, as illustrated in FIG. 36, when it is judged that the operation signal includes at least one of the type of shoes and the type of processing course in step S201, the first performance mode S301 is performed to reflect a user's intention to process the shoes in a specific order.

In the control method of the shoes care device 1 according to an embodiment of the present invention, when the operation signal includes the processing time, steps S310, S320, and S330 may be performed in a second performance mode S302 of distributing and conducting the stroke within the processing time of the operation signal.

That is, as illustrated in FIG. 36, when it is judged that the operation signal includes the processing time in step S202, the second performance mode S302 is performed to reflect the user's intention to process the shoes in the specific order.

In the control method of the shoes care device 1 according to an embodiment of the present invention, when the operation signal does not include at least one of the type of shoes, the type of processing course, and the processing time, steps S310, S320, and S330 may be performed in a third performance mode S303 of conducting the stroke up to a state of being capable of estimating that the processing for the shoes is completed.

That is, as illustrated in FIG. 36, when it is judged that the operation signal does not include the type of shoes, the type of processing course, and the processing time in steps S201 and S202, the third performance mode S303 is performed to reflect a user's intention to achieve the optimal processing for the shoes regardless of the order or the processing time.

In the control method of the shoes care device 1 according to an embodiment of the present invention, step S301 may be performed earlier than step S330.

That is, since step S330 is performed at one cycle, and step S310 is performed before step S330 at such one cycle, so the dehumidifying part 330 regenerated before the progress of the drying stroke may continuously perform the dehumidification function in the best state.

In the control method of the shoes care device 1 according to an embodiment of the present invention, step S320 may be performed between one step S310 and one step S330.

That is, the humidity which may be relatively high in step S320 is repeatedly lowered to enhance the processing efficiency for the shoes.

According to an embodiment of the present invention, the shoes care device 1 may be configured to include an inner cabinet 100, a connection path F10, a blowing part 310, a dehumidifying part 330, a heating part 320, a regeneration path F20, and a controller 10.

In this case, the controller 10 may control the regeneration stroker to be performed earlier than the drying stroke.

When the shoes care device 1 is in a standby mode for a long time, a large amount of moisture may be adsorbed in the dehumidifying part 330. When the drying stroke of the shoes care device 1 is immediately in such a state, a drying function through the dehumidifying part 330 may not be smoothly shown.

As a result, it may be preferable that when the shoes care device 1 is switched from the standby mode to the operation mode, the shoes care device 1 continuously performs the regeneration stroke preemptively.

Further, even when the shoes care device 1 is switched to the operation mode, and performs the drying stroke after the regeneration stroke, it may be preferable to continuously conduct the drying stroke for a long time.

The moisture in the air is collected by the dehumidifying part 330 during the drying stroke, and as the amount of the collected moisture increases, the drying function through the dehumidifying part 330 may be deteriorated.

Accordingly, it is preferable that the shoes care device 1 performs the drying stroker for a predetermined time, and then performs the regeneration stroker, and then perform performs the drying stroke.

In this case, performing the regeneration stroker after the processing for the shoes is completed may be not required in that when the shoes care device 1 is thereafter switched to the standby mode state, the performance of the dehumidifying part 330 may be deteriorated.

When all circumstances are comprehensively considered, it is preferable that the regeneration stroker and the drying stroker are achieved at one cycle so that the shoes care device 1 continuously performs the regeneration stroke preemptively, and then performs the drying stroke.

As described above, in the shoes care device 1 according to the embodiment, since the regeneration stroke and the drying stroke are performed at one cycle, and the regeneration cycle is performed before the drying stroke at such a cycle, so the dehumidifying part 330 regenerated before the progress of the drying stroke may continuously perform the dehumidification function in the best state.

In the shoe care device 1 according to one embodiment of the present invention, the controller 10 may control the air that has passed through the dehumidifying part 330 to flow into the accommodation space 101 while the first regeneration stroke is being executed.

In order to execute a sterilization function for a shoe accommodated in the accommodation space 101, steam may be sprayed as described above. However, the sterilization function may be executed more effectively when the accommodation space 101 is maintained at a predetermined temperature or higher for a predetermined period of time.

That is, it may be effective for the sterilization function to raise the temperature of the accommodation space 101 to a predetermined level before supplying steam to the accommodation space 101.

In addition, even when the accommodation space 101 has a predetermined temperature or higher, a shoe can be sterilized more effectively when the accommodation space 101 is in a relatively humid state rather than in a relatively dry state.

Meanwhile, in the regeneration stroke in which the heater 321 operates to heat the dehumidifying part 330, the air passing through the dehumidifying part 330 may also be in a highly humid state by being heated by the heater 321 and receiving moisture from the dehumidifying part 330.

Therefore, when the air passing through the dehumidifying part 330 flows into the accommodation space 101 while the first regeneration stroke is being performed, the accommodation space 101 may be in a high temperature and high humidity state suitable for the sterilization function.

However, in view of the fact that, in addition to the sterilization function, it is necessary for the shoe care device to be operated to remove moisture from the accommodation space 101, in the regeneration cycle after the second time, it may be desirable to cause the air passing through the dehumidifying part 330 to move to a regeneration path rather than flowing into the accommodation space 101.

In this way, in the shoe care device 1 according to the present embodiment, while the first regeneration stroke is being executed, the air passing through the dehumidifying part 330 flows into the accommodation space 101 to increase the temperature of the accommodation space 101. Thus, the humid air in the accommodation space 101 may be maintained at a predetermined temperature or higher, so that the sterilization function can be executed more appropriately.

Meanwhile, while the first regeneration stroke is being executed, the air that has passed through the dehumidifying part 330 may also flow directly into the interior of the shoe through the nozzle. Thus, the sterilization function can be achieved in the interior as well as the exterior of the shoe in the accommodation space 101.

The shoes care device 1 according to an embodiment of the present invention may further include the control panel 33 capable of inputting the operation signal by the user.

In this case, when the operation signal is input into the control panel 33, the controller 10 may control the regeneration stroke and the drying stroke to be performed at least once.

As such, in the shoes care device 1 according to the embodiment, since the regeneration stroke and the drying stroke are performed at least once in the operation mode, the stroke may be performed repeatedly until appropriate processing for the shoes is achieved.

The shoes care device 1 according to an embodiment of the present invention may further include a steam generator 700 configured to supply the steam to the inner cabinet 100.

In this case, the controller 10 may control a steam stroke of supplying the steam by the steam generator 700.

As such, in the shoes care device 1 according to the embodiment, since the steam stroke is performed in the operation mode, a refresh effect by puffing of a shoes material can be shown jointly with an effect of sterilization by a high temperature of steam.

In the shoes care device 1 according to an embodiment of the present invention, the controller 10 may control the steam stroke to be performed between one regeneration stroke and one drying stroke.

As such, in the shoes care device 1 according to the embodiment, since the steam stroke is performed between one regeneration stroke and one drying stroke in the operation mode, the humidity which may be relatively high in the steam stroke is repeatedly lowered in a subsequent drying stroke to enhance the processing efficiency for the shoes.

In particular, before executing a steam stroke, in the first regeneration stroke, the air that has passed through the dehumidifying part 330 may flow into the accommodation space 101 to increase the temperature of the accommodation space. Therefore, the generation of condensed water can be minimized by preheating the nozzle duct 810 and the nozzle 820 through which steam moves.

In addition, after executing the steam stroke, the condensed water generated in the nozzle duct 810 and the nozzle 820 can be dried through the subsequent drying stroke.

In this way, in the shoe care device 1 according to the present embodiment, the steam stroke is executed between the first regeneration stroke and the first drying stroke, and while the first regeneration stroke is being executed, the air that has passed through the dehumidifying part 330 flows into the accommodation space 101 to increase the temperature of the accommodation space 101. Thus, it is possible to minimize the generation of condensed water in the preheated nozzle duct 810 and nozzle 820 before executing the steam stroke.

The shoe care device 1 according to one embodiment of the present invention may further include a module housing 200 and a damper 350.

The module housing 200 may accommodate the blowing part 310, the dehumidifying part 330, and the heating part 320 to form a portion of the connection path F10, and may be provided with a dry air outlet 231 connected to the dry air duct 370 and a humid air outlet 232 connected to a regeneration path F20.

The damper 350 is installed in the module housing 200 to selectively shield the dry air outlet 231 and the humid air outlet 232.

In this case, the controller may perform control such that the steam stroke can be executed in the state in which the damper 350 shields the dry air outlet 231.

As described above, when the steam supplied from the steam generator 700 is moved to the dry air duct 370 and then to the inner cabinet 101, there is a risk that the steam in the dry air duct 370 may flow back into the module housing 200.

When the steam in the dry air duct 370 flows back into the module housing 200, a large amount of steam may come into contact with the dehumidifying part 330. Thus, there is a risk that the function of the dehumidifying part 330 may be deteriorated.

Therefore, while the steam stroke is being executed, it may be desirable to make the damper 350 shield the dry air outlet 231 to prevent the steam in the dry air duct 370 from flowing back into the module housing 200.

In this way, in the shoe care device 1 according to the present embodiment, since the steam stroke is executed in the state in which the damper 350 shields the dry air outlet 231 of the module housing 200, it is possible to prevent steam from flowing back and damaging the dehumidifying part 330 at the time of executing the steam stroke.

The shoe care device 1 according to one embodiment of the present invention may further include a condenser 400 that forms a portion of the regeneration path F20 and is connected to the humid air outlet 232 to condense moisture in the air moving along the regeneration path F20.

In this case, the condensed water inside the dry air duct 370 may be discharged to the condenser 400 in the state in which the damper 350 opens the dry air outlet 231.

As described above, during the execution of the steam stroke, since the damper 350 shields the dry air outlet 231, the condensed water generated inside the dry air duct 370 may not flow into the module housing 200 and may be collected around the shielded dry air outlet 231.

In addition, when the steam stroke is completed and the damper 350 opens the dry air outlet 231, the condensed water collected around the dry air outlet 231 may flow into the module housing 200 and then be discharged to the condenser 400 through the second condensed water discharge hole 234 and the like.

In this way, in the shoe care device 1 according to the present embodiment, since the condensed water inside the dry air duct 370 is discharged to the condenser 400 in the state in which the damper 350 opens the dry air outlet 231 of the module housing 200, the condensed water generated in the dry air duct 370 may be smoothly discharged after the steam stroke is completed.

In the shoes care device 1 according to an embodiment of the present invention, the steam stroker may include a steam generation stroke of conducting temperature rising up to a setting temperature by the steam generator 700 and generating the steam, and a steam supply stroker of supplying the steam generated by reaching the setting temperature to the inner cabinet 100.

The steam generator 700 need to generate the steam by heating water in order to supply the steam to the accommodation space 101. The generation of the steam may require relatively more time than a time of supplying the steam in that temperature rising is achieved up to the setting temperature.

Accordingly, it may be preferable that the steam stroker is divided into the steam generation stroker and the steam supply stroke to sufficiently generate the steam before the steam supply stroker is conducted.

As such, in the shoes care device 1 according to the embodiment, since the steam stroker includes the steam generation stroke and the steam supply stroke, a sufficient state of steam may be generated before supplying the steam to the accommodation space 101.

In the shoes care device 1 according to an embodiment of the present invention, the controller 10 may control the steam supply stroke to be performed in a state in which the blowing part 310 is driven.

In the steam generation stroker, it is enough if only the temperature rising of the steam generator 700 is achieved, and it is not necessary to operate other components of the shoes care device 1 such as the blowing part 310. When other components of the shoes care device 1 such as the blowing part 310 are still unnecessarily operated jointly, power efficiency may be deteriorated by overload.

On the contrary, in the steam supply stroker, other components of the shoes care device 1 such as the blowing part 310 related to the processing of the shoes need to be operated jointly. That is, the circulation air current is generated even during the steam supply stroke to smoothly process the shoes.

As a result, it may be preferable that the steam stroker is divided into the steam generation stroker and the steam supply stroke to perform the steam supply stroke jointly with the driving of the blowing part 310.

As such, in the shoes care device 1 according to the embodiment, since the steam supply stroke is performed in the state in which the blowing part 310 is driven, the circulation air current may be smoothly generated while the steam is supplied.

In the shoes care device 1 according to an embodiment of the present invention, the controller 10 may control the steam generation stroke to be performed in the state in which one regeneration stroke is being performed.

As described above, when other components of the shoes care device 1 are operated jointly, the power efficiency may be deteriorated by the overload.

However, conducting the steam generation stroke requiring relatively more time alone may increase an overall processing time for the shoes.

As a result, it may be preferable that the steam generation stroke is conducted jointly with other strokes within a limit not to deteriorate the power efficiency.

Meanwhile, as such, it may be preferable that the steam stroke is performed between one regeneration stroke and one drying stroke. In this case, when the steam generation stroke is performed jointly with one drying stroke, it may be inappropriate in that the steam supply stroke should be conducted after the termination of the drying stroke.

As a result, it may be preferable that the steam generation stroke is performed jointly with one regeneration stroke.

As such, in the shoes care device 1 according to the embodiment, since the steam generation stroke is performed in the state in which one regeneration stroke is being performed, a time required for the stroke before substantial shoes processing is not yet started may be minimized.

In addition, as described above, since it is possible to increase the temperature of the accommodation space 101 by allowing the first regeneration stroke to be executed simultaneously with the steam generation stroke, when the subsequent steam supply stroke is performed, the accommodation space 101 can maintain a high temperature and humidity state that is more appropriate for the sterilization function of shoes.

In the shoe care device 1 according to one embodiment of the present invention, the controller 10 may perform control such that the steam supply stroke is executed in the state in which the first regeneration stroke is completed.

As described above, it is desirable for the first regeneration stroke and the steam generation stroke to be executed simultaneously, but it may not be desirable for the first regeneration stroke to be executed while the steam supply stroke is being executed.

When the air that has passed through the dehumidifying part 330 flows into the accommodation space 101 while the first regeneration stroke is being executed, a certain amount of the air in the accommodation space 101 may flow back into the regeneration path F20.

In this case, when steam is supplied to the accommodation space 101, a certain amount of the steam may also flow into the regeneration path F20 and there is a risk that the steam may damage the operating heater 321.

Therefore, it may be desirable to control the first regeneration stroke and the steam supply stroke not to be executed simultaneously.

In this way, in the shoe care device 1 according to the present embodiment, since the steam supply stroke is executed in the state in which the first regeneration stroke is completed, damage to the heater 321 that may occur when the steam supply stroke and the regeneration stroke are executed simultaneously can be prevented.

On the other hand, after execution of the sterilization function for a shoe through the first regeneration stroke and the steam stroke, when the temperature of the accommodation space 101 during the drying stroke is too high, there is a risk that the shoe may be deformed or damaged.

Therefore, it is necessary to ensure that the internal and external temperatures of the shoe accommodated in the accommodation space 101 while the drying stroke is being executed are maintained at a target temperature (e.g., 50 °C) or lower.

To this end, as illustrated in FIGS. 38B and 38C, when the first regeneration stroke is executed, it may be desirable to set the temperature for heating the dehumidifying part 330 relatively low (e.g., to set the regeneration temperature to 75 °C).

In addition, by increasing (e.g., by adding 10 minutes to) the first drying stroke execution time to be relatively longer than the drying stroke execution time of the other rounds, the internal and external temperatures of the shoe accommodated in the accommodation space 101 during the dry stroke may be maintained at the target temperature (e.g., 50 °C) or less.

Meanwhile, when the target temperature of the accommodation space 101 is maintained at the target temperature or lower while the drying stroke is being executed, it may be desirable to additionally execute the regeneration stroke and the drying stroke in order to ensure that residual water inside the accommodation space 101 is properly removed even at a relatively low temperature.

Hereinafter, a control method of the shoes care device 1 according to an embodiment of the present invention will be described.

First, the standby mode state is switched to the operation mode (S 100). That is, the shoes care device 1 in the standby mode state is switched to the operation mode, so the stroke for processing the shoes may be started.

Next, a regeneration stroke S310 in which air is moved and heated along the regeneration path F20 to regenerate the dehumidifying part 330 and a drying stroke S330 in which the air is moved and dehumidified along the connection path F10 to dry the accommodation space 101 are performed.

In this case, step S310 may be performed earlier than step S330. That is, the regeneration stroke and the drying stroke may be made at one cycle so that the shoes care device 1 continuously performs the regeneration stroke preemptively, and then performs the drying stroke.

In a method of controlling the shoe care device 1 according to one embodiment of the present invention, step S310 of the first round may be executed such that the air that has passed through the dehumidifying part flows into the accommodation space

That is, at step S310 of the first round, the air that has passed through the dehumidifying part 330 flows into the accommodation space 101 to increase the temperature of the accommodation space 101, so that the humid air in the accommodation space 101 can be maintained at a predetermined temperature or higher above a certain temperature to ensure that the sterilization function can be executed more properly.

In the control method of the shoes care device 1 according to an embodiment of the present invention, step S100 may be performed by inputting the operation signal by the user. In this case, the user may input the operation signal through the control panel 33.

In addition, after being switched to step S100, steps S310 and S330 may be performed at least once.

That is, when it is judged that the processing for the shoes reaches the appropriate level, steps S310 and S330 may be conducted once or steps S310-1 and S330 may be repeatedly performed.

The control method of the shoes care device 1 according to an embodiment of the present invention may further include a step S320 of performing the steam stroke of supplying steam to the accommodation space 101.

That is, the shoes care device 1 conducts steps S310, S320, and S330 to appropriately perform the regeneration stroke, the drying stroke, and the steam stroker for processing the shoes.

In the control method of the shoes care device 1 according to an embodiment of the present invention, step S320 may be performed between one step S310 and one step S330.

That is, the humidity which may be relatively high in step S320 is repeatedly lowered to enhance the processing efficiency for the shoes.

In the control method of the shoes care device 1 according to an embodiment of the present invention, step S320 may include a step S321 in which the steam generation stroke of conducting the temperature rising up to the setting temperature and generating the steam by the steam generator 700 and a step S322 in which the steam supply stroke of supplying the steam generated by reaching the setting temperature to the accommodation space 101.

That is, the steam stroke is divided into the steam generation stroke and the steam supply stroke to sufficiently generate the steam before the steam supply stroke is conducted.

In the control method of the shoes care device 1 according to an embodiment of the present invention, step S322 may be performed in the state in which the blowing part 310 is driven.

That is, the circulation air current is generated by driving the blowing part 310 during the steam supply stroke to smoothly process the shoes.

In the control method of the shoes care device 1 according to an embodiment of the present invention, step S321 may be performed during step S310 in which one regeneration stroke is performed.

That is, step S321 is conducted jointly with step S310 within the limit not to deteriorate the power efficiency to minimize the overall processing time for the shoes.

In addition, the temperature of the accommodation space 101 can be increased by executing step S310 simultaneously with step S321, so that when the subsequent step S322 is executed, the accommodation space 101 can be maintained in a high temperature and humidity state more appropriate for the sterilization function of shoes.

In the method of controlling the shoe care device 1 according to one embodiment of the present invention, step S322 may be executed when step S310 is completed.

That is, since step S322 is executed in the state in which step S310 is completed, damage to the heater 321 that may occur when steps S322 and S310 are executed simultaneously may be prevented.

According to an embodiment of the present invention, the shoes care device 1 may be configured to include the inner cabinet 100, the connection path F10, the blowing part 310, the dehumidifying part 330, the controller 10, a first sensor 361, and a second sensor 362.

In this case, the controller may control the drying stroke to be performed up to a state in which it may be estimated that the processing for the shoes is completed through a temperature difference of air measured by the first sensor 361 and the second sensor 362 during the performing of the drying stroke.

Specifically, the controller 10 as a part that controls the shoes care device 1 may control the drying stroke in which the air is moved and dehumidified along the connection path F10 to dry the accommodation space 101.

The first sensor 361 as a part that is disposed in a part in which the air flows into the dehumidifying part 330 in the connection path F10 to measure the temperature of the air may measure a temperature the air before passing through the dehumidifying part 330 (see FIG. 9).

The second sensor 362 as a part in which the air is discharged from the dehumidifying part 330 in the connection path F10 to measure the temperature of the air may measure the temperature of the air after passing through the dehumidifying part 330 (see FIG. 9).

As illustrated in FIG. 41, as the humidity in the air passing through a dehumidification agent 331 (in particular, zeolite) of the dehumidifying part 330 is the higher, the temperature rising of the air due to heat dissipation of the dehumidification agent 331 may be achieved.

As a result, it may be estimated that a temperature difference between the air which flows into the dehumidifying part 330 and the air discharged from the dehumidifying part 330 is the larger, the humidity in the air is the higher On the contrary, the drying stroke is appropriately conducted, and as a result, as the humidity in the air is the lower, the temperature difference between the air which flows into the dehumidifying part 330 and the air discharged from the dehumidifying part 330 may become the smaller.

Accordingly, when the temperature difference between the air which flows into the dehumidifying part 330 and the air discharged from the dehumidifying part 330 is measured through the first sensor 361 and the second sensor 362, it may be determined whether the state in which it may be estimated that the processing for the shoes is completed is reached.

In addition, the controller 10 may control the number of performance times and/or a performance hour of the drying stroke until the processing for the shoes reaches the appropriate level based on a measurement result of the temperature difference.

As such, in the shoes care device 1 according to the embodiment, since the drying stroke is performed up to the state in which it may be estimated that the processing for the shoes is completed through the temperature difference between the air which flows into the dehumidifying part 330 and the air discharged from the dehumidifying part 330, the operation of the shoes care device 1 may be automatically terminated without a separate manipulation of the user.

In the shoes care device 1 according to an embodiment of the present invention, when the temperature difference is sensed as a set value or less, the controller 10 may control the drying stroke to be terminated by estimating that the processing for the shoes is completed.

In this case, the set value refers to any temperature difference corresponding to a relative humidity which may be handled as the processing for the shoes reaching the appropriate level through experimental data in advance.

For example, as illustrated in FIG. 42, when the drying stroke is performed, the relative humidity may be lowered. In this case, when it is handled that a case where the relative humidity is 10% or less is handled as the processing for the shoes reaching the appropriate level, a set value of a temperature difference corresponding thereto may be 5°C.

Therefore, when a temperature difference of the airs measured by the first sensor 361 and the second sensor 362, respectively is equal to or less than 5°C which is the set value, it is estimated that the processing for the shoes is completed to terminate the drying stroke.

As such, in the shoes care device 1, when the temperature difference is equal to or less than the set value, , the drying stroke is terminated, so an operation termination time of the shoes care device 1 may be more clearly determined.

The shoes care device 1 according to an embodiment of the present invention may further include the control panel 33 capable of inputting the operation signal by the user.

In this case, when the operation signal is input into the control panel 33, the controller 10 may control the regeneration stroke and the drying stroke to be performed at least once.

As such, in the shoes care device 1 according to the embodiment, since the regeneration stroke and the drying stroke are performed at least once in the operation mode, the stroke may be performed repeatedly until appropriate processing for the shoes is achieved.

In the shoes care device 1 according to an embodiment of the present invention, when it is continuously sensed at a first set number of times that the temperature difference is equal to or less than the set value, the controller 10 may control the drying stroke to be terminated by estimating that the processing for the shoes is completed.

As described above, when the temperature difference of the airs measured by the first sensor 361 and the second sensor 362, respectively is equal to or less than the set value, the drying stroke may be immediately terminated, but when the temperature is measured due to a measurement error of the first sensor 361 and the second sensor 362 or in a momentary special situation, the reliability may be deteriorated.

Accordingly, instead of immediately terminating the drying stroke when the temperature difference of the airs measured by the first sensor 361 and the second sensor 362, respectively is equal to or less than the set value, it may be preferable that the drying stroke is at last terminated when a case where the temperature difference is equal to or less than the set value is sensed continuously at a first set number of times or more.

In this case, the first set number of times may be appropriately set (for example, three times) through the experimental data in advance, and appropriately changed by considering an operating environment of the shoes care device 1 and user's tendency.

As such, in the shoes care device 1, when the temperature difference is equal to or less than the set value at the first set number of times or more, the drying stroke is terminated, so the reliability for whether the processing for the shoes being completed may be secured.

In the shoes care device 1 according to an embodiment of the present invention, when the number of sensing times of the temperature difference reaches a second set number of times, the controller 10 may control the drying stroke to be terminated by estimating that the processing for the shoes is completed.

As described above, when it is judged that the processing for the shoes reaches the appropriate level through the temperature difference of the airs measured by the first sensor 361 and the second sensor 362, respectively, the drying stroke may be terminated, but a case where the temperature difference does not decrease to the set value or less due to the measurement error of the first sensor 361 and the second sensor 362 or damage may occur.

In this case, it may be inefficient to continuously repeat the drying process because the processing time for the shoes is excessively longer.

Accordingly, regardless of the case where the temperature difference of the airs measured by the first sensor 361 and the second sensor 362, respectively is equal to or less than the set value, it may be preferable that an additional drying stroke is not conducted but terminated when the number of sensing times of the temperature difference reaches the second set number of times.

In this case, the second set number of times may be appropriately set (for example, 15 times) through the experimental data in advance, and appropriately changed by considering an operating environment of the shoes care device 1 and user's tendency.

As such, in the shoes care device 1 according to the embodiment, when the temperature difference is sensed by the second set number of times, the drying stroke is terminated, so it is possible to prevent the stroke for processing the shoes from being conducted excessively much.

In the shoes care device 1 according to an embodiment of the present invention, when the drying stroke is temporarily stopped, and then reperformed, the controller 10 may control the first set number of times and the second set number of times not to be initialized.

A case where the stroke is momentarily stopped due to the manipulation of the user or occurrence of an unexpected situation during the operation of the shoes care device 1 may occur.

When the stroke is momentarily stopped, and then the stroke is reperformed again, if the first set number of times and the second set number of times are initialized, the number of times of the drying stroke which is conducted for the processing of the shoes may be excessively increased.

In particular, in that a predetermined part of the processing for the shoes is conducted before momentary stop, and the processing for the shoes is reperformed, and the remaining steps of the processing for the shoes is continuously conducted, it may be not preferable that the first set number of times and the second set number of times are initialized.

As such, in the shoes care device 1 according to the embodiment, when the drying stroke is momentarily stopped, and then reperformed, the stroke is not initialized and the stroke is performed in connection with the stroke before the momentary stop, so the processing for the shoes is prevented from being excessively conducted, thereby minimizing the damage to the shoes.

The shoes care device 1 according to an embodiment of the present invention may further include a heating part 320 and a regeneration path F320.

In this case, the controller 10 may control the regeneration stroke in which the air is moved and heated along the regeneration path F20 to regenerate the dehumidifying part 330, and the drying stroke to be selectively performed.

In the shoes care device 1 according to an embodiment of the present invention, the controller 10 may control the regeneration stroke to be performed earlier than the drying stroke.

That is, since the regeneration stroke and the drying stroke are performed at one cycle, and the regeneration cycle is performed before the drying stroke at such a cycle, so the dehumidifying part 330 regenerated before the progress of the drying stroke may continuously perform the dehumidification function in the best state.

The shoes care device 1 according to an embodiment of the present invention may further include a steam generator 700 configured to supply the steam to the inner cabinet 100.

In this case, the controller 10 may control a steam stroke of supplying the steam by the steam generator 700.

As such, in the shoes care device 1 according to the embodiment, since the steam stroke is performed in the operation mode, a refresh effect by puffing of a shoes material can be shown jointly with an effect of sterilization by a high temperature of steam.

In the shoes care device 1 according to an embodiment of the present invention, the controller 10 may control the steam stroke to be performed between one regeneration stroke and one drying stroke.

As such, in the shoes care device 1 according to the embodiment, since the steam stroke is performed between one regeneration stroke and one drying stroke in the operation mode, the humidity which may be relatively high in the steam stroke is repeatedly lowered in a subsequent drying stroke to enhance the processing efficiency for the shoes.

The shoes care device 1 according to an embodiment of the present invention is coupled to a lower side of the inner cabinet 100 and has the module chamber 210 which is in communication with the accommodation space 101, and the module chamber 310 may further include may further include a module housing 200 including a first module chamber 212, a second module chamber 213, and a third module chamber 214.

In this case, the blowing part 310 is accommodated in the first module chamber 212 to blow the air of the module chamber 210, the heating part 320 is accommodated in the second module chamber 213 to heat the air of the module chamber 210, the dehumidifying part 330 is accommodated in the third module chamber 214 to dehumidify the air of the module chamber 210, and the first module chamber 212, the second module chamber 213, and the third module chamber 214 may be formed at different locations from each other on the plan view.

As such, since the shoes care device 1 according to the embodiment includes the first module number 212, the second module chamber 213, and the third module chamber 214 formed at different locations on the plan view and accommodating the blowing part 310, the heating part 320, and the dehumidifying part 330, respectively, a layout of each component for performing the regeneration stroke and the drying stroke may be optimized.

In the shoes care device 1 according to an embodiment of the present invention, the air may be configured to move in the first module chamber 212, the second module chamber 213, and the third module chamber 214 in sequence.

That is, since the air is configured to move in the first module chamber 212, the second module chamber 213, and the third module chamber 214 in sequence, the drying efficiency by the dehumidifying part 330 may be enhanced by connecting the third module chamber 214 and a drying flow path at a shortest distance, and when the dehumidifying part 330 is regenerated, the air heated by the heating part 320 directly moves to the dehumidifying part 330, so the regeneration efficiency of the dehumidifying part 330 may also be enhanced.

In the shoes care device 1 according to an embodiment of the present invention, the first sensor 361 is installed in the second module chamber 213 to measure the temperature of the air which flows into the dehumidifying part 330, and the second sensor 362 is installed in the third module chamber 214 to measure the temperature of the air discharged from the dehumidifying part 330.

That is, since the second module chamber 213 measures the temperature of the air which flows into the dehumidifying part 330, and the third module chamber 214 measures the temperature of the air discharged from the dehumidifying part 330, a layout of the first sensor 361 and the second sensor 362 for measuring the temperature of the air may be effectively achieved.

Referring to FIG. 40, hereinafter, a control method of the shoes care device 1 according to an embodiment of the present invention will be described.

First, the standby mode state is switched to the operation mode (S100). That is, the shoes care device 1 in the standby mode state is switched to the operation mode, so the stroke for processing the shoes may be started.

Next, a drying stroke S330 is performed in which the air is moved and dehumidified along the connection path F10 to dry the accommodation space 101.

Next, in step S341, an air temperature difference (T2 - T1) before and after passing through the dehumidifying part 330 is sensed to judge that appropriate processing is achieved. That is, it is judged whether the air temperature difference (T2 - T1) is equal to or less than a set value to judge whether processing for shoes reaches an appropriate level.

If the air temperature difference (T2 - T1) is equal to or less than the set value, it is judged whether such a state continuously reaches a first set number of times in step S342. As a result, when such a state reaches the first set number of times, it is judged that the processing for the shoes is completed to terminate the drying stroke.

On the contrary, if the air temperature difference (T2 - T1) is equal to or less than the set value, the drying stroke may be repeatedly performed. In this case, in step S343, it is judged whether the number of sensing times of the air temperature difference (T2 - T1) reaches a second set number of times. As a result, when the number of sensing times of the air temperature difference (T2 - T1) reaches the second set number of times, the drying stroke may be terminated in order to prevent the drying stroke from being performed excessively a lot.

Hereinabove, a specific embodiment of the present invention is described and illustrated, but the present invention is not limited to the disclosed embodiment, and it may be appreciated by those skilled in the art that the exemplary embodiment can be variously modified and transformed to another specific embodiment without departing from the spirit and the scope of the present invention. Therefore, the scope of the present invention will not be defined by the described embodiment, but defined by the technical spirit disclosed in the claims.

### INDUSTRIAL APPLICABILITY

By at least one of exemplary embodiments of the present invention, a dehumidifying part is disposed in a module chamber not only to collect moisture and bacteria in a ventilation air but also to regenerate the dehumidifying part by heating the dehumidifying part in the module chamber, thereby maintaining proper shoes treatment performance all the times.

In addition, by at least one of exemplary embodiments of the present invention, a connection path through which air circulates is formed between a outlet and a nozzle respectively disposed inside an inner cabinet, thereby preventing the air used for dehumidifying and deodorizing shoes from being exposed to the user.

In addition, by at least one of exemplary embodiments of the present disclosure, since the steam supplied to the inner cabinet is sprayed not only to the exterior of a shoe through the upper discharge port of the nozzle duct but also to the interior of the shoe through the lower discharge port of the nozzle, steam treatment of the interior and exterior of the shoe may be performed more appropriately.

In addition, by at least one of exemplary embodiments of the present invention, since the steam from the steam generator is supplied to a portion of the dry air duct and moved to the nozzle duct and nozzle, steam treatment of a shoe may be performed smoothly without providing a separate flow path for supplying steam to the inner cabinet.

In addition, by at least one of exemplary embodiments of the present invention, since the steam from the steam generator is supplied to the steam separator and then moved to the dry air duct through the steam connection pipe, the steam may be supplied to the inner cabinet after condensed water is removed from the steam in the steam separator.

In addition, by at least one of exemplary embodiments of the present invention, since the dry air duct is disposed above the steam separator so that steam is moved upward, steam may be moved more smoothly due to its rising nature.

In addition, by at least one of exemplary embodiments of the present invention, the steam stroke is executed between the first regeneration stroke and the first drying stroke, and while the first regeneration stroke is being executed, the air that has passed through the dehumidifying part flows into the accommodation space to increase the temperature of the accommodation space. Therefore, the generation of condensed water in the preheated nozzle duct and nozzle before executing the steam stroke may be minimized.

In addition, by at least one of exemplary embodiments of the present invention, since the steam stroke is executed in the state in which the damper shields the dry air outlet of the module housing, steam may be prevented from flowing back and damaging the dehumidifying part when executing the steam stroke.

In addition, by at least one of exemplary embodiments of the present invention, since the condensed water inside the dry air duct is discharged to the condenser in the state in which the damper opens the dry air outlet of the module housing, the condensed water generated in the dry air duct after completing the steam stroke may be discharged smoothly.

In addition, by at least one of exemplary embodiments of the present invention, since the nozzle includes the nozzle body and the nozzle protrusion, the lower discharge port provided on the nozzle protrusion may be more easily inserted into the interior of a shoe.

In addition, by at least one of exemplary embodiments of the present invention, since the nozzle duct is installed to face the front side from the rear wall of the inner cabinet, the internal space of the inner cabinet may be effectively utilized.

In addition, by at least one of exemplary embodiments of the present invention, since the discharge slope is provided to guide the steam sprayed from the lower discharge port to the rear side of the inner cabinet, in the state in which the lower discharge port is inserted into a shoe, steam may be sprayed to the toe of the shoe.

In addition, by at least one of exemplary embodiments of the present invention, the discharge through hole is provided in a portion of the discharge slope, in the state in which the lower discharge port is inserted into a shoe, a certain amount of steam may also be sprayed into the heel of the shoe.

In addition, by at least one of exemplary embodiments of the present invention, since steam is also sprayed through an auxiliary discharge port provided the nozzle body within the inner cabinet, steam may be sprayed to various portions in a shoe within the inner cabinet.

In addition, by at least one of exemplary embodiments of the present invention, since the steam supplied to the inner cabinet is distributed to and sprayed from the upper discharge port and the auxiliary discharge port before being sprayed into the interior of a shoe, the spray pressure of steam at the lower discharge port may be relatively lowered to minimize the condensed water flowing into the interior of the shoe.

In addition, by at least one of exemplary embodiments of the present invention, since the nozzle duct is coupled to the inner cabinet in a penetration structure, steam may be smoothly moved from the dry air duct to the nozzle duct.

In addition, by at least one of exemplary embodiments of the present invention, since the nozzle duct is sealed on the coupling surface with the inner cabinet other than the penetration portion by the nozzle sealing part, steam may be prevented from leaking to parts other than the penetration portion while ensuring a certain degree of mobility of the nozzle duct with respect to the inner cabinet.

In addition, by at least one of exemplary embodiments of the present invention, since the nozzle sealing part is made to have heat-resistant performance, the performance of the nozzle sealing part may be prevented from being deteriorated by steam corresponding to a relatively high temperature.

## Claims

1. A shoe care device comprising:
an inner cabinet having an accommodation space configured to accommodate a shoe;
a connection path configured to provide a flow path into which air from the accommodation space is introduced and then discharged back into the accommodation space;
a nozzle duct installed inside the inner cabinet to provide an air passage and having an upper discharge port that opens upward;
a nozzle coupled to an end of the nozzle duct to be insertable into the shoe inside the inner cabinet and having a lower discharge port that opens downward to spray air into the shoe;
a blowing part disposed in the connection path to blow air;
a dehumidifying part disposed in the connection path to dehumidify the air;
a heating part disposed in the connection path to heat the air;
a regeneration path branching from the connection path to allow the air heated by the heating path moves therethrough; and
a steam generator configured to supply steam to the inner cabinet,
wherein steam supplied to the inner cabinet is sprayed to the shoe through the upper discharge port and the lower discharge port.

2. The shoe care device of claim 1, further comprising:
a dry air duct that forms a portion of the connection path, the dry air duct being configured to guide air passing through the dehumidifying part to the nozzle duct,
wherein the steam generator supplies steam to a portion of the dry air duct.

3. The shoe care device of claim 2, further comprising:
a steam separator disposed between the steam generator and the dry air duct to remove condensed water in the steam; and
a steam connection pipe interconnecting a portion of the dry air duct and a separating connection port to discharge steam inside the steam separator.

4. The shoe care device of claim 3, wherein the dry air duct is connected to the steam connection pipe above the steam separator.

5. The shoe care device of claim 2, further comprising:
a controller configured to control a drying stroke for drying the accommodation space, a regeneration stroke for regenerating the dehumidifying part prior to the drying stroke, and a steam stroke for supplying steam by the steam generator,
wherein the controller controls air that has passed through the dehumidifying part to flow into the accommodation space while the first regeneration stroke is being executed, and controls the steam stroke to be executed between the first regeneration stroke and the first drying stroke.

6. The shoe care device of claim 5, further comprising:
a module housing configured to accommodate the blowing part, the dehumidifying part, and the heating part to form a portion of the connection path, the module housing being provided with each of a dry air outlet connected to the dry air duct and a humid air outlet connected to the regeneration path; and
a damper installed in the module housing to selectively shield the dry air outlet and the humid air outlet,
wherein the controller controls the steam stroke to be executed while the damper shields the dry air outlet.

7. The shoe care device of claim 6, further comprising:
a condenser that forms a portion of the regeneration path and is connected to the humid air outlet to condense moisture in air moving along the regeneration path,
wherein condensed water inside the dry air duct is discharged to the condenser in a state in which the damper opens the dry air outlet.

8. The shoe care device of claim 1, wherein the nozzle comprises:
a nozzle body hinged to the nozzle duct; and
a nozzle protrusion that protrudes downward from the nozzle body and is provided with the lower discharge port at an end thereof.

9. The shoe care device of claim 8, wherein the nozzle duct is installed to protrude forward from the rear wall of the inner cabinet.

10. The shoe care device of claim 9, wherein the lower discharge port has a discharge slope configured to guide sprayed air with a shape having a height that decreases from a front side to a rear side of the inner cabinet.

11. The shoe care device of claim 10, wherein the lower discharge port has a discharge through hole provided in a portion of the discharge slope.

12. The shoe care device of claim 8, wherein the nozzle body has an auxiliary discharge port provided in an upper surface thereof.

13. The shoe care device of claim 12, wherein the steam supplied to the inner cabinet is distributed to and sprayed from the upper discharge port and the auxiliary discharge port and then sprayed into the shoe through the lower discharge port.

14. The shoe care device of claim 9, wherein one end of the nozzle duct penetrates the inner cabinet and is coupled thereto.

15. The shoe care device of claim 14, wherein the nozzle duct comprises a nozzle sealing part interposed to surround an outer peripheral surface of the nozzle duct at a portion where the nozzle sealing part is coupled to the inner cabinet.

16. The shoe care device of claim 15, wherein the nozzle sealing part is made of a heat-resistant material.
